# EUROPEAN PATENT APPLICATION

(11) **EP 2 177 610 A1**
(43) Date of publication of application: **21.04.2010**
(21) Application number: 08778000.3
(22) Date of filing: 09.07.2008
(51) Int. Cl.: C12N 15/09, C12N 1/15, C12N 1/19, C12N 1/21, C12N 5/00, C12N 5/10, C12N 9/42

(54) **METHOD FOR MAINTAINING FOREIGN GENE IN CELL STABLY**

(30) Priority: 13.07.2007 JP 2007183931
(71) Applicant: Japan Agency for Marine-Earth Science and Technology, Yokosuka-shi Kanagawa 237-0061 (JP)
(72) Inventor: HATADA, Yuji, Yokosuka-shi Kanagawa 237-0061 (JP); OHTA, Yukari, Yokosuka-shi Kanagawa 237-0061 (JP); HIDAKA, Yuko, Yokosuka-shi Kanagawa 237-0061 (JP); NAKAMURA, Nobuyuki, Yokosuka-shi Kanagawa 237-0061 (JP)
(74) Representative: Vossius & Partner
(86) International application number: PCT/JP2008/062391
(87) International publication number: WO 2009/011258

(57) **Abstract**

A method for preparing a protein or peptide encoded by a foreign gene by expressing a foreign gene, which comprises the steps of: preparing a recombinant vector comprising in an expressible state a gene encoding an aminoacyl-tRNA synthetase, in which a desired foreign gene has been inserted in an expressible state; preparing a mutant host cell or the like in which a chromosomal gene encoding an aminoacyl-tRNA synthetase has been knocked out; transforming the mutant host cell with the recombinant vector to obtain a transformant; and culturing the transformant to prepare the protein or peptide encoded by the foreign gene. It becomes possible to provide a novel means for permitting the retention of a recombinant DNA cloning vector without employing an antibiotic and without limiting the composition of the medium; and a method for preparing a protein or peptide encoded by a foreign gene by using the means.

SEQUENCE LISTING
<110> Japan Agency for Marine-Earth Science and Technology
<120> Method of stably maintaining forigne genes in cells
<130> A85139H
<160> 89

<210> 1
<211> 2631
<212> DNA
<213> E. coli

<220>
<223> alaS Alanine-tRNA synthetase

<400> 1
atgagcaaga gcaccgctga gatccgtcag gcgtttctcg actttttcca tagtaaggga 60
catcaggtag ttgccagcag ctccctggta ccccataacg acccaacttt gttgtttacc 120
aacgccggga tgaaccagtt caaggatgtg ttccttgggc tcgacaagcg taattattcc 180
cgcgctacca cttcccaacg ctgcgtgcgt gcgggtggta aacacaacga cctggaaaac 240
gtcggttaca ccgcgcgtca ccataccttc ttcgaaatgc tgggcaactt cagcttcggc 300
gactatttca aacacgatgc cattcagttt gcatgggaac tgctgaccag cgaaaaatgg 360
tttgccctgc cgaaagagcg tctgtgggtt accgtctatg aaagcgacga cgaagcctac 420
gaaatctggg aaaaagaagt agggatcccg cgcgaacgta ttattcgcat cggcgataac 480
aaaggtgcgc catacgcatc tgacaacttc tggcagatgg gtgacactgg tccgtgcggc 540
ccgtgcaccg aaatcttcta cgatcacggc gaccacattt ggggggggcc tccgggaagc 600
ccggaagaag acggcgaccg ctacattgag atctggaaca tcgtcttcat gcagttcaac 660
cgccaggccg atggcacgat ggaaccgctg ccgaagccgt ctgtagatac cggtatgggt 720
ctggagcgta ttgctgcggt gctgcaacac gttaactcta actatgacat cgacctgttc 780
cgcacgctga tccaggcggt agcgaaagtc actggcgcaa ccgatctgag caataaatcg 840
ctgcgcgtaa tcgctgacca cattcgttct tgtgcgttcc tgatcgcgga tggcgtaatg 900
ccgtccaatg aaaaccgtgg ttatgtactg cgtcgtatca ttcgtcgcgc agtgcgtcac 960
ggtaatatgc tcggcgcgaa agaaaccttc ttctacaaac tggttggtcc gctgatcgac 1020
gttatgggct ctgcgggtga agacctgaaa cgccagcagg cgcaggttga gcaggtgctg 1080
aagactgaag aagagcagtt tgctcgtact ctggagcgcg gtctggcgtt gctggatgaa 1140
gagctggcaa aactttctgg tgatacgctg gatggtgaaa ctgctttccg tctgtacgac 1200
acctatggct tcccggttga cctgacggct gatgtttgtc gtgagcgcaa catcaaagtt 1260
gacgaagctg gttttgaagc tgcaatggaa gagcagcgtc gtcgcgcgcg cgaagccagc 1320
ggctttggtg ccgattacaa cgcaatgatc cgtgttgaca gtgcatctga atttaaaggc 1380
tatgaccatc tggaactgaa cggcaaagtg actgcgctgt ttgttgatgg taaagcggtt 1440
gatgccatca atgcaggcca ggaagctgtg gtcgtgctgg atcaaacgcc attctatgcg 1500
gaatccggcg gtcaggttgg cgataaaggc gaactgaaag gcgctaactt ctcctttgcg 1560
gtggaagata cgcagaaata cggccaggcg attggtcaca tcggtaaact tgctgcgggt 1620
tctctgaaag tgggcgacgc ggtgcaggct gatgttgatg aggctcgtcg cgcccgtatt 1680
cgtctgaatc actccgcaac gcacctgatg cacgctgcgc tgcgccaggt tctgggtact 1740
catgtatcgc agaaaggttc actggttaac gacaaggtgc tgcgcttcga cttctcacac 1800
aacgaagcga tgaaaccaga agagattcgt gcggtcgaag acctggtgaa cacacagatt 1860
cgtcgcaatt tgccgatcga aaccaacatc atggatctcg aagcggcgaa agcgaaaggt 1920
gcgatggcgc tgttcggcga gaagtatgat gagcgcgtac gcgtgctgag catgggcgat 1980

ttctctaccg agttgtgtgg cggtactcac gccagccgca ctggtgatat tggtctgttc 2040
cgcatcatct ctgaatcggg tactgctgca ggcgttcgtc gtatcgaagc ggtaaccgga 2100
gaaggtgcta tcgccaccgt tcatgcagac agcgatcgct taagcgaagt cgcgcatctg 2160
ctgaaaggcg atagcaataa tctggctgat aaagtgcgct cagtactgga acgtacgcgt 2220
cagctggaaa aagagttaca acagcttaaa gaacaagctg ccgcacagga gagcgcaaat 2280
ctttccagta aggcaattga tgttaatggt gttaagctgt tggttagcga gcttagcggt 2340
gttgagccga aaatgttgcg taccatggtt gacgatttaa aaaatcagct ggggtcgaca 2400
attatcgtgc tggcaacggt agtcgaaggt aaggtttctc tgattgcagg cgtatctaag 2460
gacgtcacag atcgtgtgaa agcaggggaa ctgattggta tggtcgctca gcaggtgggc 2520
ggcaagggtg gtggacgtcc tgacatggcg caagccggtg gtacggatgc tgcggcctta 2580
cctgcagcgt tagccagtgt gaaaggctgg gtcagcgcga aattgcaata a 2631

<210> 2
<211> 1734
<212> DNA
<213> E. coli

<220>
<223> argS Arginine-tRNA synthetase

<400> 2
gtgaatattc aggctcttct ctcagaaaaa gtccgtcagg ccatgattgc ggcaggcgcg 60
cctgcggatt gcgaaccgca ggttcgtcag tcagcaaaag ttcagttcgg cgactatcag 120
gctaacggca tgatggcagt tgctaaaaaa ctgggtatgg caccgcgaca attagcagag 180
caggtgctga ctcatctgga tcttaacggt atcgccagca aagttgagat cgccggtcca 240
ggctttatca acattttcct tgatccggca ttcctggctg aacatgttca gcaggcgctg 300
gcgtccgatc gtctcggtgt tgctacgcca gaaaaacaga ccattgtggt tgactactct 360
gcgccaaacg tggcgaaaga gatgcatgtc ggtcacctgc gctctaccat tattggtgac 420
gcagcagtgc gtactctgga gttcctcggt cacaaagtga ttcgcgcaaa ccacgtcggc 480
gactggggca ctcagttcgg tatgctgatt gcatggctgg aaaagcagca gcaggaaaac 540
gccggtgaaa tggagctggc tgaccttgaa ggtttctacc gcgatgcgaa aaagcattac 600
gatgaagatg aagagttcgc cgagcgcgca cgtaactacg tggtaaaact gcaaagcggt 660
gacgaatatt tccgcgagat gtggcgcaaa ctggtcgaca tcaccatgac gcagaaccag 720
atcacctacg atcgtctcaa cgtgacgctg acccgtgatg acgtgatggg cgaaagcctc 780
tacaacccga tgctgccagg aattgtggcg gatctcaaag ccaaaggtct ggcagtagaa 840
agcgaagggg cgaccgtcgt attccttgat gagtttaaaa acaaggaagg cgaaccgatg 900
ggcgtgatca ttcagaagaa agatggcggc tatctctaca ccaccactga tatcgcctgt 960
gcgaaatatc gttatgaaac actgcatgcc gatcgcgtgc tgtattacat cgactcccgt 1020
cagcatcaac acctgatgca ggcatgggcg atcgtccgta aagcaggcta tgtaccggaa 1080
tccgtaccgc tggaacacca catgttcggc atgatgctgg gtaaagacgg caaaccgttc 1140
aaaacccgcg cgggtggtac agtgaaactg gccgatctgc tggatgaagc cctggaacgt 1200
gcacgccgtc tggtggcaga aaagaacccg gatatgccag ccgacgagct ggaaaaactg 1260
gctaacgcgg ttggtattgg tgcggtgaaa tatgcggatc tctccaaaaa ccgcaccacg 1320
gactacatct tcgactggga caacatgctg gcgtttgagg gtaataccgc gccatacatg 1380
cagtatgcat acacgcgtgt attgtccgtg ttccgtaaag cagaaattga cgaagagcaa 1440
ctggctgcag ctccggttat catccgtgaa gatcgtgaag cgcaactggc agctcgcctg 1500
ctgcagtttg aagaaaccct caccgtggtt gcccgtgaag gcacgccgca tgtaatgtgt 1560
gcttacctgt acgatctggc cggtctgttc tctggcttct acgagcactg cccgatcctc 1620

agcgcagaaa acgaagaagt gcgtaacagc cgtctaaaac tggcacaact gacggcgaag    1680
acgctgaagc tgggtctgga tacgctgggt attgagactg tagagcgtat gtaa          1734

<210> 3
<211> 1401
<212> DNA
<213> E. coli

<220>
<223> asnS Asparagine-tRNA synthetase

<400> 3
atgagcgttg tgcctgtagc cgacgtactc cagggccgtg tagccgttga cagcgaagtc      60
accgtgcgcg gatgggtacg tacccgccga gattcaaaag ctggcatctc cttcctcgcc     120
gtttatgacg gttcctgctt tgatcctgta caggctgtca tcaataattc tctgcccaat     180
tacaatgaag acgtcctgcg tctgaccacc ggctgctcgg tcattgtgac gggtaaagtc     240
gtggcgtcgc cgggccaggg gcaacaattt gaaattcagg ccagcaaggt tgaagttgct     300
ggttgggttg aagatccaga cacttacccg atggcggcaa aacgccacag cattgagtat     360
ctgcgtgaag tcgctcacct gcgtccgcgc acaaacctga ttggtgccgt cgcgcgcgtt     420
cgccatacgc tggcgcaggc gctgcatcgc ttctttaacg agcagggatt cttctgggtt     480
tcaacgccac tgattaccgc atctgatacc gaaggtgcag gcgaaatgtt ccgcgtttct     540
acgctggatc tggaaaacct gccgcgtaac gatcagggca aagtggattt cgacaaagac     600
ttctttggta aagagtcttt cctgaccgta tctggccagt tgaacggcga aacctacgct     660
tgcgcattgt ccaaaattta taccttcggc ccgactttcc gtgctgaaaa ctccaacacc     720
agccgtcacc tggcggaatt ctggatgctg gagccggaag tggcgtttgc taacctgaac     780
gatattgcgg gtctggctga agccatgctg aaatatgtct tcaaagcggt tctcgaagaa     840
cgcgctgacg acatgaaatt cttcgctgaa cgcgtagata aagatgccgt ttcacgtctg     900
gaacgcttca ttgaagccga ttttgcgcag gtggattata ccgacgcagt gaccattctc     960
gaaaactgcg gcaggaagtt tgaaaacccg gtttactggg gagtcgatct ctcttctgag    1020
catgagcgtt atctggcgga agaacacttt aaagcaccgg tagtggttaa aaactatccg    1080
aaagatatta aagcgttcta tatgcgcctt aacgaagacg gtaaaaccgt tgcggctatg    1140
gacgttctgg ctccgggcat cggtgagatc attggtggct cccagcgtga agaacgtctg    1200
gacgtgctgg acgagcgtat gctggaaatg ggcctgaata aagaagatta ctggtggtat    1260
cgcgatctgc gtcgctacgg tactgttccg cattcaggtt tcggtcttgg ttttgaacgt    1320
ctgattgctt acgtaactgg cgtgcaaaac gtacgtgatg tgattccgtt cccacgtact    1380
ccgcgtaacg ccagcttcta a                                              1401

<210> 4
<211> 1773
<212> DNA
<213> E. coli

<220>
<223> aspS Aspartate-tRNA synthetase

<400> 4
atgcgtacag aatattgtgg acagctccgt ttgtcccacg tggggcagca ggtgactctg      60

tgtggttggg tcaaccgtcg tcgtgatctt ggtagcctga tcttcatcga tatgcgcgac 120
cgcgaaggta tcgtgcaggt atttttcgat ccggatcgtg cggacgcgtt aaagctggcc 180
tctgaactgc gtaatgagtt ctgcattcag gtcacgggca ccgtacgtgc gcgtgacgaa 240
aaaaatatta accgcgatat ggcgaccggc gaaatcgaag tgctggcgtc ctcgctgact 300
atcatcaacc gcgcagatgt tctgccgctt gactctaacc acgtcaacac cgaagaagcg 360
cgtctgaaat accgctacct cgacctgcgt cgtccggaaa tggctcagcg cctgaaaacc 420
cgcgctaaaa tcaccagcct ggtgcgccgt tttatggatg accacggctt cctcgacatc 480
gaaactccga tgctgaccaa agccacgccg gaaggcgcgc gtgactacct ggtgccttct 540
cgtgtgcaca aaggtaaatt ctacgcactg ccgcaatccc cgcagttgtt caaacagctg 600
ctgatgatgt ccggttttga ccgttactat cagatcgtta aatgcttccg tgacgaagac 660
ctgcgtgctg accgtcagcc tgaatttact cagatcgatg tggaaacttc tttcatgacc 720
gcgccgcaag tgcgtgaagt gatggaagcg ctggtgcgtc atctgtggct ggaagtgaag 780
ggtgtggatc tgggcgattt cccggtaatg acctttgcgg aagcagaacg ccgttatggt 840
tctgataaac cggatctgcg taacccgatg gaactgactg acgttgctga tctgctgaaa 900
tctgttgagt ttgctgtatt tgcaggtccg gcgaacgatc cgaaaggtcg cgtagcggct 960
ctgcgcgttc cgggcggcgc atcgctgacc cgtaagcaga tcgacgaata cggtaacttc 1020
gttaaaatct acggcgcgaa aggtctggct tacatcaaag ttaacgaacg cgcgaaaggt 1080
ctggaaggta tcaacagccc ggtagcgaag ttccttaatg cagaaatcat cgaagacatc 1140
ctggatcgta ctgccgcgca agatggcgat atgattttct tcggtgccga caacaagaaa 1200
attgttgccg acgcgatggg tgcactgcgc ctgaaagtgg gtaaagacct tggtctgacc 1260
gacgaaagca aatgggcacc gctgtgggtt atcgacttcc cgatgtttga agacgacggt 1320
gaaggcggcc tgacggcaat gcaccatccg ttcacctcac cgaaagatat gacggctgca 1380
gaactgaaag ctgcaccgga aaatgcggtg gcgaacgctt acgatatggt catcaatggt 1440
tacgaagtgg gcggtggttc agtacgtatc cataatggtg atatgcagca gacggtgttt 1500
ggtattctgg gtatcaacga agaggaacag cgcgagaaat tcggcttcct gctcgacgct 1560
ctgaaatacg gtactccgcc gcacgcaggt ctggcattcg gtcttgaccg tctgaccatg 1620
ctgctgaccg gcaccgacaa tatccgtgac gttatcgcct tcccgaaaac cacggcggca 1680
gcgtgtctga tgactgaagc accgagcttt gctaacccga ctgcactggc tgagctgagc 1740
attcaggttg tgaagaaggc tgagaataac tga 1773

<210> 5
<211> 1386
<212> DNA
<213> E. coli

<220>
<223> cysS Cysteine-tRNA synthetase

<400> 5
atgctaaaaa tcttcaatac tctgacacgc caaaaagagg aatttaagcc tattcacgcc 60
ggggaagtcg gcatgtacgt gtgtggaatc accgtttacg atctctgtca tatcggtcac 120
gggcgtacct ttgttgcttt tgacgtggtt gcgcgctatc tgcgtttcct cggctataaa 180
ctgaagtatg tgcgcaacat taccgatatc gacgacaaaa tcatcaaacg cgccaatgaa 240
aatggcgaaa gctttgtggc gatggtggat cgcatgatcg ccgaaatgca caaagatttt 300
gatgctttga acattctgcg cccggatatg gagccgcgcg cgacgcacca tatcgcagaa 360
attattgaac tcactgaaca actgatcgcc aaaggtcacg cttatgtggc ggacaacggc 420
gacgtgatgt tcgacgtccc gaccgatcca acttatggcg tgctgtcgcg tcaggatctc 480
gaccagctgc aggcaggcgc gcgcgttgac gtggtcgacg acaaacgcaa cccaatggac 540

ttcgttctgt ggaagatgtc gaaagagggc gaaccgagct ggccgtctcc gtggggcgcg 600
ggtcgtcctg gctggcacat tgaatgttcg gcaatgaact gcaagcagct gggtaaccac 660
tttgatatcc acggcggcgg ttcagacctg atgttcccgc accacgaaaa cgaaatcgcg 720
cagtccacct gtgcccatga tggtcagtat gtgaactact ggatgcactc ggggatggtg 780
atggttgacc gcgagaagat gtccaaatcg ctgggtaact tctttaccgt gcgcgatgtg 840
ctgaaatact acgacgcgga aaccgtgcgt tacttcctga tgtcgggcca ctatcgcagc 900
cagttgaact acagcgaaga gaacctgaag caggcgcgtg cggcgctgga gcgtctctac 960
actgcgctgc gcggcacaga taaaaccgtt gcgcctgccg gtggcgaagc gtttgaagcg 1020
cgctttattg aagcgatgga cgacgatttc aacaccccgg aagcctattc cgtactgttt 1080
gatatggcgc gtgaagtaaa ccgtctgaaa gcagaagata tggcagcggc gaatgcaatg 1140
gcatctcacc tgcgtaaact ttccgctgta ttgggcctgc tggagcaaga accggaagcg 1200
ttcctgcaaa gcggcgcgca ggcagacgac agcgaagtgg ctgagattga agcgttaatt 1260
caacagcgtc tggatgcccg taaagcgaaa gactgggcgg cggcggatgc ggcgcgtgat 1320
cgtcttaacg agatggggat cgtgctggaa gatggcccgc aagggaccac ctggcgtcgt 1380
aagtaa 1386

<210> 6
<211> 1665
<212> DNA
<213> E. coli

<220>
<223> glnS Glutamine-tRNA synthetase

<400> 6
   atgagtgagg cagaagcccg cccgactaac tttatccgtc agatcatcga tgaagatctg 60
   gccagtggta agcacaccac agtacacacc cgtttcccgc cggagccgaa tggctatctg 120
   catattggcc atgcgaaatc tatctgcctg aacttcggga tcgcccagga ctataaaggc 180
   cagtgcaacc tgcgtttcga cgacactaac ccggtaaaag aagatatcga gtatgttgag 240
   tcgatcaaaa acgacgtaga gtggttaggt tttcactggt ctggtaacgt ccgttactcc 300
   tccgattatt ttgatcagct ccacgcctat gcgatcgaac tgatcaataa aggcctggcg 360
   tacgttgatg aactgacgcc ggaacagatc cgcgaatacc gcggcaccct gacgcaaccg 420
   ggtaaaaaca gcccgtaccg cgaccgcagc gttgaagaga acctggcgct gttcgaaaaa 480
   atgcgtgccg gtggttttga agaaggtaaa gcctgcctgc gtgcgaaaat cgacatggct 540
   tcaccgttta tcgtgatgcg cgatccggtg ctgtaccgta ttaaatttgc tgaacaccac 600
   cagactggca acaagtggtg catctacccg atgtacgact tcacccactg catcagcgat 660
   gccctggaag gtattacgca ctctctgtgt acgcttgagt tccaggacaa ccgtcgtctg 720
   tacgactggg tactggacaa catcacgatt cctgttcacc cgcgccagta tgagttctcg 780
   cgcctgaatc tggaatacac cgtgatgtcc aagcgtaagt tgaacctgct ggtgaccgac 840
   aagcacgttg aaggctggga tgacccgcgt atgccgacca tttccggtct gcgtcgtcgt 900
   ggttacactg cggcttctat tcgtgagttc tgcaaacgca tcggcgtgac caagcaggac 960
   aacaccattg agatggcgtc gctggaatcc tgcatccgtg aagatctcaa cgaaaatgcg 1020
   ccgcgcgcaa tggcggttat cgatccggtg aaactggtta tcgaaaacta tcagggcgaa 1080
   ggcgaaatgg ttaccatgcc gaaccatccg aacaaaccgg aaatgggcag ccgtcaggtg 1140
   ccgtttagcg gtgagatttg gattgatcgc gccgatttcc gcgaagaagc taacaagcag 1200
   tacaaacgtc tggtgctggg taaagaagtg cgtctgcgta atgcttatgt gattaaggca 1260
   gaacgcgtcg agaaagatgc cgaaggtaat atcaccacca tcttctgtac ttatgacgcc 1320
   gataccttaa gcaaagatcc ggcagatggt cgtaaagtca aaggtgttat tcactgggtg 1380
   agcgcggcac atgcgctgcc ggttgaaatc cgtttgtatg accgtctgtt cagcgtgcct    1440
   aacccaggtg ctgcggatga tttcctgtcg gtgattaacc cggaatcgct ggtgatcaaa    1500
   cagggctttg ctgaaccgtc gctgaaagat gcggttgcgg gtaaagcatt ccagtttgag    1560
   cgtgaaggtt acttctgcct cgatagccgc cattctacgg cggaaaaacc ggtatttaac    1620
   cgcaccgttg ggctgcgtga tacctgggcg aaagtaggcg agtaa                    1665

<210> 7
<211> 1665
<212> DNA
<213> E. coli

<220>
<223> gltX Glutamate-tRNA synthetase

<400> 7
atgagtgagg cagaagcccg cccgactaac tttatccgtc agatcatcga tgaagatctg      60
gccagtggta agcacaccac agtacacacc cgtttcccgc cggagccgaa tggctatctg     120
catattggcc atgcgaaatc tatctgcctg aacttcggga tcgcccagga ctataaaggc     180
cagtgcaacc tgcgtttcga cgacactaac ccggtaaaag aagatatcga gtatgttgag     240
tcgatcaaaa acgacgtaga gtggttaggt tttcactggt ctggtaacgt ccgttactcc     300
tccgattatt ttgatcagct ccacgcctat gcgatcgaac tgatcaataa aggcctggcg     360
tacgttgatg aactgacgcc ggaacagatc cgcgaatacc gcggcaccct gacgcaaccg     420
ggtaaaaaca gcccgtaccg cgaccgcagc gttgaagaga acctggcgct gttcgaaaaa     480
atgcgtgccg gtggttttga agaaggtaaa gcctgcctgc gtgcgaaaat cgacatggct     540
tcaccgttta tcgtgatgcg cgatccggtg ctgtaccgta ttaaatttgc tgaacaccac     600
cagactggca acaagtggtg catctacccg atgtacgact tcacccactg catcagcgat     660
gccctggaag gtattacgca ctctctgtgt acgcttgagt tccaggacaa ccgtcgtctg     720
tacgactggg tactggacaa catcacgatt cctgttcacc cgcgccagta tgagttctcg     780
cgcctgaatc tggaatacac cgtgatgtcc aagcgtaagt tgaacctgct ggtgaccgac     840
aagcacgttg aaggctggga tgacccgcgt atgccgacca tttccggtct gcgtcgtcgt     900
ggttacactg cggcttctat tcgtgagttc tgcaaacgca tcggcgtgac caagcaggac     960
aacaccattg agatggcgtc gctggaatcc tgcatccgtg aagatctcaa cgaaaatgcg    1020
ccgcgcgcaa tggcggttat cgatccggtg aaactggtta tcgaaaacta tcagggcgaa    1080
ggcgaaatgg ttaccatgcc gaaccatccg aacaaaccgg aaatgggcag ccgtcaggtg    1140
ccgtttagcg gtgagatttg gattgatcgc gccgatttcc gcgaagaagc taacaagcag    1200
tacaaacgtc tggtgctggg taaagaagtg cgtctgcgta atgcttatgt gattaaggca    1260
gaacgcgtcg agaaagatgc cgaaggtaat atcaccacca tcttctgtac ttatgacgcc    1320
gataccttaa gcaaagatcc ggcagatggt cgtaaagtca aaggtgttat tcactgggtg    1380
agcgcggcac atgcgctgcc ggttgaaatc cgtttgtatg accgtctgtt cagcgtgcct    1440
aacccaggtg ctgcggatga tttcctgtcg gtgattaacc cggaatcgct ggtgatcaaa    1500
cagggctttg ctgaaccgtc gctgaaagat gcggttgcgg gtaaagcatt ccagtttgag    1560
cgtgaaggtt acttctgcct cgatagccgc cattctacgg cggaaaaacc ggtatttaac    1620
cgcaccgttg ggctgcgtga tacctgggcg aaagtaggcg agtaa                    1665

<210> 8
<211> 912

<212> DNA
<213> E. coli

<220>
<223> glyQ Glycine-tRNA synthetase, alpha-subunit

<400> 8
atgcaaaagt ttgataccag gaccttccag ggcttgatcc tgaccttaca ggattactgg      60
gctcgccagg gctgcaccat tgttcaacca ttggacatgg aagtcggcgc gggaacctct     120
cacccaatga cctgtctgcg cgagctgggg ccagaaccga tggcggctgc ttatgttcag     180
ccttctcgtc gcccgaccga tggtcgctac ggcgaaaacc ccaaccgttt acagcactac     240
tatcagttcc aggtggtcat taagccatcg ccggacaata ttcaggagct gtacctcggt     300
tctctgaaag agctgggcat ggacccgact attcacgaca tccgtttcgt ggaagataac     360
tgggaaaacc cgacgctggg tgcctgggga ctgggctggg aagtgtggct gaacggcatg     420
gaagtgacgc agttcactta cttccagcag gttggtggtc tggagtgtaa accggttacc     480
ggcgagatca cctacggtct ggaacgtctg gccatgtaca ttcagggcgt agacagcgtt     540
tacgacctgg tctggagcga cggcccgctg ggtaaaacca cctacggcga cgtgttccat     600
cagaacgaag tggagcagtc cacttacaac ttcgaatacg cggatgtgga cttcctgttc     660
acctgcttcg agcagtacga gaaagaagcg cagcagctgc tggcgctgga aaatccgctg     720
ccgctgccag cctacgagcg tattctgaaa gccgcccaca gcttcaacct gctggatgcg     780
cgtaaagcca tctccgtcac cgagcgtcag cgctacattc tgcgcattcg caccctgacc     840
aaagcagtgg cagaagcata ctacgcttcc cgtgaagccc tcggcttccc gatgtgcaac     900
aaagataagt aa                                                         912

<210> 9
<211> 2070
<212> DNA
<213> E. coli

<220>
<223> glyS Glycine-tRNA synthetase, beta-subunit

<400> 9
atgtctgaga aaacttttct ggtggaaatc ggcactgaag agctgccacc aaaagcactg      60
cgcagcctgg ctgagtcctt tgctgcgaac tttactgcgg agctggataa cgctggcctc     120
gcacacggca ccgttcaatg gtttgctgct ccgcgtcgtc tggcgctgaa agtagctaac     180
ctggcggaag cgcaaccgga tcgtgaaatc gaaaaacgcg gcccggcgat tgcccaggcg     240
ttcgacgctg aaggcaaacc gagcaaagcg gcagaaggtt gggcgcgtgg ttgcggtatt     300
accgttgacc aggctgagcg tctgactacc gataaaggcg aatggctgct gtatcgcgcc     360
catgtgaagg gcgaaagcac cgaagcactg ctgccgaata tggttgcgac ttctctggcg     420
aaactgccga tcccgaaact gatgcgttgg ggcgcaagcg acgtgcactt cgtgcgtccg     480
gtgcacaccg tgaccctgct gctgggcgac aaagtcattc cggcaaccat tctgggcatt     540
cagtccgatc gcgtgattcg cggccaccgc tttatgggcg agccggaatt caccatcgat     600
aacgccgatc agtatccgga aattctgcgt gagcgtggga aagtcatcgc cgattacgaa     660
gaacgtaagg cgaagattaa agccgatgcc gaagaagcag cgcgtaagat tggcggtaac     720
gctgacttaa gcgaaagcct gctggaagaa gtggcttcgc tggtggagtg gccggtcgtt     780
ctgaccgcaa aattcgaaga gaaattcctc gcggtgccgg ctgaagcgct ggtttacacc     840
atgaaaggtg accagaaata cttcccggtg tatgcgaacg acggcaaact gctgccgaac     900

tttatcttcg ttgccaacat cgaatcgaaa gatccgcagc agattatctc cggtaacgag 960
aaagtcgttc gtccgcgtct ggcggatgcc gagttcttct tcaacaccga ccgtaaaaaa 1020
cgtcttgaag ataacctgcc gcgcctgcaa accgtgttgt tccagcaaca gttggggacg 1080
ctgcgcgaca aaactgaccg catccaggcg ctggctggct ggattgctga acagattggc 1140
gctgacgtta accacgctac ccgtgcgggt ctgctgtcta agtgcgacct gatgaccaac 1200
atggtcttcg agttcaccga cacccagggc gttatgggga tgcactatgc gcgtcacgat 1260
ggcgaagcgg aagatgtcgc ggtggcgctg aatgagcagt atcagccgcg ttttgctggt 1320
gatgacctgc cgtccaaccc agtagcttgt gcgctggcga ttgctgacaa gatggatacc 1380
ctggcgggta tcttcggtat cggtcagcat ccgaaaggcg acaaagaccc gtttgcgctg 1440
cgtcgtgccg cgcttggcgt gctgcgaatt atcgttgaga agaacctcaa ccttgatctg 1500
caaacgctga ccgaagaagc ggtgcgtctg tatggcgata agctgactaa tgccaacgta 1560
gttgatgatg ttatcgactt tatgctcggt cgcttccgcg cctggtatca ggacgaaggt 1620
tataccgttg acaccatcca ggcggtactg gcgcgtcgtc cgactcgtcc ggctgatttc 1680
gatgcccgta tgaaagcggt atcgcatttc cgtaccctgg atgcagctgc tgcactggcg 1740
gcggcgaaca aacgtgtatc taacattctg gcgaaatctg acgaagtgct gagcgaccgc 1800
gtgaatgcct ctaccctgaa agagccggaa gaaattaaac tggcgatgca ggttgtggtg 1860
ctacgtgaca agctggagcc gtactttacg gaaggtcgtt accaggatgc gctggtcgaa 1920
ctggctgagc tgcgtgaacc ggttgatgct ttcttcgata aagtgatggt catggttgat 1980
gacaaagaat tgcgtatcaa ccgtctgacc atgctggaga aactgcgcga actgttcctg 2040
cgcgttgcgg atatttcgct gttgcaataa 2070

<210> 10
<211> 1275
<212> DNA
<213> E. coli

<220>
<223> hisS Histidine-tRNA synthetase

<400> 10
gtggcaaaaa acattcaagc cattcgcggc atgaacgatt acctgcctgg cgaaacggcc 60
atctggcagc gcattgaagg cacactgaaa aacgtgctcg gcagctacgg ttacagtgaa 120
atccgcttgc cgattgtaga gcagaccccg ctattcaaac gtgcgattgg tgaagtcacc 180
gacgtggttg aaaaagagat gtacaccttt gaggatcgca atggcgacag cctgactctg 240
cgccctgaag ggacggcggg ctgtgtacgc gccggcatcg agcatggtct tctgtacaat 300
caggaacagc gtctgtggta tatcggcccg atgttccgtc acgagcgtcc gcagaaaggg 360
cgttatcgtc agttccatca gttgggctgc gaagttttcg gtctgcaagg tccggatatc 420
gacgctgaac tgattatgct cactgcccgc tggtggcgcg cgctgggtat ttccgagcac 480
gtaactcttg agctgaactc tatcggttcg ctggaagcac gcgccaatta ccgcgatgcg 540
ctggtggcat tccttgagca gcataaagaa aagctggacg aagactgcaa acgccgcatg 600
tacactaacc cgctgcgcgt gctggattca aaaaatccgg aagtgcaggc gcttctcaac 660
gacgctccgg cattaggtga ctatctggac gaggaatctc gtgagcattt tgccggtctg 720
tgcaaactgc tggagagcgc ggggatcgct tacaccgtaa accagcgtct ggtgcgtggt 780
ctggattact acaaccgtac cgttttcgag tgggtgacta acagtctcgg ctcccagggc 840
accgtgtgtg caggcggtcg ttatgacggt cttgtggaac aactgggcgg tcgtgcaaca 900
ccggctgtcg gttttgctat gggcctcgaa cgtcttgtat tgttagtaca ggccgttaat 960
ccggaattta aagccgatcc tgttgtcgat atatacctgg tggcttcagg tgctgataca 1020
caatctgcgg ctatggcatt agctgagcgt ctgcgtgatg aattaccggg cgtgaaattg 1080

atgaccaacc acggcggcgg caactttaag aaacagtttg cccgtgctga taaatggggt 1140
gcccgcgttg ctgtggtgct gggtgagtct gaagtggcta acggcacagc agtagtgaag 1200
gatttgcgct ctggtgagca aacggcagtt gcgcaggata gcgtagccgc gcatttgcgc 1260
acgttactgg gttaa 1275

<210> 11
<211> 2817
<212> DNA
<213> E. coli

<220>
<223> ileS Isoleucine-tRNA synthetase

<400> 11
atgagtgact ataaatcaac cctgaatttg ccggaaacag ggttcccgat gcgtggcgat 60
ctcgccaagc gcgaacccgg aatgctggcg cgttggactg atgatgatct gtacggcatc 120
atccgtgcgg ctaaaaaagg caaaaaaacc ttcattctgc atgatggccc tccttatgcg 180
aatggcagca ttcatattgg tcactcggtt aacaagattc tgaaagacat tatcgtgaag 240
tccaaagggc tttccggtta tgactcgccg tatgtgcctg gctgggactg ccacggtctg 300
ccgatcgagc tgaaagtcga gcaagaatac ggtaagccgg gtgagaaatt caccgccgcc 360
gagttccgcg ccaagtgccg cgaatacgcg gcgacccagg ttgacggtca acgcaaagac 420
tttatccgtc tgggcgtgct gggcgactgg tcgcacccgt acctgaccat ggacttcaaa 480
actgaagcca acatcatccg cgcgctgggc aaaatcatcg gcaacggtca cctgcacaaa 540
ggcgcgaagc cagttcactg gtgcgttgac tgccgttctg cgctggcgga agcggaagtt 600
gagtattacg acaaaacttc tccgtccatc gacgttgctt tccaggcagt cgatcaggat 660
gcactgaaag caaaatttgc cgtaagcaac gttaacggcc caatctcgct ggtaatctgg 720
accaccacgc cgtggactct gcctgccaac cgcgcaatct ctattgcacc agatttcgac 780
tatgcgctgg tgcagatcga cggtcaggcc gtgattctgg cgaaagatct ggttgaaagc 840
gtaatgcagc gtatcggcgt gaccgattac accattctcg gcacggtaaa aggtgcggag 900
cttgagctgc tgcgctttac ccatccgttt atgggcttcg acgttccggc aatcctcggc 960
gatcacgtta ccctggatgc cggtaccggt gccgttcaca ccgcgcctgg ccacggcccg 1020
gacgactatg tgatcggtca gaaatacggc ctggaaaccg ctaacccggt tggcccggac 1080
ggcacttatc tgccgggcac ttatccgacg ctggatggcg tgaacgtctt caaagcgaac 1140
gacatcgtcg ttgcgctgct gcaggaaaaa ggcgcgctgc tgcacgttga gaaaatgcag 1200
cacagctatc cgtgctgctg gcgtcacaaa acgccgatca tcttccgcgc gacgccgcag 1260
tggttcgtca gcatggatca gaaaggtctg cgtgcgcagt cactgaaaga gatcaaaggc 1320
gtgcagtgga tcccggactg gggccaggcg cgtatcgagt cgatggttgc taaccgtcct 1380
gactggtgta tctcccgtca gcgcacctgg ggtgtaccga tgtcactgtt cgtgcacaaa 1440
gacacggaag agctgcatcc gcgtaccctt gaactgatgg aagaagtggc aaaacgcgtt 1500
gaagtcgatg gcatccaggc gtggtgggat ctcgatgcga aagagatcct cggcgacgaa 1560
gctgatcagt acgtgaaagt gccggacaca ttggatgtat ggtttgactc cggatctacc 1620
cactcttctg ttgttgacgt gcgtccggaa tttgccggtc acgcagcgga catgtatctg 1680
gaaggttctg accaacaccg cggctggttc atgtcttccc taatgatctc caccgcgatg 1740
aagggtaaag cgccgtatcg tcaggtactg acccacggct ttaccgtgga tggtcagggc 1800
cgcaagatgt ctaaatccat cggcaatacc gtttcgccgc aggatgtgat gaacaaactg 1860
ggcgcggata ttctgcgtct gtgggtggca tcaaccgact acaccggtga aatggccgtt 1920
tctgacgaga tcctgaaacg tgctgccgat agctatcgtc gtatccgtaa caccgcgcgc 1980
ttcctgctgg caaacctgaa cggttttgat ccagcaaaag atatggtgaa accggaagag 2040

atggtggtac tggatcgctg ggccgtaggt tgtgcgaaag cggcacagga agacatcctc 2100
aaggcgtacg aagcatacga tttccacgaa gtggtacagc gtctgatgcg cttctgctcc 2160
gttgagatgg gttccttcta cctcgacatc atcaaagacc gtcagtacac cgccaaagcg 2220
gacagtgtgg cgcgtcgtag ctgccagact gcgctatatc acatcgcaga agcgctggtg 2280
cgctggatgg caccaatcct ctccttcacc gctgatgaag tgtggggcta cctgccgggc 2340
gaacgtgaaa aatacgtctt caccggtgag tggtacgaag gcctgtttgg cctggcagac 2400
agtgaagcga tgaacgatgc gttctgggac gagctgttga aagtgcgtgg cgaagtgaac 2460
aaagtcattg agcaagcgcg tgccgacaag aaagtgggtg gctcgctgga agcggcagta 2520
accttgtatg cagaaccgga actgtcggcg aaactgaccg cgctgggcga tgaattacga 2580
tttgtcctgt tgacctccgg cgctaccgtt gcagactata acgacgcacc tgctgatgct 2640
cagcagagcg aagtactcaa agggctgaaa gtcgcgttga gtaaagccga aggtgagaag 2700
tgcccacgct gctggcacta cacccaggat gtcggcaagg tggcggaaca cgcagaaatc 2760
tgcggccgct gtgtcagcaa cgtcgccggt gacggtgaaa aacgtaagtt tgcctga 2817

<210> 12
<211> 2583
<212> DNA
<213> E. coli

<220>
<223> leuS Leucine-tRNA synthetase

<400> 12
atgcaagagc aataccgccc ggaagagata gaatccaaag tacagcttca ttgggatgag 60
aagcgcacat ttgaagtaac cgaagacgag agcaaagaga agtattactg cctgtctatg 120
cttccctatc cttctggtcg actacacatg ggccacgtac gtaactacac catcggtgac 180
gtgatcgccc gctaccagcg tatgctgggc aaaaacgtcc tgcagccgat cggctgggac 240
gcgtttggtc tgcctgcgga aggcgcggcg gtgaaaaaca acaccgctcc ggcaccgtgg 300
acgtacgaca acatcgcgta tatgaaaaac cagctcaaaa tgctgggctt tggttatgac 360
tggagccgcg agctggcaac ctgtacgccg gaatactacc gttgggaaca gaaattcttc 420
accgagctgt ataaaaaagg cctggtatat aagaagactt ctgcggtcaa ctggtgcccg 480
aacgaccaga ccgtactggc gaacgaacaa gttatcgacg gctgctgctg gcgctgcgat 540
accaaagttg aacgtaaaga gatcccgcag tggtttatca aaatcactgc ttacgctgac 600
gagctgctca acgatctgga taaactggat cactggccag acaccgttaa aaccatgcag 660
cgtaactgga tcggtcgttc cgaaggcgtg gagatcacct tcaacgttaa cgactatgac 720
aacacgctga ccgtttacac tacccgcccg gacaccttta tgggttgtac ctacctggcg 780
gtagctgcgg gtcatccgct ggcgcagaaa gcggcggaaa ataatcctga actggcggcc 840
tttattgacg aatgccgtaa caccaaagtt gccgaagctg aaatggcgac gatggagaaa 900
aaaggcgtcg atactggctt taaagcggtt cacccattaa cgggcgaaga aattcccgtt 960
tgggcagcaa acttcgtatt gatggagtac ggcacgggcg cagttatggc ggtaccgggg 1020
cacgaccagc gcgactacga gtttgcctct aaatacggcc tgaacatcaa accggttatc 1080
ctggcagctg acggctctga gccagatctt tctcagcaag ccctgactga aaaaggcgtg 1140
ctgttcaact ctggcgagtt caacggtctt gaccatgaag cggccttcaa cgccatcgcc 1200
gataaactga ctgcgatggg cgttggcgag cgtaaagtga actaccgcct gcgcgactgg 1260
ggtgtttccc gtcagcgtta ctggggcgcg ccgattccga tggtgacgct ggaagacggt 1320
accgtaatgc cgaccccgga cgaccagctg ccggtgatcc tgccggaaga tgtggtaatg 1380
gacggcatta ccagcccgat taaagcagat ccggagtggg cgaaaactac cgttaacggt 1440
atgccagcac tgcgtgaaac cgacactttc gacaccttta tggagtcctc ctggtactat 1500

gcgcgctaca cttgcccgca gtacaaagaa ggtatgctgg attccgaagc ggctaactac 1560
tggctgccgg tggatatcta cattggtggt attgaacacg ccattatgca cctgctctac 1620
ttccgcttct tccacaaact gatgcgtgat gcaggcatgg tgaactctga cgaaccagcg 1680
aaacagttgc tgtgtcaggg tatggtgctg gcagatgcct tctactatgt tggcgaaaac 1740
ggcgaacgta actgggtttc cccggttgat gctatcgttg aacgtgacga gaaaggccgt 1800
atcgtgaaag cgaaagatgc ggcaggccat gaactggttt ataccggcat gagcaaaatg 1860
tccaagtcga agaacaacgg tatcgacccg caggtgatgg ttgaacgtta cggcgcggac 1920
accgttcgtc tgtttatgat gtttgcttct ccggctgata tgactctcga atggcaggaa 1980
tccggtgtgg aaggggctaa ccgcttcctg aaacgtgtct ggaaactggt ttacgagcac 2040
acagcaaaag gtgatgttgc ggcactgaac gttgatgcgc tgactgaaaa tcagaaagcg 2100
ctgcgtcgcg atgtgcataa aacgatcgct aaagtgaccg atgatatcgg ccgtcgtcag 2160
accttcaaca ccgcaattgc ggcgattatg gagctgatga acaaactggc gaaagcacca 2220
accgatggcg agcaggatcg cgctctgatg caggaagcac tgctggccgt tgtccgtatg 2280
cttaacccgt tcaccccgca catctgcttc acgctgtggc aggaactgaa aggcgaaggc 2340
gatatcgaca acgcgccgtg gccggttgct gacgaaaaag cgatggtgga agactccacg 2400
ctggtcgtgg tgcaggttaa cggtaaagtc cgtgccaaaa tcaccgttcc ggtggacgca 2460
acggaagaac aggttcgcga acgtgctggc caggaacatc tggtagcaaa atatcttgat 2520
ggcgttactg tacgtaaagt gatttacgta ccaggtaaac tcctcaatct ggtcgttggc 2580
taa 2583

<210> 13
<211> 1518
<212> DNA
<213> E. coli

<220>
<223> lysS Lysine-tRNA synthetase, constitutive

<400> 13
atgtctgaac aacacgcaca gggcgctgac gcggtagtcg atcttaacaa tgaactgaaa 60
acgcgtcgtg agaagctggc gaacctgcgc gagcagggga ttgccttccc gaacgatttc 120
cgtcgcgatc atacctctga ccaattgcac gcagaattcg acggcaaaga gaacgaagaa 180
ctggaagcgc tgaacatcga agtcgccgtt gctggccgca tgatgacccg tcgtattatg 240
ggtaaagcgt ctttcgttac cctgcaggac gttggcggtc gcattcagct gtacgttgcc 300
cgtgacgatc tcccggaagg cgtttataac gagcagttca aaaaatggga cctcggcgac 360
atcctcggcg cgaaaggtaa gctgttcaaa accaaaaccg gcgaactgtc tatccactgc 420
accgagttgc gtctgctgac caaagcactg cgtccgctgc cggataaatt ccacggcttg 480
caggatcagg aagcgcgcta tcgtcagcgt tatctcgatc tcatctccaa cgatgaatcc 540
cgcaacacct ttaaagtgcg ctcgcagatc ctctctggta ttcgccagtt catggtgaac 600
cgcggcttta tggaagttga aacgccgatg atgcaggtga tccctggcgg tgccgctgcg 660
cgtccgttta tcacccacca taacgcgctg gatctcgaca tgtacctgcg tatcgcgccg 720
gaactgtacc tcaagcgtct ggtggttggt ggcttcgagc gtgtattcga aatcaaccgt 780
aacttccgta acgaaggtat ttccgtacgt cataacccag agttcaccat gatggaactc 840
tacatggctt acgcagatta caaagatctg atcgagctga ccgaatcgct gttccgtact 900
ctggcacagg atattctcgg taagacggaa gtgacctacg gcgacgtgac gctggacttc 960
ggtaaaccgt tcgaaaaact gaccatgcgt gaagcgatca agaaatatcg cccggaaacc 1020
gacatggcgg atctggacaa cttcgactct gcgaaagcaa ttgctgaatc tatcggcatc 1080
cacgttgaga agagctgggg tctgggccgt atcgttaccg agatcttcga agaagtggca 1140

gaagcacatc tgattcagcc gaccttcatt actgaatatc cggcagaagt ttctccgctg 1200
gcgcgtcgta acgacgttaa cccggaaatc acagaccgct ttgagttctt cattggtggt 1260
cgtgaaatcg gtaacggctt tagcgagctg aatgacgcgg aagatcaggc gcaacgcttc 1320
ctggatcagg ttgccgcgaa agacgcaggt gacgacgaag cgatgttcta cgatgaagat 1380
tacgtcaccg cactggaaca tggcttaccg ccgacagcag gtctgggaat tggtatcgac 1440
cgtatggtaa tgctgttcac caacagccat accatccgcg acgttattct gttcccggcg 1500
atgcgtccgg taaaataa 1518

<210> 14
<211> 1518
<212> DNA
<213> E. coli

<220>
<223> lysU Lysine-tRNA synthetase, heat inducible

<400> 14
atgtctgaac aagaaacacg gggagccaat gaggctattg attttaacga tgaactgaga 60
aatcgccgcg aaaaactggc ggcactacgt cagcaaggtg tggcgtttcc caatgatttt 120
cgccgcgacc atacctctga ccagttgcac gaagagtttg atgcgaagga taaccaggaa 180
ctggaatcct taaacattga agtctcggtt gctggccgaa tgatgacccg tcgtatcatg 240
gggaaagcct cctttgtaac gttgcaggat gtcggtggcc gtattcaact gtacgttgca 300
agagatagcc tgccagaagg tgtttataac gatcagttta aaaaatggga tctgggtgac 360
attatcggtg cccgcggtac gctgtttaag acgcaaacgg gtgagctttc cattcactgt 420
actgagctgc gcctgctgac taaagcacta cgtcctttac cagataaatt ccatggtctg 480
caggatcagg aagtccgtta tcgtcaacgt tatctggacc tcatcgctaa cgataaatcc 540
cgtcaaacgt ttgttgtccg ttcaaaaatt ctggccgcta tccgtcaatt catggtcgcg 600
cgcggcttta tggaagtaga aaccccgatg atgcaggtaa ttccaggtgg ggcatctgct 660
cgcccgttta ttacccatca taatgctctg gatttagata tgtatctgcg tatcgcgccg 720
gagctgtatc tgaaacgtct ggttgtaggc ggttttgaac gggtattcga aatcaaccgt 780
aacttccgta atgaaggtat ttctgttcgc cataatcctg agttcacaat gatggaactc 840
tacatggcgt atgcggatta ccacgatttg attgaactga cagagtcact gttccgcacc 900
ctggcacaag aggttctggg taccactaaa gtcacttatg gcgagcatgt gtttgatttc 960
ggcaaaccgt ttgaaaaact caccatgcgc gaagcaatca aaaaatatcg tccagaaacc 1020
gatatggccg acctggataa ttttgatgct gctaaagcat tagctgaatc tatcggtatt 1080
acggtagaga aaagctgggg gttgggacgt attgtcacag agatctttga tgaagtggca 1140
gaagcacatc tgattcagcc aacctttatt acggaatatc cggcagaagt gtccccgctg 1200
gcacgccgta atgatgttaa cccggaaatc accgaccgtt ttgaattctt catcggtggt 1260
cgtgaaatcg gtaatggttt tagcgaatta aacgacgcag aagatcaggc tgaacgtttc 1320
caggaacagg ttaatgctaa agctgcaggt gacgacgaag ccatgttcta tgacgaagat 1380
tacgtgactg cgctggaata tggtctgccg ccaaccgctg gtctgggtat tggtatcgac 1440
cgaatgatta tgctgtttac taacagccat actattcgcg acgttattct cttcccggcg 1500
atgcgcccac agaaataa 1518

<210> 15
<211> 2034
<212> DNA
<213> E. coli

<220>
<223> metG Methionine-tRNA synthetase

<400> 15
atgactcaag tcgcgaagaa aattctggtg acgtgcgcac tgccgtacgc taacggctca      60
atccacctcg gccatatgct ggagcacatc caggctgatg tctgggtccg ttaccagcga     120
atgcgcggcc acgaggtcaa cttcatctgc gccgacgatg cccacggtac accgatcatg     180
ctgaaagctc agcagcttgg tatcaccccg gagcagatga ttggcgaaat gagtcaggag     240
catcagactg atttcgcagg ctttaacatc agctatgaca actatcactc gacgcacagc     300
gaagagaacc gccagttgtc agaacttatc tactctcgcc tgaaagaaaa cggttttatt     360
aaaaaccgca ccatctctca gctgtacgat ccggaaaaag gcatgttcct gccggaccgt     420
tttgtgaaag gcacctgccc gaaatgtaaa tccccggatc aatacggcga taactgcgaa     480
gtctgcggcg cgacctacag cccgactgaa ctgatcgagc cgaaatcggt ggtttctggc     540
gctacgccgg taatgcgtga ttctgaacac ttcttctttg atctgccctc tttcagcgaa     600
atgttgcagg catggacccg cagcggtgcg ttgcaggagc aggtggcaaa taaaatgcag     660
gagtggtttg aatctggcct gcaacagtgg gatatctccc gcgacgcccc ttacttcggt     720
tttgaaattc cgaacgcgcc gggcaaatat ttctacgtct ggctggacgc accgattggc     780
tacatgggtt ctttcaagaa tctgtgcgac aagcgcggcg acagcgtaag cttcgatgaa     840
tactggaaga aagactccac cgccgagctg taccacttca tcggtaaaga tattgtttac     900
ttccacagcc tgttctggcc tgccatgctg gaaggcagca acttccgcaa gccgtccaac     960
ctgtttgttc atggctatgt gacggtgaac ggcgcaaaga tgtccaagtc tcgcggcacc    1020
tttattaaag ccagcacctg gctgaatcat tttgacgcag acagcctgcg ttactactac    1080
actgcgaaac tctcttcgcg cattgatgat atcgatctca acctggaaga tttcgttcag    1140
cgtgtgaatg ccgatatcgt taacaaagtg gttaacctgg cctcccgtaa tgcgggcttt    1200
atcaacaagc gttttgacgg cgtgctggca agcgaactgg ctgacccgca gttgtacaaa    1260
accttcactg atgccgctga agtgattggt gaagcgtggg aaagccgtga atttggtaaa    1320
gccgtgcgcg aaatcatggc gctggctgat ctggctaacc gctatgtcga tgaacaggct    1380
ccgtgggtgg tggcgaaaca ggaaggccgc gatgccgacc tgcaggcaat ttgctcaatg    1440
ggcatcaacc tgttccgcgt gctgatgact tacctgaagc cggtactgcc gaaactgacc    1500
gagcgtgcag aagcattcct caatacggaa ctgacctggg atggtatcca gcaaccgctg    1560
ctgggccaca aagtgaatcc gttcaaggcg ctgtataacc gcatcgatat gaggcaggtt    1620
gaagcactgg tggaagcctc taaagaagaa gtaaaagccg ctgccgcgcc ggtaactggc    1680
ccgctggcag atgatccgat tcaggaaacc atcacctttg acgacttcgc taaagttgac    1740
ctgcgcgtgg cgctgattga aaacgcagag tttgttgaag gttctgacaa actgctgcgc    1800
ctgacgctgg atctcggcgg tgaaaaacgc aatgtcttct ccggtattcg ttctgcttac    1860
ccggatccgc aggcactgat tggtcgtcac accattatgg tggctaacct ggcaccacgt    1920
aaaatgcgct tcggtatctc tgaaggcatg gtgatggctg ccggtcctgg cgggaaagat    1980
attttcctgc taagcccgga tgccggtgct aaaccgggtc atcaggtgaa ataa          2034

<210> 16
<211> 984
<212> DNA
<213> E. coli

<220>
<223> pheS Phenylalanine-tRNA synthetase, alpha-subunit

<400> 16
atgtcacatc tcgcagaact ggttgccagt gcgaaggcgg ccattagcca ggcgtcagat 60
gttgccgcgt tagataatgt gcgcgtcgaa tatttgggta aaaaagggca cttaaccctt 120
cagatgacga ccctgcgtga gctgccgcca gaagagcgtc cggcagctgg tgcggttatc 180
aacgaagcga aagagcaggt tcagcaggcg ctgaatgcgc gtaaagcgga actggaaagc 240
gctgcactga atgcgcgtct ggcggcggaa acgattgatg tctctctgcc aggtcgtcgc 300
attgaaaacg gcggtctgca tccggttacc cgtaccatcg accgtatcga aagtttcttc 360
ggtgagcttg gctttaccgt ggcaaccggg ccggaaatcg aagacgatta tcataacttc 420
gatgctctga acattcctgg tcaccacccg gcgcgcgctg accacgacac tttctggttt 480
gacactaccc gcctgctgcg tacccagacc tctggcgtac agatccgcac catgaaagcc 540
cagcagccac cgattcgtat catcgcgcct ggccgtgttt atcgtaacga ctacgaccag 600
actcacacgc cgatgttcca tcagatggaa ggtctgattg ttgataccaa catcagcttt 660
accaacctga aaggcacgct gcacgacttc ctgcgtaact tctttgagga agatttgcag 720
attcgcttcc gtccttccta cttcccgttt accgaacctt ctgcagaagt ggacgtcatg 780
ggtaaaaacg gtaaatggct ggaagtgctg ggctgcggga tggtgcatcc gaacgtgttg 840
cgtaacgttg gcatcgaccc ggaagtttac tctggtttcg ccttcgggat ggggatggag 900
cgtctgacta tgttgcgtta cggcgtcacc gacctgcgtt cattcttcga aaacgatctg 960
cgtttcctca aacagtttaa ataa 984

<210> 17
<211> 2388
<212> DNA
<213> E. coli

<220>
<223> pheT Phenylalanine-tRNA synthetase, beta-subunit

<400> 17
atgaaattca gtgaactgtg gttacgcgaa tgggtgaacc cggcgattga tagcgatgcg 60
ctggcaaatc aaatcactat ggcgggcctg gaagttgacg gtgtagaacc ggttgccggc 120
agcttccacg gcgtggtcgt tggtgaagtg gttgagtgtg cgcagcatcc gaacgctgac 180
aaactgcgtg tgacaaaagt gaatgtcggc ggcgatcgcc tgctggacat cgtctgcggt 240
gcgccaaact gccgtcaggg cctgcgtgta gcggtagcga ccattggtgc tgttctgccg 300
ggtgatttca aaattaaagc ggcgaaactg cgtggcgaac cgtctgaagg gatgctgtgc 360
tccttctctg aactgggcat ttctgacgat cacagcggca ttatcgaact gcctgcggat 420
gcgccgattg gcaccgatat ccgtgaatac ctgaaacttg atgacaacac catcgaaatc 480
agcgtgacgc caaaccgtgc cgactgctta ggcatcattg gtgttgcgcg tgacgttgcc 540
gtgctgaacc agctgccgct ggttcaaccg gaaatcgttc cggttggtgc gaccatcgac 600
gacacgctgc cgattacagt cgaagcgccg gaagcctgcc cgcgttatct tggccgtgtg 660
gtaaaaggca ttaacgttaa agcgccaact ccgctgtgga tgaaagaaaa actgcgtcgt 720
tgcgggatcc gttctatcga tgcagttgtt gacgtcacca actatgtgct gctcgaactg 780
ggccagccga tgcacgcttt cgataaagat cgcattgaag gcggcattgt ggtgcggatg 840
gcgaaagagg gcgaaacgct ggtgctgctc gacggtactg aagcgaagct gaatgctgac 900
actctggtca tcgccgacca caacaaggcg ctggcgatgg gcggcatctt cggtggcgaa 960
cactctggcg tgaatgacga aacacaaaac gtgctgctgg aatgcgcgtt ctttagcccg 1020
ctgtctatca ccggtcgtgc tcgtcgtcat ggcctgcata ccgatgcgtc tcaccgttat 1080
gagcgtggcg ttgatccggc actgcagcac aaagcgatgg aacgtgcgac ccgtctgctg 1140
atcgacatct gcggtggtga ggctggcccg gtaattgata tcaccaacga agcaacgctg 1200

ccgaagcgtg caaccatcac tctacgtcgt agcaaactgg atcgcctgat cggccatcat 1260
attgcggatg agcaggtaac tgacattctg cgtcgtctcg gctgcgaagt gaccgaaggc 1320
aaagacgaat ggcaggcagt tgcgccgagc tggcgtttcg atatggagat tgaagaagat 1380
ctggttgaag aagtcgcgcg tgtttacggc tacaacaaca tcccggatga gccggtacag 1440
gcaagcctga ttatgggtac tcaccgtgaa gctgacctgt cgctcaagcg cgtgaaaacg 1500
ctgctcaacg acaaaggcta tcaggaagtg atcacctaca gcttcgttga tccgaaagtg 1560
cagcagatga tccatccagg cgttgaagcc ttactgctgc caagcccgat ctctgttgaa 1620
atgtcagcaa tgcgtctttc tctgtggact ggcctgctgg caaccgtggt gtacaaccag 1680
aaccgtcagc agaaccgtgt gcgcattttc gaaagcggtc tgcgtttcgt accagatact 1740
caggcaccgt tgggcattcg tcaggatctg atgttagccg gtgtgatttg cggtaaccgt 1800
tacgaagagc actggaacct ggcaaaagag accgttgatt tctatgattt gaaaggcgat 1860
cttgaatccg ttctcgacct gaccggtaaa ctgaatgagg ttgagttccg tgcagaagcg 1920
aatccggcac tgcatccggg gcaatccgca gcgatttatc tgaaaggtga acgtattggt 1980
tttgttgggg ttgttcatcc tgaactggaa cgtaaactgg atcttaacgg tcgcactctg 2040
gtgttcgaac tggagtggaa caagctcgca gaccgcgtgg tgcctcaggc gcgcgagatt 2100
tctcgcttcc cggcgaaccg tcgtgacatc gcggtggtgg tcgcagaaaa cgttcccgca 2160
gcggatattt tatccgaatg taagaaagtt ggcgtaaatc aggtagttgg cgtaaactta 2220
tttgacgtgt accgcggtaa gggtgttgcg gaggggtata agagcctcgc cataagcctg 2280
atcctgcaag ataccagccg tacactcgaa gaagaggaga ttgccgctac cgtcgccaaa 2340
tgtgtagagg cattaaaaga gcgattccag gcatcattga gggattga 2388

<210> 18
<211> 978
<212> DNA
<213> E. coli

<220>
<223> lysyl-tRNA synthetase, genX

<400> 18
atgagcgaaa cggcatcctg gcagccgagc gcatccattc ctaacttatt aaaacgcgcg 60
gcgattatgg cggagatccg tcgtttcttt gccgatcgtg gagtgctgga ggtggagacg 120
ccttgtatga gccaggcgac ggtaaccgat attcatttgg tcccgtttga gacacgtttc 180
gttggccccg ggcattcgca ggggatgaat ctctggttaa tgaccagccc ggaataccat 240
atgaaacgcc tgctggttgc cggttgtggg ccggtattcc agctgtgccg cagcttccgt 300
aatgaagaga tggggcgtta tcacaaccct gagttcacta tgctggagtg gtatcgaccg 360
cactatgata tgtaccggtt gatgaacgag gtggacgatc tcttacaaca ggtgctggac 420
tgcccggcag cagaaagcct ttcttatcaa caagctttct tgcgttatct ggaaattgac 480
ccgctctctg ccgacaaaac gcaactgcgg gaagtcgcag cgaaactgga tttgagcaat 540
gttgctgata ccgaagaaga ccgcgacacg ctgctacaat tgctgtttac ctttggcgta 600
gagccaaata ttggcaaaga aaaaccgacc tttgtgtacc actttccagc cagccaggca 660
tcactggcgc aaatcagtac cgaagatcat cgggtcgctg aacgctttga ggtttattat 720
aaaggtattg agctggcgaa tggtttccat gaattgacgg atgcccgtga gcagcaacaa 780
cgctttgaac aagataaccg taagcgcgcg gcgcgcggtt tgccgcagca ccccattgac 840
cagaatctga ttgaagcctt gaaagtcggt atgcctgact gttccggcgt ggcattaggt 900
gttgatcgtc tggtgatgtt ggcgctgggc gcggagacac tggctgaagt catcgccttt 960
agcgttgacc gggcataa 978

<210> 19
<211> 1719
<212> DNA
<213> E. coli

<220>
<223> proS Proline-tRNA synthetase

<400> 19
atgcgtacta gccaatacct gctctccact ctcaaggaga cacctgccga cgccgaggtg 60
atcagccatc agctgatgct gcgcgccggg atgatccgca agctggcctc cgggttatat 120
acctggctgc cgaccggcgt gcgcgttctg aaaaaagtcg aaaacatcgt gcgtgaagag 180
atgaacaacg ccggtgcgat cgaggtgtcg atgccggtgg ttcagccagc cgatttgtgg 240
caagagagtg gtcgttggga acagtacggt ccggaactgc tgcgttttgt tgaccgtggc 300
gagcgtccgt tcgtactcgg cccaactcat gaagaagtta tcactgacct gattcgtaac 360
gagcttagct cttacaaaca gctgccgctg aacttctatc agatccagac caagttccgc 420
gacgaagtgc gtccgcgttt cggcgtcatg cgttcccgcg aattcctgat gaaagatgct 480
tactctttcc atacttctca ggaatccctg caggaaacct acgatgcaat gtatgcggcc 540
tacagcaaaa tcttcagccg catggggctg gatttccgcg ccgtacaagc cgacaccggt 600
tctatcggcg gcagcgcctc tcacgaattc caggtgctgg cgcagagcgg tgaagacgat 660
gtggtcttct ccgacacctc tgactatgca gcgaacattg aactggcaga agctatcgcg 720
ccgaaagaac cgcgcgctgc tgctacccag gaaatgacgc tggttgatac gccgaacgcg 780
aaaaccatcg cggaactggt tgaacagttc aatctgccga ttgagaaaac ggttaagact 840
ctgctggtta aagcggttga aggcagcagc ttcccgcagg ttgcgctgct ggtgcgcggt 900
gatcacgagc tgaacgaagt taaagcagaa aaactgccgc aggttgcaag cccgctgact 960
ttcgcgaccg aagaagaaat tcgtgccgtg gttaaagccg gtccgggttc actgggtccg 1020
gtaaacatgc cgattccggt ggtgattgac cgtaccgttg cggcgatgag tgatttcgct 1080
gctggtgcta acatcgatgg taaacactac ttcggcatca actgggatcg cgatgtcgct 1140
accccggaag ttgcagatat ccgtaacgtg gtggctggcg atccaagccc ggatggccag 1200
ggtaggctgc tgatcaaacg tggtatcgaa gttggtcaca tcttccagct gggtaccaag 1260
tactccgaag cactgaaagc ctccgtacag ggtgaagatg gccgtaacca aatcctgacg 1320
atgggttgct acggtatcgg ggtaacgcgt gtggtagctg cggcgattga gcagaactac 1380
gacgaacgag gcatcgtatg gcctgacgct atcgcgccgt tccaggtggc gattctgccg 1440
atgaacatgc acaaatcctt ccgcgtacaa gagcttgctg agaaactgta cagcgaactg 1500
cgtgcacaag gtatcgaagt gctgctggat gaccgcaaag agcgtccggg cgtgatgttt 1560
gctgatatgg aactgatcgg tattccgcac actattgtgc tgggcgaccg taacctcgac 1620
aacgacgata tcgaatataa atatcgtcgc aacggcgaga aacagttaat taagactggt 1680
gacatcgtcg aatatctggt gaaacagatt aaaggctga 1719

<210> 20
<211> 1293
<212> DNA
<213> E. coli

<220>
<223> serS Serine-tRNA synthetase

<400> 20

atgctcgatc ccaatctgct gcgtaatgag ccagacgcag tcgctgaaaa actggcacgc 60
cggggcttta agctggatgt agataagctg ggcgctcttg aagagcgtcg taaagtattg 120
caggtcaaaa cggaaaacct gcaagcggag cgtaactccc gatcgaaatc cattggccag 180
gcgaaagcgc gcggggaaga tatcgagcct ttacgtctgg aagtgaacaa actgggcgaa 240
gagctggatg cagcaaaagc cgagctggat gctttacagg ctgaaattcg cgatatcgcg 300
ctgaccatcc ctaacctgcc tgcagatgaa gtgccggtag gtaaagacga aaatgacaac 360
gttgaagtca gccgctgggg taccccgcgt gagtttgact ttgaagttcg tgaccacgtg 420
acgctgggtg aaatgcactc tggcctcgac tttgcagctg cagttaagct gactggttcc 480
cgctttgtgg taatgaaagg gcagattgct cgcatgcacc gcgcactgtc gcagtttatg 540
ctggatctgc ataccgaaca gcatggctac agtgagaact atgttccgta cctggttaac 600
caggacacgc tgtacggtac gggtcaactg ccgaaatttg ctggcgatct gttccatact 660
cgtccgctgg aagaagaagc agacaccagt aactatgcgc tgatcccaac ggcagaagtt 720
ccgctgacta acctggtgcg cggtgaaatc atcgatgaag atgatctgcc aattaagatg 780
accgcccaca ccccatgctt ccgttctgaa gccggttcat atggtcgtga cacccgtggt 840
ctgatccgta tgcaccagtt cgacaaagtt gaaatggtgc agatcgtgcg cccagaagac 900
tcaatggcgg cgctggaaga gatgactggt catgcagaaa aagtcctgca gttgctgggc 960
ctgccgtacc gtaaaatcat cctttgcact ggcgacatgg gctttggcgc ttgcaaaact 1020
tacgacctgg aagtatggat cccggcacag aacacctacc gtgagatctc ttcctgctcc 1080
aacgtttggg atttccaggc acgtcgtatg caggcacgtt gccgcagcaa gtcggacaag 1140
aaaacccgtc tggttcatac cctgaacggt tctggtctgg ctgttggtcg tacgctggtt 1200
gcagtaatgg aaaactatca gcaggctgat ggtcgtattg aagtaccaga agttctgcgt 1260
ccgtatatga acggactgga atatattggc taa 1293

<210> 21
<211> 1929
<212> DNA
<213> E. coli

<220>
<223> thrS Threonine-tRNA synthetase, autogenously regulated

<400> 21
atgcctgtta taactcttcc tgatggcagc caacgccatt acgatcacgc tgtaagcccc 60
atggatgttg cgctggacat tggtccaggt ctggcgaaag cctgtatcgc agggcgcgtt 120
aatggcgaac tggttgatgc ttgcgatctg attgaaaacg acgcacaact gtcgatcatt 180
accgccaaag acgaagaagg tctggagatc attcgtcact cctgtgcgca cctgttaggg 240
cacgcgatta aacaactttg gccgcatacc aaaatggcaa tcggcccggt tattgacaac 300
ggtttttatt acgacgttga tcttgaccgc acgttaaccc aggaagatgt cgaagcactc 360
gagaagcgga tgcatgagct tgctgagaaa aactacgacg tcattaagaa gaaagtcagc 420
tggcacgaag cgcgtgaaac tttcgccaac cgtggggaga gctacaaagt ctccattctt 480
gacgaaaaca tcgcccatga tgacaagcca ggtctgtact tccatgaaga atatgtcgat 540
atgtgccgcg gtccgcacgt accgaacatg cgtttctgcc atcatttcaa actaatgaaa 600
acggcagggg cttactggcg tggcgacagc aacaacaaaa tgttgcaacg tatttacggt 660
acggcgtggg cagacaaaaa agcacttaac gcttacctgc agcgcctgga agaagccgcg 720
aaacgcgacc accgtaaaat cggtaaacag ctcgacctgt accatatgca ggaagaagcg 780
ccgggtatgg tattctggca caacgacggc tggaccatct tccgtgaact ggaagtgttt 840
gttcgttcta aactgaaaga gtaccagtat caggaagtta aaggtccgtt catgatggac 900
cgtgtcctgt gggaaaaaac cggtcactgg gacaactaca aagatgcaat gttcaccaca 960

tcttctgaga accgtgaata ctgcattaag ccgatgaact gcccgggtca cgtacaaatt 1020
ttcaaccagg ggctgaagtc ttatcgcgat ctgccgctgc gtatggccga gtttggtagc 1080
tgccaccgta acgagccgtc aggttcgctg catggcctga tgcgcgtgcg tggatttacc 1140
caggatgacg cgcatatctt ctgtactgaa gaacaaattc gcgatgaagt taacggatgt 1200
atccgtttag tctatgatat gtacagcact tttggcttcg agaagatcgt cgtcaaactc 1260
tccactcgtc ctgaaaaacg tattggcagc gacgaaatgt gggatcgtgc tgaggcggac 1320
ctggcggttg cgctggaaga aaacaacatc ccgtttgaat atcaactggg tgaaggcgct 1380
ttctacggtc cgaaaattga atttaccctg tatgactgcc tcgatcgtgc atggcagtgc 1440
ggtacagtac agctggactt ctctttgccg tctcgtctga gcgcttctta tgtaggcgaa 1500
gacaatgaac gtaaagtacc ggtaatgatt caccgcgcaa ttctggggtc gatggaacgt 1560
ttcatcggta tcctgaccga agagttcgct ggtttcttcc cgacctggct tgcgccggtt 1620
caggttgtta tcatgaatat taccgattca cagtctgaat acgttaacga attgacgcaa 1680
aaactatcaa atgcgggcat tcgtgttaaa gcagacttga gaaatgagaa gattggcttt 1740
aaaatccgcg agcacacttt gcgtcgcgtc ccatatatgc tggtctgtgg tgataaagag 1800
gtggaatcag gcaaagttgc cgttcgcacc cgccgtggta aagacctggg aagcatggac 1860
gtaaatgaag tgatcgagaa gctgcaacaa gagattcgca gccgcagtct taaacaattg 1920
gaggaataa 1929

<210> 22
<211> 1005
<212> DNA
<213> E. coli

<220>
<223> trpS Tryptophan-tRNA synthetase

<400> 22
atgactaagc ccatcgtttt tagtggcgca cagccctcag gtgaattgac cattggtaac 60
tacatgggtg cgctgcgtca gtgggtaaac atgcaggatg actaccattg catttactgt 120
atcgttgacc aacacgcgat caccgtgcgc caggatgcac agaagctgcg taaagcgacg 180
ctggatacgc tggccttgta tctggcttgt ggtatcgatc ctgagaaaag caccattttt 240
gttcagtccc acgtgccgga acatgcacag ttaggctggg cactgaactg ctatacctac 300
ttcggcgaac tgagtcgcat gacgcagttt aaagataaat ctgcgcgtta tgccgagaac 360
atcaacgctg gtctgtttga ctatccggtg ctgatggcag cggacatcct gctgtatcaa 420
actaatctgg taccggtggg tgaagaccag aaacagcacc tcgaactgag ccgcgatatt 480
gcccagcgtt tcaacgcgct gtatggcgag atctttaagg tgccggagcc gtttattccg 540
aaatctggcg cgcgcgtaat gtcgctgctg gagccgacca agaagatgtc caagtctgac 600
gataatcgca ataacgttat cggcctgctg gaagatccga aatcggtagt gaagaaaatc 660
aaacgtgcgg tcactgactc cgacgagccg ccggtagttc gctacgatgt gcagaacaaa 720
gcgggcgttt ccaacctgtt ggatatcctt tcagcggtaa cgggccagag catcccagaa 780
ctggaaaaac agttcgaagg caagatgtat ggtcatctga aaggtgaagt ggctgatgcc 840
gtttccggta tgctgactga attgcaggaa cgctatcacc gtttccgcaa cgatgaagcc 900
ttcctgcaac aggtgatgaa agatggcgcg gaaaaagcca gcgcgcacgc ttcccgtacg 960
ctaaaagcgg tgtacgaagc gattggtttt gtggcgaagc cgtaa 1005

<210> 23
<211> 1275

<212> DNA
<213> E. coli

<220>
<223> tyrS Tyrosine-tRNA synthetase

<400> 23
atggcaagca gtaacttgat taaacaattg caagagcggg ggctggtagc ccaggtgacg 60
gacgaggaag cgttagcaga gcgactggcg caaggcccga tcgcgctcta ttgcggcttc 120
gatcctaccg ctgacagctt gcatttgggg catcttgttc cattgttatg cctgaaacgc 180
ttccagcagg cgggccacaa gccggttgcg ctggtaggcg gcgcgacggg tctgattggc 240
gacccgagct tcaaagctgc cgagcgtaag ctgaacaccg aagaaactgt tcaggagtgg 300
gtggacaaaa tccgtaagca ggttgccccg ttcctcgatt tcgactgtgg agaaaactct 360
gctatcgcgg cgaacaacta tgactggttc ggcaatatga atgtgctgac cttcctgcgc 420
gatattggca aacacttctc cgttaaccag atgatcaaca aagaagcggt taagcagcgt 480
ctcaaccgtg aagatcaggg gatttcgttc actgagtttt cctacaacct gttgcagggt 540
tatgacttcg cctgtctgaa caaacagtac ggtgtggtgc tgcaaattgg tggttctgac 600
cagtggggta acatcacttc tggtatcgac ctgacccgtc gtctgcatca gaatcaggtg 660
tttggcctga ccgttccgct gatcactaaa gcagatggca ccaaatttgg taaaactgaa 720
ggcggcgcag tctggttgga tccgaagaaa accagcccgt acaaattcta ccagttctgg 780
atcaacactg cggatgccga cgtttaccgc ttcctgaagt tcttcacctt tatgagcatt 840
gaagagatca acgccctgga agaagaagat aaaaacagcg gtaaagcacc gcgcgcccag 900
tatgtactgg cggagcaggt gactcgtctg gttcacggtg aagaaggttt acaggcggca 960
aaacgtatta ccgaatgcct gttcagcggt tctttgagtg cgctgagtga agcggacttc 1020
gaacagctgg cgcaggacgg cgtaccgatg gttgagatgg aaaagggcgc agacctgatg 1080
caggcactgg tcgattctga actgcaacct tcccgtggtc aggcacgtaa aactatcgcc 1140
tccaatgcca tcaccattaa cggtgaaaaa cagtccgatc ctgaatactt ctttaaagaa 1200
gaagatcgtc tgtttggtcg ttttacctta ctgcgtcgcg gtaaaaagaa ttactgtctg 1260
atttgctgga aataa 1275

<210> 24
<211> 2856
<212> DNA
<213> E. coli

<220>
<223> valS Valine-tRNA synthetase

<400> 24
atggaaaaga catataaccc acaagatatc gaacagccgc tttacgagca ctgggaaaag 60
cagggctact ttaagcctaa tggcgatgaa agccaggaaa gtttctgcat catgatcccg 120
ccgccgaacg tcaccggcag tttgcatatg ggtcacgcct tccagcaaac catcatggat 180
accatgatcc gctatcagcg catgcagggc aaaaacaccc tgtggcaggt cggtactgac 240
cacgccggga tcgctaccca gatggtcgtt gagcgcaaga ttgccgcaga agaaggtaaa 300
acccgtcacg actacggccg cgaagctttc atcgacaaaa tctgggaatg gaaagcggaa 360
tctggcggca ccattacccg tcagatgcgc cgtctcggca actccgtcga ctgggagcgt 420
gaacgcttca ccatggacga aggcctgtcc aatgcggtga aagaagtttt cgttcgtctg 480

tataaagaag acctgattta ccgtggcaaa cgcctggtaa actgggatcc gaaactgcgc 540
accgctatct ctgacctgga agtcgaaaac cgcgaatcga aaggttcgat gtggcacatc 600
cgctatccgc tggctgacgg tgcgaaaacc gcagacggta aagattatct ggtggtcgcg 660
actacccgtc cagaaaccct gctgggcgat actggcgtag ccgttaaccc ggaagatccg 720
cgttacaaag atctgattgg caaatatgtc attctgccgc tggttaaccg tcgtattccg 780
atcgttggcg acgaacacgc cgacatggaa aaaggcaccg gctgcgtgaa aatcactccg 840
gcgcacgact ttaacgacta tgaagtgggt aaacgtcacg ccctgccgat gatcaacatc 900
ctgacctttg acggcgatat ccgtgaaagc gcccaggtgt tcgataccaa aggtaacgaa 960
tctgacgttt attccagcga aatccctgca gagttccaga aactggagcg ttttgctgca 1020
cgtaaagcag tcgttgccgc agttgacgcg cttggcctgc tggaagaaat taaaccgcac 1080
gacctgaccg ttccttacgg cgaccgtggc ggcgtagtta tcgaaccaat gctgaccgac 1140
cagtggtacg tgcgtgccga tgtcctggcg aaaccggcgg ttgaagcggt tgagaacggc 1200
gacattcagt tcgtaccgaa gcagtacgaa aacatgtact tctcctggat gcgcgatatt 1260
caggactggt gtatctctcg tcagttgtgg tggggtcacc gtatcccggc atggtatgac 1320
gaagcgggta acgtttatgt tggccgcaac gaagacgaag tgcgtaaaga aaataacctc 1380
ggtgctgatg ttgtcctgcg tcaggacgaa gacgttctcg atacctggtt ctcttctgcg 1440
ctgtggacct tctctaccct tggctggccg gaaaataccg acgccctgcg tcagttccac 1500
ccaaccagcg tgatggtatc tggtttcgac atcattttct tctggattgc ccgcatgatc 1560
atgatgacca tgcacttcat caaagatgaa aatggcaaac cgcaggtgcc gttccacacc 1620
gtttacatga ccggcctgat tcgtgatgac gaaggccaga agatgtccaa atccaagggt 1680
aacgttatcg acccactgga tatggttgac ggtatttcgc tgccagaact gctggaaaaa 1740
cgtaccggca atatgatgca gccgcagctg gcggacaaaa tccgtaagcg caccgagaag 1800
cagttcccga acggtattga gccgcacggt actgacgcgc tgcgcttcac cctggcggcg 1860
ctggcgtcta ccggtcgtga catcaactgg gatatgaagc gtctggaagg ttaccgtaac 1920
ttctgtaaca agctgtggaa cgccagccgc tttgtgctga tgaacacaga aggtcaggat 1980
tgcggcttca acggcggcga aatgacgctg tcgctggcgg accgctggat tctggcggag 2040
ttcaaccaga ccatcaaagc gtaccgcgaa gcgctggaca gcttccgctt cgatatcgcc 2100
gcaggcattc tgtatgagtt cacctggaac cagttctgtg actggtatct cgagctgacc 2160
aagccggtaa tgaacggtgg caccgaagca gaactgcgcg gtactcgcca tacgctggtg 2220
actgtactgg aaggtctgct gcgcctcgcg catccgatca ttccgttcat caccgaaacc 2280
atctggcagc gtgtgaaagt actttgcggt atcactgccg acaccatcat gctgcagccg 2340
ttcccgcagt acgatgcatc tcaggttgat gaagccgcac tggccgacac cgaatggctg 2400
aaacaggcga tcgttgcggt acgtaacatc cgtgcagaaa tgaacatcgc gccgggcaaa 2460
ccgctggagc tgctgctgcg tggttgcagc gcggatgcag aacgtcgcgt aaatgaaaac 2520
cgtggcttcc tgcaaaccct ggcgcgtctg gaaagtatca ccgtgctgcc tgccgatgac 2580
aaaggtccgg tttccgttac gaagatcatc gacggtgcag agctgctgat cccgatggct 2640
ggcctcatca acaaagaaga tgagctggcg cgtctggcga aagaagtggc gaagattgaa 2700
ggtgaaatca gccgtatcga gaacaaactg gcgaacgaag gctttgtcgc ccgcgcaccg 2760
gaagcggtca tcgcgaaaga gcgtgagaag ctggaaggct atgcggaagc gaaagcgaaa 2820
ctgattgaac agcaggctgt tatcgccgcg ctgtaa 2856

<210> 25
<211> 2634
<212> DNA
<213> B. subtilis

<220>
<223> alanyl-tRNA synthetase

<400> 25
atgaaacact taacttctgc ggaagtgcgt caaatgtttt tggatttctt taaagaaaaa 60
ggacatgcgg tagagccaag cgcgtcatta gtgcctcatg aggatccttc actgctttgg 120
atcaacagcg gtgttgcgac gctgaaaaaa tattttgacg gccgtgtcgt gccggaaaat 180
ccaagaatcg taaacgctca aaaagcgatc agaacaaacg atatagaaaa tgtaggtaaa 240
actgcgcgcc atcacacttt ctttgaaatg ctcggaaact tttccatcgg cgattatttc 300
aaagaagaag ccattacatg ggcttgggag tttttaacga gcgacaagtg gattggcttc 360
gacaaagagc ttctctctgt tacggttcat cctgaagatg aagaggcata tgagttttgg 420
gcgaaaaaaa tcggtattcc tgaagaaaga attatccgtc tggaagggaa cttctgggat 480
atcggtgaag ggccgagcgg accgaatacg gaaatctttt acgaccgcgg tgaagcatac 540
ggtaatgatc cagaagatcc ggagctttac ccaggcgggg aaaacgaccg ttacctggaa 600
gtatggaacc ttgtgttctc agagttcaac cataaccctg acggcacgta cacaccgctt 660
ccaaagaaaa atattgatac aggcatgggt cttgaaagaa tggtgtctgt catccagaat 720
gttccgacaa actttgacac cgatttgttt gttccgatca ttaaagcaac agaatcgatt 780
tctggtgaaa catatggcaa agacaatgtg aaagacactg cgtttaaagt gattgctgac 840
catatcagaa cggttgcctt tgctgtcagt gacggtgcgc tgccgtcaaa tgaaggccgc 900
ggctatgtat taagacgctt attacgccgt gccgtgcgtt atgccaaaac gatcaacatc 960
catcgtccgt tcatgtttga tttagtgccg gttgtcgcag aaatcatggc tgatttctat 1020
cctgaagtga aagagaaagc ggatttcatc gcaaaagtca ttaaaaccga ggaagaacgc 1080
ttccatgaga cccttaatga agggcttgcg atcctgtcag aaatgatcaa aaaagaaaaa 1140
gacaagggca gcagcgttat ctcaggtgct gatgtgttta aactgtatga tacgtatgga 1200
ttcccggttg aattgactga agaatacgca gaagacgaga acatgacggt tgaccatgag 1260
ggctttgagg aggaaatgaa ccagcagcgt gaacgggcaa gaaacgcccg ccaggatgtc 1320
ggcagcatgc aggtgcaggg cggcgcactg cgcgacgtga cagttgaaag cacatttgtc 1380
ggctactctc aaaccaaagc tgatgccaat atcattgtgc ttcttcagga cggccaatta 1440
atcgaagagg ctcatgaagg agaaagcgtt caaatcattc ttgatgaaac accgttctat 1500
gcagaaagcg gcggccaaat cggcgacaaa ggctatctcc gaagcgagca ggcagttgta 1560
agaattaaag atgttcaaaa agcgccgaac ggccagcatg tgcatgaagg tgttgtggag 1620
agcggtactg ttcaaaaagg cctgcatgtt acggctgaag ttgaagacca tatgagaagc 1680
ggcgtcatta aaaaccacac ggcaacgcat ttattgcatc aggctctaaa agatgttctt 1740
ggaactcatg tcaatcaggc gggctctctt gtaacggaaa accgccttcg ttttgacttc 1800
tcgcactttg gccaagtgac aaaagaagag ctcgaacaaa ttgaaagaat cgtaaacgaa 1860
aagatctggg cgagcatccc ggtcagcatt gatttgaaac cgatcgctga agcaaaagaa 1920
atgggtgcga tggcactgtt cggcgaaaaa tatggcgata ttgtccgtgt cgttcaagtt 1980
ggagattaca gcttagagct gtgcggcggc tgccacgtca gaaatacagc agaaatcggc 2040
ttgtttaaaa tcgtttctga atccggaatc ggagcaggca caagacggat tgaagctgta 2100
acgggacaag gcgcttacgt cgaaatgaac agccagattt ctgtattgaa gcagaccgct 2160
gacgagctga aaacaaatat caaagaagtg ccgaaacggg tcgcggctct gcaggctgaa 2220
ctgaaggatg cacaaagaga aaatgaatct cttcttgcaa aactaggcaa cgtggaagca 2280
ggagcaatcc tgtcgaaagt aaaagaagtt gacggcgtaa acgtgcttgc ggcaaaagta 2340
aatgcaaaag acatgaatca tctccgcact atggtcgatg aactcaaagc aaagcttggc 2400
tctgcggtga tcgtgcttgg tgcggtacaa aacgataaag ttaatatctc tgccggagtc 2460
acaaaggacc tcattgagaa aggacttcac gccggcaagc tggttaaaca agctgcggaa 2520
gtttgcggcg gaggcggcgg aggccgtccg gacatggcgc aggcaggcgg taaacagccg 2580
gaaaaattag aagaagcttt ggcttctgta gaagattggg tgaaatccgt ttta 2634

<210> 26

<211> 1668
<212> DNA
<213> B. subtilis

<220>
<223> arginyl-tRNA synthetase

<400> 26
atgaacattg cggaacaaat gaaggacgtg ctgaaagaag aaatcaaagc ggccgttctg 60
aaagcgggac tggctgaaga aagccagatt cccaatgttg ttttagaaac accgaaagat 120
aaaacacacg gcgactactc aacgaatatg gcgatgcagt tggcaagagt ggcaaaaaaa 180
gcgccgcgtc aaatcgctga agagattgtc gcccattttg ataaagggaa ggcttcgatt 240
gaaaaactgg atatcgccgg cccgggtttt atcaatttct atatgaacaa tcaatattta 300
acaaagctga ttccgtctgt actggaagca ggggaagcat acggggaaac gaacatcgga 360
aacggtgaac gagttcaggt agaattcgta tcagcgaatc cgacgggaga ccttcacctc 420
gggcatgcgc gcggggcggc tgtcggcgat tccttgtgca acgtgctttc aaaagcgggt 480
tacgatgtaa gccgcgaata ctacattaac gatgcgggaa accaaatcaa caatctggcg 540
ctttctgttg aagtccgtta ctttgaagca ctcggattgg aaaaaccgat gccggaggat 600
ggctaccgcg gtgaggatat cattgcgatc ggaaagcgtc tcgctgagga atacggtgat 660
cgtttcgtta acgaagagga aagtgaacgc ctcgcgtttt tccgtgaata cggcctgaaa 720
tacgagctgg aaaagcttcg caaggacttg gaaaacttcc gtgtgccgtt tgatgtgtgg 780
tattcagaaa cttcactgta ccaaaatgga aaaatcgaca aggcgcttga agcgctccgc 840
gaaaaaggcc acgtctatga agaagacggc gcgacttggt tccgttccac gactttcggc 900
gatgataaag accgcgtatt gatcaagaag gacggcacgt acacgtacct tcttcctgat 960
atcgcgtatc ataaggacaa gcttgaccgc ggctttgata agctgatcaa cgtttggggc 1020
gccgatcacc acggctacat cccgcgtatg aaagcggcaa tcgaagcgct gggctatgaa 1080
aaaggaacgc ttgaagtaga aatcattcag ctcgttcacc tttacaaaaa cggcgagaaa 1140
atgaaaatga gcaaacggac cggtaaagcc gtaacgatgc gtgacctgat tgaagaagtc 1200
ggcttggatg ccgtgcgtta cttctttgca atgcgaagtg cagacaccca tatggatttt 1260
gacttagatc ttgctgtatc aacatctaac gaaaaccctg tgtattatgc acaatacgcg 1320
catgcccgta tttgcagcat gcttcgccaa ggggaagagc aggggcttaa accggcggca 1380
gatcttgatt tcagccatat tcaatcagaa aaagaatacg atctgctgaa aacgatcggc 1440
ggcttcccag aggcagtggc ggaagcagcg gaaaaacgaa ttccgcaccg tgtcactaac 1500
tatatttatg atctggcttc tgctctgcac agcttctaca acgcagagaa agtcatcgac 1560
cctgaaaatg aagagaaaag ccgcgcgcgc ctggctttaa tgaaggcaac gcaaattacg 1620
ctgaacaatg cgcttcagct gatcggcgta tcggctccgg aaaaaatg 1668

<210> 27
<211> 1290
<212> DNA
<213> B. subtilis

<220>
<223> asparaginyl tRNA synthetase

<400> 27
ttgaaaacaa caatcaacca agtgtacaag cacgtaggtg aggaagtaac gatcggagct 60
tgggtcgcta ataagcgttc aagcgggaaa attgcgtttt tacagcttcg ggacggtacc 120

ggttttattc agggtgtcgt agtaaaagcg gaagtggaag aaagcatttt ccaaacagct 180
aaatcagtga cgcaggaaac gtcgctctat ataaaaggga ttgtcaaaga ggacgagcgt 240
tctccgcttg gatatgaact tgctgtgaca gatattgaag tcattcacga agcgaccgat 300
tatccaatta caccaaaaga acacggaacg gaatttttga tggatcacag acatttatgg 360
ctgcgttcaa agcgccagca tgcgatcatg aaaatccgta atgaaatcat tcgcgcgact 420
tacgaattct ttaataacga aggcttcgta aaagtggatc cgccgatttt gactggaagc 480
gcacctgaag gaacaacaga actctttgcg acaaagtact ttgatgaaga tgcatatctg 540
tctcaaagcg gacagctcta catggaagct gcggcaatgg ctttaggaaa agtattctct 600
ttcggaccga cattcagagc ggaaaagtct aaaacaaagc gtcacttaat cgaattctgg 660
atgatcgaac cggaaatggc gtttgtagaa tttgaagaaa accttcaagt acaggaaaat 720
tacgtttctt tcatcgtgca atcagttctt aaaaattgca aaattgaact aaacacattg 780
ggaagagaca cgtcaaaact tgagcaaatc aaagccccgt tcccaagaat tacgtatgat 840
gaagcgattg aatttctgaa agaaaaaggc tttgacgata tcgagtgggg agatgatttc 900
ggagcgcctc atgaaacagc gattgctgaa cattatgaca aaccggtatt catcactcgc 960
tatccgacgt ccttaaaacc gttctatatg cagccggctt ctgaccgtga ggacgttgtg 1020
ctttgcgctg acttgattgc gccggaaggc tatggagaaa tcatcggcgg gtctgaacgg 1080
attcacgata tggaactttt ggaatcgcgt cttaaagaac atggactgga ttctgacgct 1140
tataaatggt atgctgaact tagaaaatat ggatcagttc ctcattccgg cttcggcctt 1200
ggattagagc ggacagtagc ttggatcagc ggagcgcctc acgttcgtga aacgattccg 1260
ttcccaagac tgttaaaccg tctgtatccg 1290

<210> 28
<211> 1776
<212> DNA
<213> B. subtilis

<220>
<223> aspartyl-tRNA synthetase

<400> 28
ttgtttggaa gaacatatta ttgcggtgat ataactgaaa aagcaattgg cgaatctgta 60
acgctgaaag gctgggtcca aaaaagacga gacctcggcg gattgatttt tattgacttg 120
cgtgaccgta cgggcattgt tcaagtcgtt tttaaccctg atgtgtcaaa agaagcgctt 180
gctattgcgg aaggcatcag aaatgaatac gtgcttgata ttcaaggaaa agtggtggcg 240
cgtgaagagg gaacagttaa tccgaatttg aaaacaggcg cgatcgaaat acatgctgat 300
ggagtgaacg tattaaatgc tgcaaagaca cctccatttg cgatttctga tcaagctgaa 360
gaagtgtcag aggacgttcg tttaaaacac cgttacttag atttgcgcag accggctatg 420
ttccagacga tgcagctgcg ccataacgtg acgaaagctg ttcgcagctt tttagatgaa 480
aatgggtttc ttgatattga aacgccgatt ttaacaggaa gcacgcctga aggcgcacgt 540
gactacttgg tgccgagccg tgtgcacgaa ggtgagttct acgcgctgcc gcagtctccg 600
cagctattta aacagcttct gatggtatca ggcattgaac gatattatca aattgcccgc 660
tgtttccgcg atgaagactt gcgtgcggac cgtcagcctg agtttacgca aattgatatt 720
gaaatgtcct ttatgagcca ggaagacatt atgtcattgg ctgaggagat gatggcaaag 780
gttatgcgtg aaacaaaagg tgaggaactt cagcttcctc ttcctcgaat gacgtatgac 840
gaagcaatga acaagtacgg gtctgataaa ccggatacgc gttttgacat gcttttgacg 900
gatgtgtctg acattgtaaa ggatacggaa tttaaagtgt tctcatcagc tgtagcaaac 960
ggcggcgtgg tcaaagccat caatgtaaaa ggcggtgccg gcgattactc cagaaaagac 1020
atcgacgctc ttggcgcttt tgctgcaaac tacggggcaa aagggcttgc ctgggtgaag 1080

gttgaggcag acggagtgaa aggcccgatc gctaaattct ttgatgaaga aaagcagtct 1140
aagctgattg aagcacttga tgctgctgaa ggtgatttgc tgctgttcgg agcggatcaa 1200
ttcgaagttg tggccgcatc actcggcgcc ctgcgcttaa agcttgggaa agaacgcgga 1260
ctgattgacg aaaaattgtt caatttctta tgggtcatcg actggccgct attagagcat 1320
gatccggaag aaggccgttt ttacgcagcg caccatccgt tcacaatgcc tgtccgagaa 1380
gaccttgagc tgattgaaac agcgcctgaa gacatgaaag cgcaagctta tgacctcgtc 1440
ttaaacggct atgagcttgg cggcggttca atccgtattt tcgaaaagga tattcaggaa 1500
aaaatgtttg cgcttctcgg cttctcacca gaagaagcgg ctgaacagtt cggattcctg 1560
ctggaagcat ttgaatacgg cgctcctccg cacggcggaa tcgcactcgg cttagaccgt 1620
ctcgttatgc ttctcgctgg acgtacgaac ctgagagata cgattgcatt cccgaaaacg 1680
gcaagtgcaa gctgcctgat gacagaagcg ccaggcgaag taagcgacgc ccagcttgac 1740
gagcttcatc tttctatcaa gaaaaaagtt aagaac 1776

<210> 29
<211> 1398
<212> DNA
<213> B. subtilis

<220>
<223> cysteinyl-tRNAsynthetase

<400> 29
atgacaatca cactttataa tacattgact agacagaagg aaacattcgt tcctcttgaa 60
gagggaaaag tgaaaatgta tgtatgcgga cccacggttt acaattacat tcatatcggg 120
aacgcgcgtc cggcaatcgt ttacgatacg gttcgaaact atttagagta taaaggctat 180
gatgtgcagt atgtctctaa cttcacagac gtagacgata aattaattaa agcggcaaat 240
gaactcggtg aggatgtacc caccatttca gagcgtttta ttaaagcata ctttgaagac 300
gtaggtgcgc tcggctgccg aaaagccgac cttcatccgc gagtaatgga gaacatggat 360
gccattatcg aattcgtaga tcagctcgtg aaaaagggct acgcatatga atcagaaggt 420
gacgtatatt tcaaaaccag agcatttgaa gggtacggaa agctttctca gcaatcaatc 480
gatgaactaa gatcaggtgc acgcatccgg gtcggcgaga aaaaagaaga tgctcttgat 540
ttcgcactgt ggaaagcggc aaaagaagga gaaatctctt gggatagccc ttgggggaaa 600
gggcgtccgg gctggcacat tgaatgctca gcaatggtga aaaagtatct cggtgaccag 660
attgatatcc atgcgggcgg acaggattta acattccctc accatgaaaa cgaaattgcg 720
caatctgagg cgctgacagg caaaacgttt gcgaagtact ggcttcataa tggttatatc 780
aatattgata atgaaaaaat gtcaaaatca ctaggcaact ttgtgcttgt gcatgacatc 840
attaaacagc atgatccgca gcttttgaga ttctttatgc tatctgttca ttatcgccat 900
ccgattaact attcagaaga gcttctggag aatacgaaaa gcgcgttcag ccgtttaaaa 960
acagcgtaca gcaatcttca gcaccgtctg aacagcagta cgaatttaac cgaagatgac 1020
gatcaatggc ttgaaaaggt tgaagaacac cgcaaagcat tcgaagaaga gatggacgat 1080
gattttaata cggcgaatgc catttcagtc ttgtttgact tagcgaaaca cgccaattat 1140
tatcttcaga aagatcatac ggctgatcat gtgattacgg cgtttattga gatgtttgac 1200
cgcattgttt ctgtcctcgg tttttcgttg ggtgagcagg aacttctcga tcaagagatt 1260
gaagacttaa tcgaaaagcg aaatgaagcg cgccggaatc gcgattttgc attgtcagac 1320
cagatccgcg accagctgaa aagcatgaat atcattcttg aagatacggc tcaaggcact 1380
cgctggaaac ggggagaa 1398

<210> 30
<211> 1449
<212> DNA
<213> B. subtilis

<220>
<223> glutamyl-tRNA synthetase

<400> 30
atgggaaacg aagtacgcgt ccgttatgca ccgagtccaa ccggacattt gcatattgga 60
aatgccagaa cggcgctttt taattattta tttgcccgca atcaaggcgg taagtttatc 120
attcgagttg aggatactga taaaaagcgc aatattgagg gcggagaaca aagccagctg 180
aattatctga agtggctagg tattgactgg gatgagagtg tggatgtcgg aggagagtac 240
ggtccatacc gtcagtcaga gcgtaacgat atctataaag tgtactatga agagcttctt 300
gaaaaagggc ttgcttataa atgttactgt acggaagaag agcttgaaaa agagcgtgaa 360
gaacagattg cccgcggaga aatgcctcgt tattccggaa aacacagaga cctgactcag 420
gaagaacagg agaaatttat cgccgaaggc agaaaaccaa gtattcgttt ccgtgtgccg 480
gaaggaaaag tcatcgcctt caacgacatc gtaaaaggcg aaatttcttt tgaatcagat 540
ggcatcggcg acttcgttat tgtgaaaaag gacggaacgc ctacttataa cttcgcggta 600
gctattgatg actacttaat gaaaatgaca cacgtgctgc gcggtgagga tcatatttct 660
aacacaccga aacagattat gatctatcaa gcattcgggt gggatattcc tcagttcgga 720
cacatgacgc tgattgtaaa cgaaagccgt aaaaagctca gcaaacgtga tgaatccatt 780
attcaattca tcgagcagta caaagagctt ggctacctgc cagaagcgct gttcaacttt 840
atcggcttgt taggctggtc accggttgga gaagaagagc ttttcacaaa agagcagttt 900
attgaaattt ttgatgtaaa tcgtttatct aaatcaccag ctttgtttga tatgcataag 960
ctaaaatggg ttaacaacca atatgtgaag aagctggatc ttgatcaggt tgttgaactg 1020
acgcttccgc atttgcaaaa agccggcaaa gttggcactg agctttctgc tgaagaacaa 1080
gaatgggttc gtaaactgat ttccctgtat catgagcaat taagctacgg tgcggaaatt 1140
gttgagctga ctgatttgtt ctttacggat gagatcgagt ataatcaaga agcgaaagct 1200
gttctggaag aagaacaggt tcctgaagtg ctcagcacat tcgcagcgaa gcttgaagag 1260
cttgaggagt tcactccgga taatatcaaa gcatcgatca aagcagtgca gaaagaaact 1320
ggccataaag ggaaaaaact gtttatgccg attcgtgttg ctgtaacagg gcaaactcac 1380
ggtccggaac tgccgcaatc aattgaattg atcggtaaag agactgcaat tcagcgttta 1440
aagaatatc 1449

<210> 31
<211> 1458
<212> DNA
<213> B. subtilis

<220>
<223> glutamyl-tRNA amidotransferase, subumit a

<400> 31
atgtcattat ttgatcataa aatcacagaa ttaaaacagc tcatacataa aaaagagatt 60
aagatttctg atctggttga tgaatcttat aaacgcatcc aagcggttga tgataaggta 120
caagcctttt tggcattaga tgaagaaaga gcgcgcgcat acgcgaagga gcttgatgag 180
gcggttgacg gccgttctga gcacggtctt cttttcggta tgccgatcgg cgtaaaagat 240

aatatcgtaa caaaagggct gcgcacaaca tgctccagca aaattctcga aaactttgat 300
ccgatttacg atgctactgt cgttcagcgc cttcaagacg ctgaagcggt cacaatcgga 360
aaactgaaca tggacgaatt cgccatgggg tcatctacag aaaactcagc ttacaagctg 420
acgaaaaacc cttggaacct ggatacagtt cccggcggtt caagcggcgg atctgcagct 480
gcggttgctg cgggagaagt tccgttttct cttggatctg acacaggcgg ctccatccgt 540
cagccggcat ctttctgcgg cgttgtcgga ttaaaaccta catacggacg tgtatctcgt 600
tacggcctgg tcgcatttgc gtcttcattg gaccaaatcg gaccgattac acgtacggtt 660
gaggataacg cgtttttact tcaagcgatt tccggcgtag acaaaatgga ctctacgagt 720
gcaaatgtgg acgtgcctga ttttctttct tcattaactg gcgacatcaa aggactgaaa 780
atcgccgttc cgaaagaata ccttggtgaa ggtgtcggca aagaagcgag agaatctgtc 840
ttggcagcgc tgaaagtcct tgaaggtctc ggcgctacat gggaagaagt gtctcttccg 900
cacagtaaat acgcgcttgc gacatattac ctgctgtcat cttctgaagc gtcagcgaac 960
cttgcacgct ttgacggcat ccgctacggc taccgcacag acaacgcgga taacctgatc 1020
gacctttaca agcaaacgcg cgctgaaggt ttcggaaatg aagtcaaacg ccgcatcatg 1080
ctcggaacgt ttgctttaag ctcaggctac tacgatgcgt actacaaaaa agcgcaaaaa 1140
gtgcgtacgt tgattaagaa ggatttcgag gacgtatttg aaaaatatga tgttattgtt 1200
ggaccgacta caccgacacc tgcgtttaaa atcggtgaaa acacgaagga tccgctcaca 1260
atgtacgcaa acgatatctt aacgattccg gtcaaccttg ccggcgtacc gggaatcagt 1320
gtgccatgcg gattagcaga cggacttccg ctcggcctgc aaatcatcgg aaaacacttt 1380
gatgaaagca ctgtataccg cgttgctcat gcatttgaac aagcaacaga ccatcataaa 1440
gcaaaacctg aactgtaa 1458

<210> 32
<211> 885
<212> DNA
<213> B. subtilis

<220>
<223> glycine tRNA synthetase, alpha subunit

<400> 32
atgaatattc aagacatgat tctaaccttg caaaagcatt ggtccagtca gggctgtgtg 60
cttatgcagg cttacgatgt agaaaaagga gccggcacga tgagcccgta tacatttttg 120
cgcagtatcg gtcctgagcc gtggaaagtg gcttatgtag agccttccag acgtccggca 180
gacggccgct acggggagaa cccgaacaga ctgtatcagc atcatcagtt ccaggtcatt 240
attaaaccgt cacctgataa cattcaagag ctgtatttgg attccttgcg tgctcttgga 300
attgatccgc ttgagcacga tattcgcttt gttgaagaca actgggagaa tccgtcttta 360
ggctgcgcgg gtctaggctg ggaagtttgg cttgacggaa tggaaataac acaatttacg 420
tatttccagc aggtcggggg attagagtgt aaacccgttt ctgtagagat tacgtatgga 480
attgagcgtc tcgcgtctta tatccaggat aaagaaaacg tgtttgactt ggaatggacg 540
tcagggttta cagtaaaaga tttattcatg atggctgaat atgagcattc tgtttatacg 600
tttgaaacat cagacgtcga tatgctgttc caattgttca gcacatatga aaaagaagcg 660
atcaagcaaa tggacaacgg acttgttcat ccagcatatg actatgtgct gaaatgctcg 720
cacactttca acctgcttga tgccaaaggt gcgatctctg ttaccgagcg gacgggctat 780
atcgcaagag tgcggaattt agctagaaaa gtagcaaaaa cctactatga ggaacgagaa 840
aaactagggt tcccaatgct taaaggggag ggttcttctc atgag 885

<210> 33
<211> 2037
<212> DNA
<213> B. subtilis

<220>
<223> glycine tRNA synthetase, beta subunit

<400> 33
atgagtaaac aggatttact tttagagatc ggattagagg aaatgccggc gcgctttttg 60
aatgaaagca tggttcagct tggcgacaag ctgacaggct ggcttaaaga aaaaaatatt 120
actcacggtg aagtgaaact cttcaataca ccgagacgtc ttgctgtgtt cgttaaggat 180
gtcgcagaaa agcaggatga tataaaagaa gaagcaaaag ggcctgcgaa aaaaattgcc 240
cttgatgcgg acgggaactg gacaaaagcg gcaatcggct tttcaaaagg ccaaggcgca 300
aatgtggaag acctgtacat caaagaagta aaaggcatag agtatgtatt cgtgcaaaaa 360
ttccaagccg gccaagaaac gaagtcgctt ctgccagaat tgagcggttt aattacaagc 420
ttgcatttcc cgaaaaacat gcgctgggga aatgaagatt tgcgttatat ccgaccgatc 480
aaatggattg tcgcattatt tggacaggat gtcattccat tttcgatcac aaacgttgag 540
tctggacgga caacacaggg acaccgtttc ctcggacatg aagtgtcaat tgaatcacct 600
tcagcttatg aagagcagct caaaggacag catgttattg ctgatcctag cgtccgaaag 660
caaatgattc aatctcagct ggaaacaatg gctgctgaaa acaattggag cattccagtt 720
gacgaagacc ttcttgatga agtgaatcat ttagtagaat acccgacagc tctttacggt 780
tcatttgaat ccgagtttct gtcaattcct gaagaagtgc ttgtcacgac aatgaaagag 840
catcagcgct acttccctgt caaagacaaa aacggtgacc tgctgcctca ctttatcaca 900
gtccgaaacg gcaacagcca cgcgattgaa aatgtggccc gaggcaatga aaaagtgctg 960
cgcgcacgtt tatctgatgc ttcattcttc tacaaagaag atcagaaact aaacattgat 1020
gcaaatgtga agaagcttga aaatatcgtt ttccatgaag agcttggttc tcttgctgat 1080
aaggttagga gagtcacatc aatcgcagag aaacttgccg ttcgtcttca ggctgatgaa 1140
gatacattaa aacatgtgaa gcgggctgct gaaatttcta aattcgacct tgtgacgcat 1200
atgatatatg aattccctga acttcaagga attatgggcg aaaagtatgc gagaatgctt 1260
ggcgaagatg aagctgtggc tgcggcagtt aatgaacatt atatgcctag atcagcgggc 1320
ggagaaacgc cttctacttt caccggagct gttgtagcaa tggcggataa gcttgacaca 1380
atcgcttcat tcttctctat cggcgtaatt ccgaccggtt cccaggaccc ttacggactg 1440
ccccgccaag caagcggtat tgttgcgatt ctccttgacc gcaactgggg aatttcattt 1500
gaagagctgc ttactttcgt tcaaaccgat aaagaaaatg aactgctgga tttcttcact 1560
cagcgcttga aatatgtctt gaatgctgaa caaatcagac atgatgtcat tgacgccgtg 1620
ctggaaagct ctgagcttga gccgtactct gcgctgcata aagcacaagt actggagcaa 1680
aagcttggtg cacctggctt taaggaaaca gccgaagcgt taggccgagt gatctctatc 1740
agcaaaaaag gagtccgcgg agacattcag cctgatctgt ttgaaaatga atacgaagca 1800
aaactgtttg atgcctacca aacagcgaag gaaaatctgc aggaaaactt cagcaaaaaa 1860
gattatgagg cggcgcttgc ttcacttgca gccttaaaag aaccgatcga tgcttacttc 1920
gatcatacaa tggttatagc ggataatgag tcattaaagg caaatcgttt agcgcaaatg 1980
gtaagcttgg cggatgagat caagtccttt gcgaatatga atgcccttat tgtaaaa 2037

<210> 34
<211> 1272
<212> DNA

<213> B. subtilis

<220>
<223> histidyl tRNA synthetase

<400> 34
atgggatata acattccgag aggaacacag gatattctgc ctggagaatc agatcgctgg 60
cagtttgttg aacaaattat gagagacact tgccgcactt atcaatataa agaaatccgc 120
acaccgattt ttgagcatac agaactgttt gccagaggcg tcggagaatc aacggatatc 180
gtacaaaaag aaatgtatac cttcgaggac cgcaaaggca gaagcctgac gcttcgtccg 240
gagggaacag ctgctgcggt tcgcgctttt aatgaaaaca agctgttcgc aaatcctgta 300
cagccgacaa agctttatta tgtcgggccg atgttccgtt atgaacgtcc tcagacgggc 360
cgttaccgcc agttttatca gtttggaatt gaagcgattg gttcgaaaga ccctgcaatc 420
gacgctgaag taatggcgct tgcgatgagt atttatgaaa aagcaggttt agaaaacgtt 480
aagcttgtca taaacagtct cggcgatcaa gatagccgca aaagctacag agaagcgctc 540
gtgaaacact tcgagcctcg catcgaagaa ttttgttcag actgccagtc ccggctgcac 600
acgaacccgc ttagaatttt ggactgcaaa aaagaccgtg atcatgagct gatgaaatcg 660
gcaccttcga ttttgacata tttaaatgaa gaatcagccg cttattttga aaaagtgaaa 720
caatacttaa atgatcttgg catttcgtat gaaatcgatc cgaaccttgt gagagggctg 780
gattattaca accacaccgc atttgaaatt atgagcaatg cggagggctt tggcgcgatt 840
acgacacttg ccggcggcgg acgctacgac gggcttgtcg aacagatcgg cggacctgaa 900
gcaccgggca tcggatttgc gatgagcatc gaacgcttgc ttgccgcaat tgatgctgaa 960
aaaagagagc tgcctgttga taagggaatc gactgctata tcgtcacact cggcgaaaaa 1020
gcaaaggatt attctgtttc attggtgtac aaactgagag aagcaggcat ttccagtgaa 1080
atcgattatg aaaataagaa aatgaaaggc cagtttaaaa cagctgatcg cttaaaggcc 1140
agatttattg cgattttggg tgaagatgag cttgcccaaa acaaaatcaa tgtaaaggat 1200
gcacagacgg gcgaacagat tgaagtggca cttgatgaat ttatacatgt gatgaaggca 1260
aaccaaaagg ga 1272


<210> 35
<211> 2763
<212> DNA
<213> B. subtilis

<220>
<223> isoleucyl-tRNA synthetase

<400> 35
atggatttta aagacacgct cttaatgccg aaaacagatt tcccgatgcg tggaaatttg 60
ccaaaccgtg agcctgacat tcaaaaaaaa tgggaggaag aagatatcta ccgtcttgtt 120
caggaacgga cgaaagaccg cccgaaattt gttttacatg acggacctcc gtatgcaaac 180
ggcgacatcc atatgggcca tgcacttaac aagattttga aagacttcat tgtccgctat 240
aaatcaatga gcggctacaa cgcaccgtat gtgccgggct gggatacaca cggattgcca 300
attgaaacag ctctgacaaa aaacaaaaag gtcaaccgca aagaaatgtc agtagcggaa 360
ttccgcaaac tatgcgaaga gtacgcttgg aagcaaatcg agggacagcg tgagcaattc 420
aaacgtcttg gtgtccgcgg tgactgggaa aacccatatg tgacattaaa accggaatac 480
gaagcgcagc aaatccgcgt atttggtgaa atggcaaaac gaggctacat ttacaaaggc 540

cttaaaccgg ttaactggtc accttcaagt gagtctgctc tggctgaagc cgagatcgaa 600
tatcaagata aacgttcagc atctatttac gtcgcttttg gtgtaaaaga cggaaaaggc 660
gttcttgaaa acggcgagcg catcatcatt tggacaacaa cgccgtggac aattccggcg 720
aacctcggaa tctcagtgca ccctgatctt gagtacagcg tgattgcagt aggtgaagac 780
cgctttgttg tagcaagtgc cttagtcgaa aatgttgcat cggcatgcgg atttgatcag 840
tatgaagtga caagaacggt caaagggaaa gaccttgaga acattatcgc tgaacacccg 900
ctatatggca gagactctct cgttatgctt ggtgaacacg taacaactga tgccggaaca 960
ggctgtgttc atacagcgcc tggacatggg gaagatgact ttatcatcgg ccaaaaatac 1020
ggtttagatg tgctttgccc ggtcgatgaa aaaggtgtaa tgacaagcga agctcctggc 1080
tttgaaggca tgttctatga tgatgcaaac aaagcgatca cacagcagct tgatgaaaaa 1140
ggcgcacttg tgaagcttga attcattact cattcttatc cgcatgattg gagaacaaaa 1200
aaaccaacca ttttcagagc aacagcgcaa tggtttgcgt ctattaaaga tttcagatca 1260
gacctgctgg atgccattaa agaaaccaaa tgggttcctg aatggggcga gcagcgtttg 1320
cacaacatgg ttcgggaccg cggagactgg tgtatttcca gacagcgtgc gtggggtgtg 1380
ccgattccgg tattttacgc tgaaaacgga gaaccggtta ttacagatga aaccattgaa 1440
catgtttctg aattgttcag acagcatgga tcaaacattt ggtttgaaaa agaagcaaag 1500
gatcttcttc cggaaggctt tacgcatcct ggcagcccga acggcacatt tacaaaagaa 1560
caggatatca tggatgtttg gtttgattca ggctcttcac atcaagcagt gcttgaagaa 1620
cgtgatgacc tcgttcgccc ggctgatcta tacctagagg gatctgacca atatcgcggc 1680
tggtttaact cttctctttc tacagcagta gccgtaacag ggaaagcgcc gtataaaggt 1740
gtgctcagcc atgggttcgc actggatgga gaaggacgta agatgagtaa atcaatcggt 1800
aacgttgttg ttccggctaa agtcatgaaa cagcttggtg ccgacatctt aagattatgg 1860
gtatcttcag tggattatca ggcggacgtt cgcgtgtctg acgccattct gaagcaggtt 1920
gcggaagtat atcgtaaaat ccgcaacacg ttccgtttcc ttcacggcaa ccttttcgat 1980
tttgatccaa aaacaaatgc ggtggctgtc gaagatcttc gcgaagtgga tcagtatatg 2040
ctgattaagc tgaacaagct gattgataaa gtgaaaaaag cgtatgatga gtacgaattt 2100
gcggttgtgt atcacagcat tcataatttc tgcacaatcg aattgagctc attctacctt 2160
gattttgcaa aagatattgt ctacatcgag catgcggatc atccggatag acgcagcatg 2220
cagacagtat cctacgaaac gcttcttgca ttagtgaagc tttcagcgcc tatccttcca 2280
catacggcag acgaattgtg gtctcattta acatttgttg aagagcagag cgttcagttg 2340
accgatatgc cggaaacaat cacggttcca aacagtgaag cgactgaaga aaaatttgac 2400
cgctttatgg ctcttcgtga tgacgtgtta aaagcattag aaactgcgcg aaatgaaaaa 2460
attatcggta aatctttgga agcaaacctg aaattgtatc caaacaaaga aaacaaggag 2520
ctcttggctt ccataaaaga aaacctttct cagctgttta ttgtgtctga actgacaatc 2580
agcgaagaaa atgaagcgcc gaacgatgcg caaagctttg cgacgggtaa aatcgctgtc 2640
gagaaagcgg aaggcgaaat gtgtgagaga tctcgtgtga tttcaaaaga tgtaggggca 2700
aatccgaaat atcctacact ttcattacgc aacgctgaaa tcgttgaaaa atactatcaa 2760
aaa 2763

<210> 36
<211> 2412
<212> DNA
<213> B. subtilis

<220>
<223> leucyl-tRNA synthetase

<400> 36
ttgagttttc agcacaaaga gatagaaaag aaatggcaga catattggct tgaaaacaaa 60
acatttgcca ctcttgataa taatgaaaaa caaaaatttt acgcgctgga catgtttcct 120
tatccgtctg gagctgggtt gcacgtcggc catcctgaag gatacacagc tacggatatt 180
ctgtcccgca tgaagcgcat gcagggctat gatgtccttc atccaatggg ctgggacgca 240
ttcggcctgc cagctgaaca gtacgcgctt gacacaggga acgaccccgc tgtgtttacg 300
aagcagaata ttgataactt ccgccgccaa attcaagcgc ttggcttctc atatgactgg 360
gatcgcgaaa tcaatacgac tgaccctgaa tactataaat ggacgcaatg gattttctta 420
aagctatacg aaaaaggcct tgcttacgtt gacgaagtgc ctgtaaactg gtgccctgcg 480
ctcggtactg ttcttgccaa cgaagaagtc attgacggca agagcgaacg cggcggccat 540
ccggtagaga gacgcccaat gaagcagtgg atgctgaaaa tcaccgctta tgcggacagg 600
ctccttgagg acttggaaga gcttgattgg ccggaaagca ttaaagatat gcagcgcaac 660
tggatcggcc gttcggaagg cgctcacgtt cattttgcta tagatggaca tgatgattcc 720
tttacagtgt ttacaacaag accagatacg ctgtttggcg ctacatacac tgtccttgcc 780
ccggaacacg cattggtgga aaacatcaca acggcagagc aaaaagaagc tgttgaagct 840
tatatcaaag aaattcaatc aaagagtgac ctagaacgca cagatcttgc gaaaacaaag 900
acaggcgtat ttacaggagc gtatgcgatc aatcctgtaa acggagaaaa actgccgatt 960
tggattgcgg attatgttct tgcatcatac ggaacaggtg ctgtcatggc agttccagga 1020
cacgatgagc gtgattttga attcgccaaa acattcggcc ttccggtgaa ggaagtcgta 1080
aaaggcggga acgttgagga agcagcctat actggcgacg gcgagcacgt gaactctgat 1140
ttcctgaacg gccttcacaa acaggaagcg attgaaaaag tgatcgcttg gctggaagaa 1200
acgaaaaacg gtgagaagaa agtgacgtac cgtcttcgtg actggctctt cagccgccag 1260
cgttattggg gcgagccgat tccggtcatt cattgggaag acggaacgtc aacagctgtc 1320
ccggaagagg agctgccgct gattttgcca aaaacggatg aaatcaaacc gagcggaacg 1380
ggcgaatcac cgcttgcgaa cattaaagag tgggtggaag tcacagaccc tgagacaggg 1440
aaaaaaggaa gaagagaaac gaatacaatg ccgcaatggg cgggaagctg ctggtatttc 1500
ttgcgctata ttgatccgca caatccggat cagctggcat caccagaaaa attggaaaaa 1560
tggcttccgg tcgatatgta tatcggcggt gcagaacatg ccgtgcttca ccttctgtat 1620
gcccgcttct ggcataagtt cctttatgat atcggcgtag tgccgacgaa agaaccgttc 1680
caaaagctgt acaaccaagg aatgattctc ggcgaaaaca acgaaaaaat gagtaaatct 1740
aaagggaacg ttgtcaatcc tgacgaaatc gtggcctctc acggtgctga tacgctgaga 1800
ttgtacgaaa tgttcatggg acctcttgat gcttcaatcg cctggtctga atcaggatta 1860
gacggtgcgc gccgtttcct tgaccgtgta tggcgcctat ttattgaaga cagcggtgag 1920
cttaatggaa aaatcgttga aggcgcgggt gaaacattgg agcgcgtcta tcatgaaacg 1980
gtcatgaaag tcacagacca ttatgaaggc cttcgtttca acacgggtat ttcccagctg 2040
atggtcttta ttaatgaagc ttataaagca acagaactgc cgaaagaata tatggaaggc 2100
ttcgtgaagc ttctttctcc tgtcgcgcca cacttagcgg aagagctatg ggagaagctt 2160
ggccattccg gcacaattgc ctacgaagct tggcctgtat atgatgaaac aaaacttgtg 2220
gatgatgaag ttgaaatcgt tgttcagctg aacggaaaag taaaagcgaa attacaggtt 2280
cctgccgatg caacgaaaga acagctggaa cagcttgctc aagcagatga aaaggtcaaa 2340
gagcagcttg aaggcaaaac gattcggaaa atcatcgcgg tgcctgggaa gcttgtcaat 2400
attgtggcaa ac 2412

<210> 37
<211> 1497
<212> DNA
<213> B. subtilis

<220>
<223> lysine tRNA synthetase

<400> 37
atgagtcaag aagagcataa ccatgaagaa ttgaatgatc agctgcaagt cagacgcgat      60
aaaatgaatc agctgagaga taacggcatc gatccattcg gcgcacgttt tgaaagaact     120
catcagtctc aagaagttat ttcggcatat caagatctaa ccaaagaaga gttagaagaa     180
aaagcgattg aagttacaat cgcaggccgc atgatgacaa aacgcggcaa aggaaaagcc     240
ggctttgccc atcttcagga tttagaaggc caaatccaaa tctacgtaag aaaagacagt     300
gtcggtgacg accaatatga aatcttcaag tcttctgacc tcggtgatct tatcggcgta     360
accggaaaag tcttcaaaac aaatgtaggc gaattgtctg ttaaagcaac ttcctttgaa     420
ttgctgacaa aagcgcttcg tccgcttcct gacaaatacc atggtttaaa agacgttgag     480
cagcgctacc gtcagcgcta tctggatctt atcgtaaacc cagacagcaa acatacgttc     540
attacacgaa gcaagatcat tcaagctatg agaaggtacc ttgatgatca tggatactta     600
gaagtagaaa cacctacaat gcacagcatt cctgggggag cttctgcacg tccgtttatc     660
actcaccaca acgcgttaga cattccactc tatatgcgta ttgctatcga actgcaccta     720
aaacgtctaa ttgtcggcgg tttagaaaaa gtatatgaaa tcggccgtgt tttccgtaac     780
gaaggtgtct ctacacgcca taaccctgaa tttacaatga ttgagttata tgaagcatat     840
gcggactata aagatatcat gagcttaact gaaaaccttg tcgctcatat cgcccaagaa     900
gtgcttggca caactacgat tcaatacggg gaagagcaaa tcgaccttaa accggagtgg     960
aaaagaatcc atatggttga cgcagtcaaa gaagcgaccg gcgttgattt ctgggaagag    1020
gttactgttg agcaggcgcg tgaatatgca aaagaacatg aagtagaaat taaagactct    1080
atgacagtag gccatatcat caacgaattc ttcgaacaaa aaattgaaga aacgcttatt    1140
cagccaacgt ttatttacgg gcatcctgta gaaatttctc ctcttgctaa gaaaaaccct    1200
gaggacccgc gttttacaga ccgttttgag ctgtttatcg ttggccgtga acatgccaac    1260
gcgtttacag agctgaatga tcctattgat caaagagaac gctttgaagc gcaattaaaa    1320
gagcgtgaag ccggtaatga tgaagctcat ttaatggatg aagactttgt tgaagctctg    1380
gaatacggta tgccgccaac aggaggttta ggcatcggta tcgaccgtct ggttatgctg    1440
ctgacgaatg ccccttctat tcgcgatgtg ctgttattcc cgcaaatgag acaacgc       1497


<210> 38
<211> 1176
<212> DNA
<213> B. subtilis

<220>
<223> histidyl-tRNA synthetase, hisZ

<400> 38
atgtttatgt ttgaaaaacc gcacggcatg agagatacac tgcccggttt atacgaaacg      60
aaaaaaaagg tgagacgatc gttaaccgat ttgattgata aatggggata tcaatttatg     120
gaaacgccga cactggagtt ttacgatacc gttggcgtcc agtcagcaat tgaagagcag     180
cagctgttta agctacttga tcaggacggc aagacattgg tgcttcgccc ggatatgacg     240
gggccgattg caagggtggc ggcatcgaag cttctgaaac acggtcatcc gctaagagtc     300
ggctatgcgg caaatgtatt cagggctcag gagcgtgaag gcggacgtcc ggctgagttc     360
gagcaggtcg gagtggagtt aatcggtgac ggcacgacga gcgcggatgc ggaggtcatt     420

gctttagtcg tcggggcatt aaaaaacgct gggctggcat cctttaaaat tgcaattggc 480
catgccggca ttgcggatgc tttgtttgtt gaggtgctcg gaaacgttga acgagctgat 540
gtgctgcgga ggttcttata tgaaaagaac tacgtcggct acagagagca tgtcaagtct 600
ctcccgcttt cctccattga taaaagcagg ctgcttgagc tccttgaact gcggggcggt 660
atagaagtat gcggacgtgc cgaggaaatc gtcgattctg cgcaaggaaa aagcgtggtt 720
gatgagctga aggcgctgtg ggacattctt gaggattacg gatgtacgga aaatgtccgc 780
ctggatctga atatggtcag ccacatgagc tattacacag ggattttatt tgaagtgtac 840
gccgagaatg tcggttttgt cattggaagc ggcggccgtt acaacaagct gctgggccat 900
tttgattcac ctgcaccggc aacaggcttc gggcttcgga tcgaccggct gattgaagcc 960
cttcatatga aggacgaacc ttgtgaaata gacgctgtta ttttcagcaa agagcagcgg 1020
gcgcaagcca tcgcttatgc gaatgaagaa cgcatgaaag ggaacaaagt ggttcttcaa 1080
gatttatcgg gaatagaaaa tatcgaccag atgacaaaat cttttgcaaa cgtcacttat 1140
tttatcggtg ccagaaagga agagcaaaat gggtaa 1176

<210> 39
<211> 1992
<212> DNA
<213> B. subtilis

<220>
<223> methionyl-tRNA synthetase

<400> 39
atgccgcaag aaaacaatac attttacatt acaacaccga tttattatcc gagcggaaaa 60
ttacatatcg gccatgcata tacgacagtc gcaggagatg caatggcacg ttacaaaaga 120
ttaaaagggt tcgatgttcg ctatttaacg ggaacggacg agcatggaca aaagatccag 180
caaaaagctg aacaggaaaa cattacacct caggagtatg tggatcgcgc agcggcagat 240
attcaaaaac tgtggaagca gcttgaaatc tcaaacgacg actttatccg cacgacagaa 300
aaacggcata aagttgtcat tgaaaaagtg tttcaaaagc ttcttgataa cggggacatc 360
tatcttgatg agtatgaagg ctggtacagc atccctgatg aaacgttcta cacggaaact 420
cagctcgttg atattgagcg gaatgaaaag ggagaggtca tcggcggaaa aagccctgac 480
agcggtcacc cagttgaatt gatcaaagag gaatcttatt tcttccgcat ggggaaatac 540
gcggatcgtc ttctgaaata ctatgaagaa aacccgacat tcattcagcc agaatcacgc 600
aaaaacgaaa tgattaataa ctttatcaaa cctggacttg aggatttggc agtatcacgt 660
accacttttg attggggcgt gaaggtgccg gaaaatccaa agcatgttgt atatgtttgg 720
attgacgcac tatttaacta tttaacggca ctcggttatg atacagaaaa tgatgagctt 780
tatcaaaaat attggcctgc cgatgttcat ttagtcggta aggagattgt acgattccat 840
actatttact ggccaattat gctgatggcg ctggatcttc cgctgccaaa gcaagtattc 900
gcgcatggct ggcttttgat gaaagacgga aaaatgtcga aatcaaaagg aaacgttgta 960
gatccggtta cattaattga acgctatggt ttagacgaac ttcgctatta cttgcttcgc 1020
gaagtgccgt tcggatcaga cggtgttttc acgccggaag gatttgttga gcgaatcaac 1080
tatgatttag cgaatgattt aggaaatcta ttgaatcgta ctgttgcgat gattaataag 1140
tattttgacg gacaaatcgg ttcttacaaa ggtgctgtta cggaatttga ccatacgctc 1200
acttctgttg ctgaagaaac agtgaaagct tatgagaaag caatggaaaa tatggagttc 1260
tcggtagcac tttcgacttt atggcagctc atcagccgca caaacaaata cattgatgag 1320
acagctccat gggtgcttgc gaaagatccg gcaaaagaag aagaattgcg atcagttatg 1380
tatcatctcg ctgaatcatt gcgtatttca gcggtactgc ttcagccatt cttaacaaaa 1440

acacctgaaa aaatgttcga gcagctgggc attactgatg aatctttaaa agcttgggac 1500
agcattacag ctttcggcca gctgaaagat acaaaagtac aaaaaggcga gccattgttc 1560
cctcgtttag aggcagaaga agaaattgct tacatcaaag ggaaaatgca aggttcagca 1620
ccagcgaaag aagaaacaaa agaagaagag cctcaagagg tcgatcgttt acctgaaatt 1680
acgattgatc aatttatgga tgtagagctt cgcgtagctg aggtcattga ggcagagcca 1740
gtgaaaaaag cagaccgttt attgaagctg cagcttgatc ttggttttga aaagcgccaa 1800
gtggtatccg gcattgcgaa gcattatacg cctgaagagc ttgttggaaa aaaactcgtt 1860
tgtgtaacaa atctaaaacc ggttaaactc agaggagagc tttctcaagg tatgatcctt 1920
gcaggagaag cagacggcgt tttaaaggtc gtatctatcg atcagtcgtt accaaaaggc 1980
acaagaatta aa 1992

<210> 40
<211> 1032
<212> DNA
<213> B. subtilis

<220>
<223> phenylalanyl-tRNA synthetase alpha subunit

<400> 40
atggaagaaa agctaaaaca gctggaacaa gaagctttag aacaagtaga agcggcaagc 60
tcattgaagg ttgtcaatga tattcgggtg caatatctcg gaaaaaaagg gccgattaca 120
gaagtgctgc gcggaatggg caagctttct gctgaggaac gtccaaaaat gggggcgctc 180
gcgaacgaag taagggagcg tattgccaat gcgattgctg acaaaaacga gaagcttgaa 240
gaagaggaaa tgaaacagaa gcttgcagga cagacaattg acgtcacgct gccggggaac 300
cctgttgcag tcggcggccg ccatccgctc actgttgtca ttgaagaaat tgaagattta 360
tttatcggta tgggctacac agtcgaggaa gggccagagg ttgaaacgga ttactacaac 420
ttcgaatcgc tcaatcttcc gaaagaacac ccagcgcgcg atatgcagga cagcttttac 480
atcacagagg aaactttgat gagaacgcaa acttctcctg tccaaacacg tacgatggaa 540
aagcatgaag gcaaaggtcc cgttaaaatc atttgcccgg gtaaagtata tcgccgtgat 600
aacgatgatg cgacgcactc tcaccaattt atgcaaattg aagggcttgt cgttgacaaa 660
aacatcagca tgagtgattt aaaaggaacg cttgaacttg ttgcgaaaaa aatgttcggg 720
caagaccgtg aaatcagact ccgcccaagc ttcttcccgt ttactgagcc ttcagtagaa 780
gtggatgtga catgctttaa atgcggtggg aacggctgct cagtatgtaa aggaacaggc 840
tggattgaaa tcctcggtgc cggaatggtt cacccgaacg tgcttaaaat ggctggcttt 900
gatccgaagg aatatcaggg cttcgcattc ggaatgggtg ttgagcgcat cgcgatgctg 960
aaatatggca ttgatgatat ccgccacttc tatacaaacg atgtcagatt tatttcgcag 1020
tttaaacagg cg 1032

<210> 41
<211> 2412
<212> DNA
<213> B. subtilis

<220>
<223> phenylalanine tRNA synthetase, beta subunit

<400> 41
atgtttgttt cttataaatg gttagaggat tatgttgatt taaaaggcat ggacccggct      60
gttcttgctg aaaaaattac aagagccggt attgaggttg aaggaattga atacaaagga     120
gaaggcatca aaggcgttgt catcggccat gtgctggagc gcgagcagca cccgaatgct     180
gataagctga ataagtgcct tgtggatatc ggagctgaag cccctgtaca aatcatttgc     240
ggcgcgccga atgtggataa gggacaaaaa gtcgcagttg caacagtcgg agcggtgctg     300
ccgggcaatt tcaaaatcaa aaaagccaag cttcgcggtg aagaatcaaa cggcatgatc     360
tgttccttac aggagcttgg tatcgaaagc aaacttgtgg cgaaggagta tgcagaaggc     420
attttcgtat tcccgaatga cgctgaaaca ggaagcgatg ccttagcggc tttacagctt     480
gacgatgcga ttcttgagct gggtttaacg ccaaaccgcg cggatgccat gaacatgctt     540
ggtgttgctt acgaggttgc ggcgatttta gacactgagg taaagcttcc ccaaacggat     600
tacccggctg cttcagaaca agcgtctgat tacatttcag tcaaaattga agatcaagaa     660
gcgaacccgc tgtacactgc aaaaatcatt aaaaatgtca cgattgctcc gtcaccgctt     720
tggatgcaga caaagctgat gaatgcaggc attcgtccgc acaacaacgt cgttgacatc     780
acaaattttg tcctgttgga atacggacag ccgcttcatg cgtttgatta tgacagattt     840
ggctctaagg aagtcgtagt aagaaaagca gctgagaatg aaatgatcgt aacacttgat     900
gatcaagaac gtaagctgtc tgccgatcac cttgtcatta caaacggaac gaaagcgcag     960
gccgttgccg gtgtcatggg aggagcagag tcagaggttc aggaagatac gaaaaccatt    1020
ttgcttgagg ctgcgtattt caacgggcag aaggttcgca aggcttccaa agaccttggt    1080
ttgagaagtg aatcaagcgt aagatttgaa aaaggaattg atccggcacg cgtacgtctt    1140
gcagcagaac gggctgcgca gctgatccat ctgtatgccg gcggtgaggt gcttgctgga    1200
acggttgagg aagatcacct gacaatcgaa gcaaataaca ttcacgtatc tgctgacaaa    1260
gtgagcagcg ttctaggcct gacgatcagt aaagaagaac tgatcagcat ttataaacgt    1320
ctcggtttta cggtcggtga agcggatgat cttctcgttg tgactgtccc atcacggcgc    1380
ggtgacatta caatagaaga ggatttaatc gaagaagcgg caagactgta cggctacgat    1440
aacattccgt ctacgcttcc tgagacagcg ggaactacag gcggattaac gccatatcag    1500
gcaaaacgcc gtaaagtcag acgcttcctt gaaggcgcag gcttatcaca agccatcact    1560
tactcactga caaacgagaa gaaagcgaca gcgtttgcga ttgaaaaatc actgaatact    1620
gtgcttgcgc ttccaatgag tgaagaaaga agcattctca gacacagtct tgttccaaat    1680
ctgcttgatt ctgtttctta caatcttgcg aggcagactg actcagttgc actgtacgaa    1740
gttggctccg ttttcctaac gaaggaagag gacacaaaac ctgttgaaac agaacgcgta    1800
gcaggagctg ttaccggatt atggcgcaag cagctatggc agggtgaaaa gaaaccagtt    1860
gatttctttg ttgtcaaagg aattgtggaa gggctgttag acaaactcaa cgtcctagac    1920
agcattgaat ttgtacagtc tgaacgcaaa cagctgcatc cgggcagaac agcgaacatt    1980
ttattgaatg gttctctgat cggcttcatc ggccaagtcc atccttcatt ggaaaaagaa    2040
ctggatataa aggaaacata cgtatttgag ctcgatcttc atgcgttgct cgcagcagaa    2100
acagcaccgc ttgtttacac ggcgattcca aaatatccgt ctgtgacacg tgatatcgct    2160
cttgtaacag ataaaaccgt cacaagcgga cagctcgaga gcgtcattaa agaggccggg    2220
ggcaagcttc tgaaagaggt aaccgtattt gacgtatacg aaggagagca tatggaggaa    2280
ggcaagaagt cagtcgcttt ctcactccag tatgtcaatc ctgaacaaac actgactgaa    2340
gaagaagtga caaaggcgca cagcaaagtg ctgaaagcgt tagaagacac atatcaagct    2400
gttttaagag gc                                                        2412

<210> 42
<211> 606
<212> DNA
<213> B. subtilis

<220>
<223> similar to phenylalanyl-tRNA synthetase, ytpR

<400> 42
atgaacgctt tttataataa agaaggtgtg ggagacacgc tcctgatctc tcttcaagat      60
gtgacacgcg aacaattagg ctatgaaaaa cacggcgacg tcgtcaaaat tttcaataac     120
gaaacaaaag aaacaacggg cttcaacatt ttcaatgcgt cttcttactt gaccattgat     180
gaaaacggcc ctgttgcgct ctcagaaaca ttcgtgcaag atgtgaatga gattttaaac     240
agaaacggcg ttgaagaaac attagtcgtt gatttatctc cgaagtttgt tgtgggttat     300
gtagaatcaa aagaaaaaca tccgaatgcg gataaattaa gcgtatgtaa agtcaacgta     360
ggagaagaaa cgcttcagat cgtctgcggc gcgcctaacg ttgaccaagg ccaaaaagtt     420
gtcgttgcca aagtcggtgc tgtgatgcct agcggacttg tcattaagga tgcagagctt     480
cgtggcgttc cgtcaagcgg gatgatctgc tcagcgaaag agcttgattt gccggatgcc     540
cctgctgaaa aaggaatcct tgtgttagaa ggagactatg aggcgggaga cgcatttcag     600
ttttag                                                                606

<210> 43
<211> 1692
<212> DNA
<213> B. subtilis

<220>
<223> prolyl-tRNA synthetase

<400> 43
atgagacaaa gcttgacgct tattcctacg ctccgtgaag ttccagctga tgccgaagca      60
aaaagtcatc agcttcttct gagagcagga tttatcagac agaatacgag cggggtatac     120
agctatatgc ctcttgcgta taaggtgatt caaaacattc agcagattgt tcgagaggaa     180
atggagaaaa ttgatgccgt agaaatgctc atgcccgcat tgcagcaggc agagacatgg     240
caggaatcag gcagatggta tacgtatggt cccgaactga tgagactaaa agaccgtcat     300
ggccgtgaat ttgctttagg ggcaacgcat gaagaggtta tcacttcgct tgttcgcgat     360
gaggttaaat cttataagcg tctccctctg actctttatc aaattcagtc taagttcaga     420
gatgaaaaac gtcctcgctt cggtttgtta agaggccgcg aatttattat gaaggatgcg     480
tactctttcc atgcatctgc agagagcctg gatgaaacgt atcaaaaaat gtacgaggcc     540
tattctaata tttttgcccg ctgcggcatt aatgtaagac ctgttatcgc tgattcaggc     600
gcaatgggag gaaaggatac gcacgaattt atggcacttt ctgcgatcgg agaggatacg     660
attgcgtatt ctgatgaatc acagtacgcg gctaatatcg agatggctga agttcttcac     720
caggaagttc cttcagatga agagcctaaa gctctagaga aggttcatac gcctaacgtg     780
aaaacaatcg aagaactgac tgcgttctta caggtttcgg ctgaagcgtg cattaagtca     840
gtattgttta aagctgatga ccgttttgtc ttagtgcttg taagagggga tcatgaagtt     900
aacgatatta aagtgaaaaa cttgcttcat gcagaagttg tagagcttgc tacacatgaa     960
gaggtcattc agcagctcgg aacagagcca ggctttgtag gccctgtcgg tattcatcag    1020
gatgtggaag tatatgccga tcaagctgtg aaagcaatgg ttaatgctgt tgccggggca    1080
aatgaaggag atcatcatta taaaaatgtc aatgtgaatc gtgacgcgca aattaaagaa    1140
tttgctgatc ttcgttttat taaagaaggt gatccttcac cagacggcaa gggaacgatc    1200
cgttttgctg agggaatcga agtcggacaa gtctttaagc tcggaacacg ctattcagaa    1260

gcgatgaatg cgacatattt agatgaaaac ggacgcgcgc agccaatgct gatgggctgt 1320
tacggaatcg gtgtgtcaag aacgctttct gctattgctg aacagcatca cgacgaaaaa 1380
ggcttaatat ggccaaaaag cgttgcgccg tacgatcttc atattcttgc tttgaacatg 1440
aaaaacgatg ggcaaagaga gcttgctgaa aagctgtatg ccgatttaaa agcggaaggc 1500
tatgaagtgc tctatgatga ccgtgctgag cgtgccggcg taaaattcgc tgattcagat 1560
ttaatcggcc ttccaatccg catcactgtc ggaaaacgag ctgacgaagg aatcgtcgaa 1620
gtgaaaattc gtcaaactgg tgagtcaact gagatttcag tagacgaatt atctgcgttt 1680
atcagcaagc ag 1692

<210> 44
<211> 1275
<212> DNA
<213> B. subtilis

<220>
<223> seryl-tRNA synthetase,

<400> 44
atgcttgata cgaaaatgct gagagcaaat tttcaagaaa ttaaagcaaa gcttgtacac 60
aaaggcgaag acttaactga ttttgataag tttgaggcgc tggatgatag acgaagagag 120
cttatcggca aggttgaaga gttaaaggca aaacgaaatg aagtttctca gcaggttgct 180
gtgctgaagc gtgagaaaaa agacgcggat cacattatta aagaaatgcg tgaagtcggc 240
gaggaaatta aaaagctcga tgaagaatta cggacagtgg aagctgagct tgatacaatc 300
ctgctctcaa tcccgaatat tccgcatgag tctgtacctg tcggtgaaac agaagacgac 360
aacgtagaag tgcgtaaatg gggtgaaaag ccttcatttg cttatgagcc gaagccgcac 420
tgggatattg cggatgagct gggtattctg gattttgaac gtgctgccaa agtaacagga 480
agccgtttcg tgttctataa aggcttaggt gctcgtctgg agcgtgcgct ttataacttt 540
atgcttgatc tgcatgtgga tgagtataac tacactgaag tgatcccgcc atatatggta 600
aaccgcgcaa gcatgacggg aacggggcag cttcctaaat ttgaagagga tgcatttaaa 660
atcagagaag aagattattt cttaattcca acggcggaag tgccgattac aaacatgcat 720
cgcgatgaaa tcctttcagg tgacagcctg ccgatcaact atgcggcatt cagtgcttgc 780
ttccgttctg aagctggttc agcaggacgt gacacacgcg gattaattcg tcagcaccaa 840
tttaataaag ttgagcttgt aaagtttgtg aagcctgaag attcttatga agaattagag 900
aaacttacaa accaagcaga acgggttctt cagctgcttg agcttccata ccgtgtcatg 960
agcatgtgta cgggtgactt aggctttacg gctgcgaaaa aatacgatat cgaagtttgg 1020
attccaagcc aagacacata tcgagaaatc tcttcttgca gcaacttcga agcgttccag 1080
gcgagacgtg cgaatattcg tttcagacgt gaagcgaaag gcaagcctga gcatgtacac 1140
acactgaacg gctcaggact ggcggttgga agaacagttg ctgctatctt agaaaattat 1200
cagcaggaag acggaagcgt tgtgattccg aaagtgcttc gtccttatat ggggaataga 1260
gaagtaatga aaccg 1275

<210> 45
<211> 1929
<212> DNA
<213> B. subtilis

<220>
<223> threonyl-tRNA synthetase, major

<400> 45
atgtcagata tggtaaaaat cacatttcct gatggagcag tcaaggagtt tgcgaaagga      60
acaacaacag aagatatcgc ggcatccatc agtccgggat taaagaaaaa gtcattagcc     120
ggaaaactga acggaaaaga aatcgatttg agaacgccga tcaatgaaga cggtacagtg     180
gaaatcatta cagaaggctc agaagaaggt cttcaaatta tgcgccacag tgcggctcac     240
ctgctggctc aagcgattaa acgcatctac aaggatgtta aattcggcgt cggtccggtt     300
atcgaaaacg gtttttacta cgatgtagaa atggacgaag cgattacacc ggaggatctg     360
ccgaaaatcg agaaagaaat gaaaaaaatc gttaatgcga accttccgat cgttcgaaaa     420
gaagtcagcc gtgaagaagc gaaagcccgt tttgcggaaa tcggcgacga cctgaagctt     480
gaactattgg atgcgattcc tgaaggagaa accgtttcga tctatgagca aggcgaattc     540
tttgacctgt gccgcggcgt ccatgttcct tcaaccggaa aaatcaaaga atttaagctg     600
ttaagccttg caggcgcata ctggcgcggt gacagcaaaa accaaatgct tcagcgcgta     660
tatggtacag ctttcttcaa aaaagctgat cttgaagagc atcttcgtat gctagaagaa     720
gcgaaagaac gcgaccacag aaagcttggc aaagaattaa agctgttcgc gaactctcaa     780
aaagtcggac aaggcctgcc gctttggctg ccaaaaggcg caacaatccg ccgcgtcatc     840
gagcgctaca ttgtcgataa agaaatcagc ctcggctatg agcacgtata cacacctgtg     900
ctaggcagca aagagctgta tgaaacatca ggacactggg atcattatca ggaaggcatg     960
ttccctccga tggaaatgga caatgaaaca cttgtattgc gtccgatgaa ctgtccgcac    1020
catatgatga tttacaaaca agacattcac agttaccgcg agcttccgat tcgtattgca    1080
gagcttggaa cgatgcaccg ctatgaaatg tcaggtgcgc tgtcaggact tcaacgggta    1140
cgcggaatga cgttaaatga tgcacacatc tttgtgcgcc cggatcaaat taaggatgag    1200
tttatccgta cagtccgttt aatccaggat gtttatgaag acttcggttt aagtgattac    1260
acattccgtc tgtcttaccg cgatccggaa gatacagaga aatattttga tgacgatgaa    1320
atgtggaaca aagcgcaatc catgctgaaa gaggcaatgg atgaaatcgg ccacgactat    1380
tacgaagcag aaggtgaagc ggcattctac ggacctaaac ttgatgttca ggtgaaaact    1440
gcgatcggaa aagaagaaac actgtctacg gttcagcttg atttcttatt gccggaacgt    1500
ttcgacctga catacatcgg tgaggacggc aagcagcacc gtccggttgt cattcacaga    1560
ggtgtcgttt caacaatgga acgctttgtt gctttcttaa tcgaagaaca caaaggcgcg    1620
ctgccgacat ggcttgcacc ggttcagttc caagtcatcc cggtttctcc ggctgtgcat    1680
ttagactacg cgaaaaaagt gcaggaacgc ctgcaatgtg aaggcctgcg tgttgaagtc    1740
gacagccgcg atgaaaaaat cggctacaaa atccgtgaag cgcaaatgca aaaaatcccg    1800
tacatgctgg tggtcggtga ccaagaagca gaaaacggag cggtaaacgt gcgtaaatac    1860
ggagaacaga actctgaaac catttcactt gatgagtttg tgaaaaaggc agtagctgaa    1920
gcgaaaaaa                                                            1929

<210> 46
<211> 1914
<212> DNA
<213> B. subtilis

<220>
<223> threonyl-tRNA synthetase, minor

<400> 46
atgagcaaac atgtacacat tcagcttccg gacggacaga tccaggaata tccgaaaggc      60

atcacaatca aagaagcggc tggttcgatc agttccagtc tgcaaaagaa ggcggccgca 120
ggtcaggtca acggcaagct tgttgatctc agtttcaagc tggaggagga tgctgagctt 180
tcgattgtca cactcgattc acaagaaggc ttgcaggtcg ttcgccacac aacagcgcac 240
gtgctggccc aagcagtaaa acggctgtac ggcgaagtgt cattgggggt agggcctgtg 300
attttagacg gcttctacta tgacatgaag ctggggaaaa gcttggcatc aggagactta 360
gaggccatcg aaaaagaaat gaaaaacata ataaatgaga accttgaaat caaacgtata 420
gaggtttctt acgaagaagc tgaagagctg tttgcacaaa aagacgagcg gctcaagctt 480
gagattttaa aggatattcc gcgcggggag gatatcacac tgtaccagca aggggaattc 540
gttgacctgt gccgagggcc gcatcttcct tctacaggga tgatcaaggc gttcaaactg 600
acaagagtat caggcgctta ttggcgcgga gacagcaaaa atgaagtcct gcagcgggtg 660
tacggggttg cgtttcaaaa gaaaaaagat ttggatgcgc acctgcatat gctagaagaa 720
gcggcaaaac gagaccatcg caagctcggc aaacagttag ggctgtttat gttttctgaa 780
gaagcgccgg ggatgccgtt ttatttgccg aaagggcaga ttgtccgcaa tgagctggag 840
cgcttctcgc gtgagctgca aacaaatgcc ggctacgatg aggtgcgtac gccgtttatg 900
atgaatcagc ggttatggga gcagtcaggc cattgggacc attatcgcga caatatgtac 960
ttttcagaag tggatgatac aagattcgca atgaagccga tgaattgccc cggccacatg 1020
ctcatcttta aaaacagcct gtattcttat cgggatctgc cgattcgcat ggcggaattc 1080
ggacaggtgc accgccatga atacagcggc gctttaaacg gaatgcttcg cgttcggacg 1140
ttctgtcagg atgacgccca tattttcgtt cgggaggatc agattgagag tgagatcaaa 1200
gaagcgattc ggctgattga tgaggtatat cggacatttg gtttcgagta ttctgttgag 1260
ctttcgactc gcccggagga ttcattaggc gatgacagcc tttgggaagc gtcggaacgt 1320
gccttagcac gcgtccttga ggagcttggc ctctcttatg agatcaatga aggcgatggg 1380
gcgttttacg ggccgaaaat tgatttccat ataaaagatg ccctgaaacg gagccaccaa 1440
tgcgcgacaa ttcagcttga ttttcaaatg ccggaaaaat ttgatttaac atatatcaac 1500
gaactcaatg agaaggttcg tcccgttgtc atccacagag cggttttcgg ctcaatcgac 1560
cgtttcttcg gcattttgat cgagcattac ggtggggctt ttccagtctg gctcgcaccg 1620
atccaggttc aaatcatacc ggtttcgcac gttcacttag actattgcag aaaagtgcag 1680
gcggagctga agcaggcggg gataagagcc ggcattgatg agcgcaatga gaagctcggc 1740
tataaaatca gagaatcgca ggtgcaaaaa attccgtacg tgctggtgct tggagatcat 1800
gaggagcagg agaatgcggt aaacgtccgc cgattcggac atcagcagaa tgaacacgtt 1860
cctttccaaa cttttaaaga taaacttgtg aaacaggtgg aaaatcgagg catg 1914

<210> 47
<211> 990
<212> DNA
<213> B. subtilis

<220>
<223> tryptophanyl-tRNA synthetase

<400> 47
atgaaacaaa cgattttttc aggcattcag ccaagcggct cagtgacgct cggcaactat 60
atcggtgcaa tgaagcagtt tgtcgaactg cagcatgatt ataacagcta tttttgcatc 120
gtcgatcagc atgcgataac tgttcctcaa gaccggcttg agcttagaaa gaatatccgc 180
aatctcgcgg cgctttactt agctgtcgga cttgatccag aaaaagcaac attgtttatt 240
cagtcagagg tccccgcaca tgcgcaggcc ggatggatga tgcagtgtgt cgcctatatc 300
ggcgagcttg agcggatgac tcaatttaag gacaaatcca aaggcaatga agctgtcgtc 360
tccggcctgt taacatatcc gccgctgatg gccgctgata ttctgctgta cggaacggat 420

cttgtacctg tcggcgagga tcaaaagcag caccttgagc tgacgcggaa tcttgcagaa 480
cgcttcaaca aaaaatacaa cgacatcttt acgattccgg aagtgaaaat tccaaaagtc 540
ggtgcacgta tcatgtctct gaatgatccg ctgaagaaaa tgagcaaatc tgatccgaat 600
cagaaagctt atattacatt gctggatgag ccgaagcagc ttgaaaagaa aatcaaaagc 660
gcagtaacgg attctgaagg cattgtcaaa tttgataagg aaaacaaacc gggcgtttcc 720
aaccttctta caatttattc aatcctcggc aatacgacaa ttgaagagct tgaagcaaag 780
tacgaaggaa aaggctacgg cgagtttaaa ggtgatttgg cagaagtcgt agtgaacgca 840
ttaaaaccga tccaggaccg ctattacgag ctgatagaat ctgaagaatt agaccggatt 900
cttgatgaag gcgcggaacg agcgaatcgg acagcaaaca aaatgctgaa aaaaatggag 960
aatgccatgg gtcttggaag aaaaagacgc 990

<210> 48
<211> 1266
<212> DNA
<213> B. subtilis

<220>
<223> tyrosyl-tRNA synthetase, major

<400> 48
atgactaact tacttgaaga cttatccttc cgcggattga ttcagcaaat gacagatgaa 60
gaagggttaa ataaacagct gaatgaagaa aaaatccgct tgtactcagg ctttgaccct 120
acagcagaca gcttgcatat cggacacctg ctgcctattt taacacttcg ccgttttcag 180
cttgcagggc atcatccgat tgcgcttgtg ggcggcgcga cgggcctcat cggtgatcca 240
agcggaaaaa aagcagagcg tacattaaac actgctgaca tcgtatccga atggtcccaa 300
aaaatcaaaa accagctgtc cagatttcta gattttgaag cagcagaaaa cccagctgtc 360
atcgcgaaca actttgactg gatcggcaaa atgaatgtga tcgatttcct tcgtgacgtt 420
ggtaaaaatt tcggcatcaa ttacatgctg gcaaaagata cggtcagctc aagaattgaa 480
tccggcatct cgtacacgga attcagctac atgattcttc aatcttatga tttcttaaat 540
ctttacagag acaaaaactg taaactgcaa atcggcggaa gcgatcagtg gggcaacatc 600
acagcgggtc ttgaactgat cagaaaatca gaagaagaag gagcaaaagc gtttggccta 660
acgattccgc ttgtcacaaa agcagacggc acgaagttcg gaaaaacgga aggcggcgcg 720
atctggcttg ataaagaaaa aacatcgcca tatgaattct atcaattctg gatcaacaca 780
gacgaccgtg acgttgtcaa atacttgaaa tacttcacgt tcctttcaaa agaggaaatt 840
gaagcatacg ctgaaaaaac agaaacggcg ccagaaaaac gtgaagcgca aaaacgcctg 900
gcagaagaag tgacatcact tgttcacgga cgcgaggcgc tggagcaagc catcaacatc 960
tcccaagcgc tgttcagcgg caatattaaa gagctttctg ctcaagacgt aaaagtaggc 1020
tttaaagatg ttccttctat ggaagttgac agcacccaag agctttcact tgtagatgtg 1080
ttggtgcaat ctaaattatc tccttctaaa cgccaagcgc gtgaagacat ccaaaacgga 1140
gctgtttaca ttaacggtga acgccagacg gaaataaatt ataccctatc aggtgaagac 1200
cgcatcgaaa accaatttac tgttctgcgc cgcgggaaga aaaaatactt ccttgtgacg 1260
tataaa 1266

<210> 49
<211> 1239
<212> DNA
<213> B. subtilis

<220>
<223> tyrosyl-tRNA synthetase, minor

<400> 49
atgatgagaa catttgagca gctcacagcg tcacaacaaa aagaggtaga aagacagctt 60
cagctataca tgacaggcgc ccacgaagtc ataccgccgg aggaattaaa ggcaaagctc 120
gtgaaatcaa tttccacggg cacgccgctt aaaattaagc tcggattaga tccgtctgca 180
ccggatgtac atttgggcca tacggttgtg ttaaataagc ttcgccaatt tcaagaaaac 240
ggccacattg tccagctgtt aattggggat ttcacaggaa aaattggtga tccgaccgga 300
aaatcggcag ccagaaagca actgactgat gaagaagttc agcacaatgc caaaacctac 360
tttgagcaat tcggaaaagt gcttgatcca gaaaaagtcg agcttcacta taactcaaaa 420
tggctgaaaa cattgaatct agaagatgtc attgaattag cagggaaaat aacggtagcc 480
cgcctgatgg agcgcgacga ctttgaagaa cgcatcgcca tgcaaaaacc aatctcactg 540
catgaattct tttacccatt gatgcagggc tatgattctg tcgttctcga aagtgatatt 600
gaattaggcg gaacggatca gcatttcaat gtcctcatgg gacggcattt ccaagaacga 660
tacaacaaag aaaaacaagt cgtcatcctt atgccgctct tggaaggctt ggatggcgtc 720
gagaaaatgt cgaagtcgaa acacaactac attggcatta acgaacatcc aaacgacatg 780
tacggaaaaa cgatgtcact gcccgacagc ctgatgaaaa agtacatcca cttggcgaca 840
gacttagagc ttgaagagaa aaaacagctc gtaaaagact tagaaaccgg cgccgttcat 900
ccgcgtgatg ccaaaatgct tttagccaga acaatcgtcc gaatgtatca cggagagaaa 960
gcagcagaag ctgccgaaca ctcgtttaaa acagtctttc aggaaaacag cctgccggaa 1020
gatataccgg ccgtaaactg gaaaggcgaa aaaacgatag cgatgattga tctgctcgtc 1080
aagctaaagc tcctctcttc gaagagcgag gcgcgccgca tgattcaaaa cggaggtgta 1140
cgcatcgacg gagagaaagt aacagatgtt cacgccaaag cagagataag agagaatatg 1200
atcatccaag tcggcaaacg caagttttta aagctccaa 1239

<210> 50
<211> 2640
<212> DNA
<213> B. subtilis

<220>
<223> valyl-tRNA synthetase

<400> 50
atggaaacga atgaacaaac aatgccgacg aaatatgatc cggcagcggt tgaaaaagac 60
cgatatgatt tttggctaaa aggcaaattt tttgaagcgg gcagtgacca gacaaaagag 120
ccatactctg ttgtcatccc gccgccaaac gtaacaggga gacttcacct tggccacgca 180
tgggacacaa cgctgcaaga cattgtaaca agaatgaaac gcatgcaggg ctatgatgtg 240
ctgtggcttc ctggcatgga ccacgccggc atcgcgacac aggctaaagt ggaggcgaaa 300
cttcgtgaag aaggcaaatc ccgctacgat ttaggccgtg aaaaattcct cgaagaaacg 360
tggaagtgga aggaagaata tgctgacttt attcgcagcc agtgggcgaa attaggtctt 420
ggcctagatt attcccgtga acgttttacg ctggatgaag gcttgagcaa agcggtaagg 480
gaagtattcg ttaagcttta tgaaaaaggc ctgatctaca gaggcgaata tattattaac 540
tgggacccag cgacaaaaac agccctatcc gacattgagg tgatttacaa ggatgttcaa 600
ggcgcattct atcatatgag ctatccgctt gctgacggtt caggctcaat tgaaattgcg 660
acgacacgtc ctgaaacaat gctcggtgat acagctgttg ccgttcaccc tgaagacgag 720
cgctataaac accttatcgg aaaaacggtg atcctgccga ttgtaaatcg tgaaattccg 780

attgtcggtg atgactatgt tgatatggaa tttggttcag gtgccgtgaa aattacgcct 840
gctcatgatc cgaacgactt cgagcttggc aaccgccaca atttagaacg catccttgtc 900
atgaatgaag atggcacgat gaatgaaaat gcgctccaat atcaaggtat ggatcgtttt 960
gaatgccgca aaaagctcgt gaaagattta caggaggcgg gagttctctt caagatcgag 1020
gatcatatgc actctgtcgg ccatagtgaa cgaagcgggg ctgttgtgga gccttatctt 1080
tctacacaat ggtttgtgcg catgcagccg cttgctgatg cagcgattga gctcgaaaaa 1140
aaagaagaaa aggtcaactt tgtgcctgac cgtttcgaaa aaacgtattt acactggatg 1200
gaaaatatcc gcgactggtg tatctcccgt caattgtggt ggggccatcg tattcctgcc 1260
tggtatcata aagaaacagg cgagctatat gtcggacttg aagcgccgga agacagcgaa 1320
aattgggaac aggatacaga cgtactggat acatggttca gttctgcgct atggcctttc 1380
tccacaatgg gctggcctga tgtaacggct gaagacttta aacgctacta tccgacagat 1440
gttctcgtga cagggtacga tatcattttc ttctgggtat cacgcatgat tttccaaggc 1500
attgaattta caggcgagcg cccgttcaaa gatgttttaa tccacggctt aatccgtgac 1560
gagcaaggcc ggaaaatgag taaatctctt ggcaacggtg tcgacccgat ggatgtgatt 1620
gacaagtacg gagccgattc tctgcgttat ttccttgcga ctggaagctc tcctggccag 1680
gatctgcgct tcagctatga aaaagtggaa tcgacctgga attttgccaa taaaatttgg 1740
aacgcctccc gttttgcatt aatgaatatg gatggcatga cgtacgatga gcttgatctg 1800
tcaggtgaaa agtcagtcgc tgacaagtgg attttaacgc gattaaatga aacgatcgag 1860
cacgtgaccc agcttgctga ccgatatgaa tttggtgaag taggacgcca tttatataac 1920
tttatttggg atgatttctg tgattggtat attgaaatgg cgaagcttcc gctttatgga 1980
gaagacgaag cggctaagaa aactacccgt tccatccttg cttacgtact tgatcaaacg 2040
atgcgtctgc tccatccgtt tatgccattc ttaacggagg aaatttggca gcaccttcct 2100
caccaagggg aatcgattac agtaagtcaa tggcctgcag tagtgccgga gcataccgat 2160
actgaagcgg cagctgacat gaagcttctt gttgagctga tccgttctgt gcgcaatatc 2220
cgcagtgaag tcaatacgcc aatgagcaag caggttgaac tgtacattaa aacaagcaca 2280
gacgaaatag cgtcccgcct cgaagcgaac cgttcatatg ttgaacgctt tacgaatccg 2340
agcgtgctta aaatcggcac cgatattgag gctgttgata aggcgatgac ggccgttgtc 2400
tcaggagcag aggttattct tccgcttgaa ggcttaatca atattgatga agaaattgcc 2460
cgtctgcaaa aagaatttga taaactgaca aaagaagttg agcgtgtcca aaagaaactt 2520
ggaaatgaag gatttatgaa aaaagcgcct gcacacgtaa ttgatgaaga acgtgaaaaa 2580
gaaaaagatt atgtggcgaa gcgtgacgct gttcaaaaac gaatggctga gctgaagggt 2640

<210> 51
<211> 1431
<212> DNA
<213> B. subtilis

<220>
<223> glutamyl-tRNA amidotransferase, subunit B

<400> 51
ttgaactttg aaacggtaat cggacttgaa gtccacgttg agttaaaaac aaaatcaaaa 60
attttctcaa gctctccaac gccattcggc gcggaggcga atacgcagac aagcgttatt 120
gacctcggat atccgggcgt cctgcctgtt ctgaacaaag aagccgttga attcgcaatg 180
aaagccgcta tggcgctcaa ctgtgagatc gcaacggata cgaagtttga ccgcaaaaac 240
tatttctatc ctgacaaccc gaaagcgtat cagatttctc aatttgataa gccaatcggc 300
gaaaacggct ggatcgaaat tgaagtcggc ggcaaaacaa aacgcatcgg catcacgcgc 360
cttcatcttg aagaggatgc cggaaaactg acgcatacgg gcgacggcta ttctcttgtt 420

gacttcaacc gtcaaggaac gccgcttgtt gagatcgtat cagagccgga catccgcacg 480
ccggaagaag cgtacgcata tcttgaaaag ctgaaatcca tcatccaata tacaggcgtt 540
tctgactgta aaatggaaga aggctcactt cgctgtgacg ccaatatctc tcttcgtccg 600
atcggccaag aggaattcgg cacaaaaaca gaattgaaaa acttgaactc ctttgcgttt 660
gttcaaaaag gccttgagca tgaagaaaaa cgccaggagc aggttcttct ttccggcggc 720
gtcatccagc aagaaactcg ccgttatgat gaagcaacga agaaaaccat tcttatgcgt 780
gtcaaagagg gatctgacga ctaccgttac ttcccagagc cagatctagt cgagctctac 840
attgatgatg aatggaagga acgcgtaaaa gcaagcattc ctgagcttcc ggatgagcgc 900
cgcaagcgtt atatcgaaga gcttggcttg cctgcatatg acgcaatggt tctgacgctg 960
acaaaagaaa tggctgattt cttcgaagaa accgttcaaa aaggcgctga agctaaacaa 1020
gcgtctaact ggctgatggg tgaagtgtca gcttacctaa acgcagaaca aaaagagctt 1080
gccgatgttg ccctgacacc tgaaggcctt gccggcatga tcaaattgat tgaaaaagga 1140
accatttctt ctaagatcgc gaagaaagtg tttaaagaat tgattgaaaa aggcggcgac 1200
gctgagaaga ttgtgaaaga gaaaggcctt gttcagattt ctgacgaagg cgtgcttctg 1260
aagcttgtca ctgaggcgct tgacaacaat cctcaatcaa tcgaagactt taaaaacgga 1320
aaagaccgcg cgatcggctt cctagtcgga cagattatga aagcgtccaa aggacaagcc 1380
aacccgccga tggtcaacaa aattctgctt gaagaaatta aaaaacgcta a 1431

<210> 52
<211> 291
<212> DNA
<213> B. subtilis

<220>
<223> glutamyl-tRNA amidotransferase, subunit C

<400> 52
atgtcacgaa tttcaataga agaagtaaag cacgttgcgc accttgcaag acttgcgatt 60
actgaagaag aagcaaaaat gttcactgaa cagctcgaca gtatcatttc atttgccgag 120
gagcttaatg aggttaacac agacaatgtg gagcctacaa ctcacgtgct gaaaatgaaa 180
aatgtcatga gagaagatga agcgggtaaa ggtcttccgg ttgaggatgt catgaaaaat 240
gcgcctgacc ataaagacgg ctatattcgt gtgccatcaa ttctggacta a 291

<210> 53
<211> 31
<212> DNA
<213> artificial
<400> 53
gtgcctcgag gattaagcat tggtaactgt c 31

<210> 54
<211> 34
<212> DNA
<213> artificial
<400> 54
tattctcgag acattaacct agaaagcact aagg 34

<210> 55

<211> 30
<212> DNA
<213> artificial
<400> 55
ctgcctgtct cgaggctgat agcagttatc 30

<210> 56
<211> 30
<212> DNA
<213> artificial
<400> 56
caactcgagg ctggtcggac aaacacctag 30

<210> 57
<211> 30
<212> DNA
<213> artificial
<400> 57
tgcgaattcc ggatgtaagg agaacggctc 30

<210> 58
<211> 32
<212> DNA
<213> artificial
<400> 58
cttgtcgacg tacatcagaa atcgtacatt cg 32

<210> 59
<211> 30
<212> DNA
<213> artificial
<400> 59
ggtggatccc ctcgccggca atagttaccc 30

<210> 60
<211> 31
<212> DNA
<213> artificial
<400> 60
aatctagact ttcgttatac aaattttaac c 31

<210> 61
<211> 31
<212> DNA
<213> artificial
<400> 61
tgtaggatcc aatatgggca gaaaacacat g 31

<210> 62
<211> 31
<212> DNA
<213> artificial
<400> 62
atatctagat tccactctgt gtaccagtag c  31

<210> 63
<211> 20
<212> DNA
<213> artificial
<400> 63
gttagctcct tcggtcctcc  20

<210> 64
<211> 20
<212> DNA
<213> artificial
<400> 64
gtaactggct tcagcagagc  20

<210> 65
<211> 21
<212> DNA
<213> artificial
<400> 65
ctgccacatc gtctaggctg c  21

<210> 66
<211> 20
<212> DNA
<213> artificial
<400> 66
caggagtcca aataccagag  20

<210> 67
<211> 20
<212> DNA
<213> artificial
<400> 67
cccttgccta tactgtggac  20

<210> 68
<211> 20
<212> DNA
<213> artificial
<400> 68
gttacaatag cgacggagag  20

<210> 69
<211> 27
<212> DNA
<213> artificial
<400> 69
gtctgaattc gaggaaggtt tacaccg  27

<210> 70
<211> 26
<212> DNA
<213> artificial
<400> 70
ctgcgaattc atcttcatgg tgaacc  26

<210> 71
<211> 20
<212> DNA
<213> artificial
<400> 71
cattgtcatt agttggctgg  20

<210> 72
<211> 28
<212> DNA
<213> artificial
<400> 72
gcaagaattc aaaatccatc ttcatcgg  28

<210> 73
<211> 29
<212> DNA
<213> artificial
<400> 73
gtgactgagg atccgctagt tccagatcg  29

<210> 74
<211> 30
<212> DNA
<213> artificial
<400> 74
ctcttggatc ccttcatcat tctatcacac  30

<210> 75
<211> 25
<212> DNA
<213> artificial
<400> 75

gcagattggg atggagttcc cgtac  25

<210> 76
<211> 24
<212> DNA
<213> artificial
<400> 76
ttacagcttc acaaagcgga tttc  24

<210> 77
<211> 26
<212> DNA
<213> artificial
<400> 77
tgctgcaaga gctgtcggaa ataaag  26

<210> 78
<211> 24
<212> DNA
<213> artificial
<400> 78
aagaagaaaa gaaagaagct aaag  24

<210>  79
<211>  31
<212>  DNA
<213>  Artificial
<220>
<223>  primer
<400>  79
gtgcctcgag gattaagcat tggtaactgt c                                    31

<210>  80
<211>  34
<212>  DNA
<213>  Artificial
<220>
<223>  primer
<400>  80
tattctcgag acattaacct agaaagcact aagg                                 34

<210>  81
<211>  32
<212>  DNA
<213>  Artificial
<220>
<223>  primer
<400>  81

ctgctatgaa ttcgagtagt acacgtttca cc 32

<210> 82
<211> 28
<212> DNA
<213> Artificial
<220>
<223> primer
<400> 82
ctgaattctg gtcggacaaa cacctagc 28

<210> 83
<211> 29
<212> DNA
<213> Artificial
<220>
<223> primer
<400> 83
cggagaattc tccgttacaa acgtcagag 29

<210> 84
<211> 29
<212> DNA
<213> Artificial
<220>
<223> primer
<400> 84
actgaattct ggtcggacaa acacctagc 29

<210> 85
<211> 30
<212> DNA
<213> Artificial
<220>
<223> primer
<400> 85
ctcgaggctg aattcagtta tcatttaagg 30

<210> 86
<211> 31
<212> DNA
<213> Artificial
<220>
<223> primer
<400> 86
gggaattcgg acaaacacct agctttttgc g 31

<210> 87
<211> 1394
<212> DNA
<213> Artificial
<220>
<223> DNA sequence

<400> 87
gaattcgagt agtacacgtt tcacctgttt gaagagagtc tgcggtgctg ggagcagata 60
acgggcaatg tggaatggac ttcggagcag ctgaccgaac tggaaaaaag taggctcagc 120
cggagcagtc tccgttacaa acgtcagagt gattccattt taatggaata atcagggtgg 180
taccacggtt cattcgtccc ttttttacag gggaagaatg agcctttttt attatgtttt 240
aagaaatggg gttgatgttt tcttgaaaca aacgattttt tcaggcattc agccaagcgg 300
ctcagtgacg ctcggcaact atatcggtgc aatgaagcag tttgtcgaac tgcagcatga 360
ttataacagc tatttttgca tcgtcgatca gcatgcgata actgttcctc aagaccggct 420
tgagcttaga aagaatatcc gcaatctcgc ggcgctttac ttagctgtcg gacttgatcc 480
agaaaaagca acattgttta ttcagtcaga ggtccccgca catgcgcagg ccggatggat 540
gatgcagtgt gtcgcctata tcggcgagct tgagcggatg actcaattta aggacaaatc 600
caaaggcaat gaagctgtcg tctccggcct gttaacatat ccgccgctga tggccgctga 660
tattctgctg tacggaacgg atcttgtacc tgtcggcgag gatcaaaagc agcaccttga 720
gctgacgcgg aatcttgcag aacgcttcaa caaaaaatac aacgacatct ttacgattcc 780
ggaagtgaaa attccaaaag tcggtgcacg tatcatgtct ctgaatgatc cgctgaagaa 840
aatgagcaaa tctgatccga atcagaaagc ttatattaca ttgctggatg agccgaagca 900
gcttgaaaag aaaatcaaaa gcgcagtaac ggattctgaa ggcattgtca aatttgataa 960
ggaaaacaaa ccgggcgttt ccaaccttct tacaatttat tcaatcctcg gcaatacgac 1020
aattgaagag cttgaagcaa agtacgaagg aaaaggctac ggcgagttta aaggtgattt 1080
ggcagaagtc gtagtgaacg cattaaaacc gatccaggac cgctattacg agctgataga 1140
atctgaagaa ttagaccgga ttcttgatga aggcgcggaa cgagcgaatc ggacagcaaa 1200
caaaatgctg aaaaaaatgg agaatgccat gggtcttgga agaaaaagac gctaatcaaa 1260
aaaccgctct ttgcaaagag cggttttttt cagttgacct ttgattcgtt ttccatttcc 1320
caaagctttt cgaaaaaagg ctgtcctttt attaggtttt cgcaaaaagc taggtgtttg 1380
tccgaccaga attc 1394

<210> 88
<211> 1270
<212> DNA
<213> Artificial
<220>
<223> DNA sequence
<400> 88
gaattctccg ttacaaacgt cagagtgatt ccattttaat ggaataatca gggtggtacc 60
acggttcatt cgtccctttt ttacagggga agaatgagcc ttttttatta tgttttaaga 120
aatggggttg atgttttcgt gaaacaaacg attttttcag gcattcagcc aagcggctca 180
gtgacgctcg gcaactatat cggtgcaatg aagcagtttg tcgaactgca gcatgattat 240
aacagctatt tttgcatcgt cgatcagcat gcgataactg ttcctcaaga ccggcttgag 300
cttagaaaga atatccgcaa tctcgcggcg ctttacttag ctgtcggact tgatccagaa 360
aaagcaacat tgtttattca gtcagaggtc cccgcacatg cgcaggccgg atggatgatg 420
cagtgtgtcg cctatatcgg cgagcttgag cggatgactc aatttaagga caaatccaaa 480

ggcaatgaag ctgtcgtctc cggcctgtta acatatccgc cgctgatggc cgctgatatt 540
ctgctgtacg gaacggatct tgtacctgtc ggcgaggatc aaaagcagca ccttgagctg 600
acgcggaatc ttgcagaacg cttcaacaaa aaatacaacg acatctttac gattccggaa 660
gtgaaaattc caaaagtcgg tgcacgtatc atgtctctga atgatccgct gaagaaaatg 720
agcaaatctg atccgaatca gaaagcttat attacattgc tggatgagcc gaagcagctt 780
gaaaagaaaa tcaaaagcgc agtaacggat tctgaaggca ttgtcaaatt tgataaggaa 840
aacaaaccgg gcgtttccaa ccttcttaca atttattcaa tcctcggcaa tacgacaatt 900
gaagagcttg aagcaaagta cgaaggaaaa ggctacggcg agtttaaagg tgatttggca 960
gaagtcgtag tgaacgcatt aaaaccgatc caggaccgct attacgagct gatagaatct 1020
gaagaattag accggattct tgatgaaggc gcggaacgag cgaatcggac agcaaacaaa 1080
atgctgaaaa aaatggagaa tgccatgggt cttggaagaa aaagacgcta atcaaaaaac 1140
cgctctttgc aaagagcggt ttttttcagt tgacctttga ttcgttttcc atttcccaaa 1200
gcttttcgaa aaaaggctgt ccttttatta ggttttcgca aaaagctagg tgtttgtccg 1260
accagaattc 1270

<210> 89
<211> 1531
<212> DNA
<213> Artificial
<220>
<223> DNA sequence
<400> 89
gaattcagtt atcatttaag gattttacag cactcgtgtt atttatatta ttacaatttt 60
actggaaaaa agaatgtttt ttcatgatga caattggtta taattgattt ataatggagc 120
aatcaaaaga aaagcttgct atgaaaaaga gtagtacacg tttcacctgt ttgaagagag 180
tctgcggtgc tgggagcaga taacgggcaa tgtggaatgg acttcggagc agccgaccga 240
actggaaaaa agtaggctca gccggagcgg tctccgttac aaacgtcaga gtgattccat 300
tttaatggaa taatcagggt ggtaccacgg ttcattcgtc ccttttttac aggggaagaa 360
tgagcctttt ttattatgtt ttaagaaatg gggttgatgt tttcatgaaa caaacgattt 420
tttcaggcat tcagccaagc ggctcagtga cgctcggcaa ctatatcggt gcaatgaagc 480
agtttgtcga actgcagcat gattataaca gctatttttg catcgtcgat cagcatgcga 540
caaccgttcc tcaagaccgg cttgagctta gaaagaatat ccgcaatctc gcggcgcttt 600
acttagctgt cggacttgat ccagaaaaag caacattgtt tattcagtca gaggtccccg 660
cacatgcgca ggccggatgg atgatgcagt gtgtcgccta tatcggcgag cttgagcgga 720
tgactcaatt taaggacaaa tccaaaggca atgaagctgt cgtctccggc ctgttaacat 780
atccgccgct gatggccgct gatattctgc tgtacggaac ggatcttgta cctgtcggcg 840
aggatcaaaa gcagcacctt gagctgacgc ggaatcttgc agaacgcttc aacaaaaaat 900
acaacgacat ctttacgatt ccggaagtga aaattccaaa agtcggtgca cgtatcatgt 960
ctctgaatga tccgctgaag aaaatgagca aatctgatcc gaatcagaaa gcttatatta 1020
cattgctgga tgagccgaag cagcttgaag agaaaatcaa gagcgcagta acggattctg 1080
aaggcattgt caaatttgat aaggaaaaca aaccgggcgt ttccaacctt cttacaattt 1140
attcaatcct cggcaatacg acaattgaag agcttgaagc aaagtacgaa ggaaaaggct 1200
acggcgagtt taaaggtgat ttggcagaag tcgtagtgaa cgcattagaa ccgatccagg 1260
accgctatta cgagctgata gaatctgaag aattagaccg gattcttgat gaaggcgcgg 1320
aacgagcgaa tcggacagca aacaaaatgc tgaaaaaaat ggagaacgcc atgggtcttg 1380
gaagaaaaag acgctaatcg aaaaaccgct ctttgcaaag ggcggttttt ttcagttgac 1440
ctttgattcg ttttccatct cccaaagctt ttcgaaaaaa ggctgtcctt ttattaggtt 1500

ctcgcaaaaa gctaggtgtt tgtccgaatt c 1531

## Description

### [Technical Field]

The present invention relates to methods for stably retaining foreign genes within cells. More specifically, the present invention relates to a recombinant vector comprising a gene encoding an aminoacyl-tRNA synthetase and a mutant host cell employed to express a foreign gene other than a gene encoding an aminoacyl-tRNA synthetase, that are employed in the above methods; and methods of preparing a protein or a peptide encoded by a foreign gene by expressing a foreign gene other than a gene encoding an aminoacyl-tRNA synthetase. As a cross-reference to a related patent application, the present application claims priority under Japanese Patent Application No. 2007-183391 filed on July 13, 2007, the entire contents of which are hereby incorporated by reference.

### [Background Art]

In attempts to produce target molecules with transformed cells obtained by the practical application of recombinant DNA techniques, there are problems in that the presence of an extrachromosomal gene in a transformed cells (that is, the targeted foreign gene contained therein) is generally highly unstable and the production of the targeted molecule is extremely difficult.

The cloning and expression vectors commonly employed in laboratories are normally multicopy recombinant vectors the stable transmission of which to subsequent generations is ensured by a large number of recombinant vectors for a single cell genome. However, the introduction of foreign genes into these recombinant vectors imparts various degrees of instability during the growth cycle of the cells. In industrial production processes, 1,000 L of culture product is required in some cases, necessitating 10¹⁶ or more cells following a succession of 50 generations or more. Accordingly, to achieve the reliable presence of the recombinant vector within the cell, and thus expression of the foreign gene, it is desirable to stabilize the recombinant vector in the cell through to the end of culturing in the fermentation vat.

There are several known methods of stabilizing a recombinant vector comprising a foreign gene in a cell.

One method consists of incorporating a gene imparting resistance to an antibiotic into a recombinant vector and adding a suitable antibiotic to the culture medium. Those cells having the recombinant vector comprising the gene for resistance to the antibiotic are selected, and those cells that do not have the recombinant vector are not selected, thereby eliminating them.

The stabilization of a recombinant vector by incorporating a gene imparting resistance to an antibiotic is regularly conducted in the laboratory. However, for the following reasons, it is undesirable in production on an industrial scale:
(i) the use of bacterial strains that are resistant to antibiotics is potentially dangerous to the environment;
(ii) the quantity of antibiotic required in the culture significantly increases production costs; and
(iii) the use of antibiotics is to be avoided in the production of substances intended for use by humans and in animals.

The method of supplementing a nutritional requirement mutation in a chromosome is also a known recombinant vector stabilization method (see Genetics. 1989 May; 122 (1): 19-27 (also referred to as Nonpatent Document 1 hereinafter) and Curr Genet. 1989 Sep; 16 (3): 159-63 (also referred to as Nonpatent Document 2 hereinafter); these descriptions are specifically incorporated herein by reference). In this method, the composition of the fermentation medium is strictly limited and fermentation must be conducted without adding a nutrient required by the host cell to the medium. Further, through syntrophism, it is possible for the cells to grow even once the recombinant vector has disappeared.

### [Disclosure of Invention]

### [Problem to Be Solved by the Invention]

Thus, the two above-described selection methods depend on special processing of the medium. Such limitations tend to increase the cost of the fermentation process and restrict the freedom of choice in enhancing production efficiency.

Accordingly, there is a strong need for a selection method permitting the retention of the recombinant DNA cloning vector without using antibiotics and without restricting the composition of the medium.

Accordingly, the present invention has for its object to provide a new means of retaining a recombinant DNA cloning vector without the use of an antibiotic and without restrictions, and a method of preparing a protein or a peptide encoded by a foreign gene using this means.

### [Means of Solving the Problem]

Extensive research was conducted to solve the above problems. The present invention was devised on the basis of the discovery that a recombinant DNA cloning vector could be retained based on the complementarity between an extrachromosomal gene comprising an aminoacyl-tRNA synthetase gene and a chromosomal mutant host cell in which aminoacyl-tRNA synthetase activity had been knocked out, without using an antibiotic and without restricting the composition of the medium. The present invention is based on ensuring the survival of only those cells comprising an extrachromosomal gene containing an aminoacyl-tRNA synthetase gene.

The present invention is as follows:
(1) A recombinant vector, comprising a site permitting the insertion in an expressible state of a foreign gene other than a gene encoding an aminoacyl-tRNA synthetase and comprising a gene encoding aminoacyl-tRNA synthetase in an expressible state, for use in a mutant host in which a chromosomal gene encoding an aminoacyl-tRNA synthetase has been knocked out, or in a mutant host in which expression of a chromosomal gene encoding an aminoacyl-tRNA synthetase has been diminished to a degree to which the host cell cannot grow.
(2) The recombinant vector according to (1), wherein said aminoacyl-tRNA synthetase is tryptophanyl-tRNA synthetase, alanyl-tRNA synthetase, arginyl-tRNA synthetase, asparginyl-tRNA synthetase, aspartyl-tRNA synthetase, cysteinyl-tRNA synthetase, glutamine-tRNA synthetase, glutamate-tRNA synthetase, glycine-tRNA synthetase, histidyl-tRNA synthetase, isoleucyl-tRNA synthetase, leucyl-tRNA synthetase, lysine-tRNA synthetase, methianyl-tRNA synthetase, phenylalanine-tRNA synthetase, prolyl-tRNA synthetase, seryl-tRNA synthetase, threonyl-tRNA synthetase, tyrosyl-tRNA synthetase, or valyl-tRNA synthetase.
(3) The recombinant vector according to (1) or (2), wherein said recombinant vector is in the form of a plasmid, bacteriophage, or retrotransposon.
(4) A mutant host cell in which a chromosomal gene encoding an aminoacyl-tRNA synthetase has been knocked out, or a mutant host in which the expression of a chromosomal gene encoding an aminoacyl-tRNA synthetase has been diminished to a degree where the host cell cannot grow, that is used for transformation by a recombinant vector comprising a site permitting the insertion in an expressible state of a foreign gene other than a gene encoding an aminoacyl-tRNA synthetase and comprising in an expressible state a gene encoding an aminoacyl-tRNA synthetase.
(5) The mutant host cell according to (4), wherein said aminoacyl-tRNA synthetase is tryptophanyl-tRNA synthetase, alanyl-tRNA synthetase, arginyl-tRNA synthetase, asparginyl-tRNA synthetase, aspartyl-tRNA synthetase, cysteinyl-tRNA synthetase, glutamine-tRNA synthetase, glutamate-tRNA synthetase, glycine-tRNA synthetase, histidyl-tRNA synthetase, isoleucyl-tRNA synthetase, leucyl-tRNA synthetase, lysine-tRNA synthetase, methionyl-tRNA synthetase, phenylalanine-tRNA synthetase, prolyl-tRNA synthetase, seryl-tRNA synthetase, threonyl-tRNA synthetase, tyrosyl-tRNA synthetase, or valyl-tRNA synthetase.
(6) The mutant host cell according to (4) or (5), wherein the host cell into which a mutation is introduced is a bacterium, yeast, animal cell, or plant cell.
(7) The mutant host cell according to (4) or (5), wherein the host cell into which a mutation is introduced is a bacterium or yeast.
(8) The mutant host cell according to (6) or (7), wherein the bacterium is a bacterium of the genus Bacillus.
(9) A method of preparing a protein or peptide encoded by a foreign gene by expressing a foreign gene other than a gene encoding an aminoacyl-tRNA synthetase, comprising:
   a step of preparing a recombinant vector, comprising a site permitting the insertion in an expressible state of a foreign gene and comprising in an expressible state a gene encoding an aminoacyl-tRNA synthetase, in which a desired foreign gene has been inserted at the site permitting the insertion in an expressible state of a foreign gene;
   a step of preparing a mutant host cell in which a chromosomal gene encoding an aminoacyl-tRNA synthetase has been knocked out or a mutant host cell in which the expression of a chromosomal gene encoding an aminoacyl-tRNA synthetase has been diminished to a degree where the host cell cannot grow;
   a step of transforming the mutant host cell with the recombinant vector to obtain a transformant; and
   a step of culturing the transformant to prepare the protein or peptide encoded by the foreign gene.
(10) A method of causing a targeted recombinant vector to be stably retained within a host cell, comprising:
   knocking out, or diminishing to a degree where the host cell cannot grow, a chromosomal gene encoding an aminoacyl-tRNA synthetase contained in the host cell, to convert the host cell to a mutant host cell;
   incorporating in an expressible state a gene encoding an aminoacyl-tRNA synthetase into the targeted recombinant vector; and
   transforming the mutant host cell with said targeted recombinant vector containing the gene encoding an aminoacyl-tRNA synthetase in an expressible state.
(11) The method according to (10), wherein the recombinant vector comprises a site permitting the insertion in an expressible state of a foreign gene other than a gene encoding an aminoacyl-tRNA synthetase.
(12) The method according to any one of (9) to (11), wherein said aminoacyl-tRNA synthetase is tryptophanyl-tRNA synthetase, alanyl-tRNA synthetase, arginyl-tRNA synthetase, asparginyl-tRNA synthetase, aspartyl-tRNA synthetase, cysteinyl-tRNA synthetase, glutamine-tRNA synthetase, glutamate-tRNA synthetase, glycine-tRNA synthetase, histidyl-tRNA synthetase, isoleucyl-tRNA synthetase, leucyl-tRNA synthetase, lysine-tRNA synthetase, methionyl-tRNA synthetase, phenylaianine-tRNA synthetase, prolyl-tRNA synthetase, seryl-tRNA synthetase, threonyl-tRNA synthetase, tyrosyl-tRNA synthetase, or valyl-tRNA synthetase.
(13) The method according to any one of (9) to (12), wherein said host cell into which a mutation is incorporated is a bacterium, yeast, animal cell, or plant cell.
(14) The method according to any one of (9) to (12), wherein said host cell into which a mutation is incorporated is a bacterium or yeast.
(15) The method according to (13) or (14), wherein said bacterium is a bacterium of the genus Bacillus.
(16) The method according to any one of (9) to (15), wherein the protein encoded by the foreign gene is an enzyme selected from the group consisting of oxidoreductases, transferases, hydrolases, phosphorylases, lyases, isomerases, ligases/synthetases, and modifying enzymes.
(17) Use of the recombinant vector according to any one of (1) to (3) to prepare a protein or peptide encoded by a foreign gene.
(18) Use of the mutant host cell according to any one of (4) to (8) to prepare a protein or peptide encoded by a foreign gene.

### [Effect of the Invention]

The present invention permits the retention of a recombinant DNA cloning vector without employing an antibiotic and without limiting the composition of the medium. As a result, it is possible to stabilize the recombinant vector in the cell through the end of cultivation in a fermentation vat, even in industrial production.

### [Best Modes of Carrying Out the Invention]

### [The recombinant vector]

The present invention relates to a recombinant vector (1) comprising in an expressible state a gene encoding an aminoacyl-tRNA synthetase, (2) comprising a site permitting the insertion in an expressible state of a foreign gene other than a gene encoding an aminoacyl-tRNA synthetase, (3) that is used in a mutant host in which a chromosomal gene encoding an aminoacyl-tRNA synthetase has been knocked out or in a mutant host in which the expression of a chromosomal gene encoding an aminoacyl-tRNA synthetase has been diminished to a degree preventing growth of the host cell.

(1) The recombinant vector comprising in an expressible state a gene encoding an aminoacyl-tRNA synthetase.

The recombinant vector of the present invention comprises in an expressible state a gene encoding an aminoacyl-tRNA synthetase. The recombinant vector is used to modify a host cell by incorporating in an expressible state the gene encoding an aminoacyl-tRNA synthetase into a desired host cell. Further, the recombinant vector of the present invention need only have a form permitting the transformation of the host cell; examples are recombinant vectors in the form of plasmids, bacteriophages, and retrotransposons.

In a manner corresponding to the 20 amino acids, there exist 20 types of aminoacyl-tRNA synthetases (some of which come in multiple forms). For example, the aminoacyl-tRNA synthetase corresponding to alanine is called alanyl-tRNA (alanyl-tRNA synthetase or alanine-tRNA synthetase). Specific examples of aminoacyl-tRNA synthetases are tryptophanyl-tRNA synthetase, alanyl-tRNA synthetase, arginyl-tRNA synthetase, asparginyl-tRNA synthetase, aspartyl-tRNA synthetase, cysteinyl-tRNA synthetase, glutamine-tRNA synthetase, glutamate-tRNA synthetase, glycine-tRNA synthetase, histidyl-tRNA synthetase, isoleucyl-tRNA synthetase, leucyl-tRNA synthetase, lysine-tRNA synthetase, methionyl-tRNA synthetase, phenylalanine-tRNA synthetase, prolyl-tRNA synthetase, seryl-tRNA synthetase, threonyl-tRNA synthetase, tyrosyl-tRNA synthetase, and valyl-tRNA synthetase.

The aminoacyl-tRNA synthetase corresponding to tryptophan is called tryptophanyl-tRNA synthetase. Cell strains lacking tryptophanyl-tRNA synthetase cannot synthesize proteins containing tryptophan and thus cannot proliferate.

Aminoacyl-tRNA synthetase genes are contained in all living organisms, from prokaryotes to eukaryotes. For example, *Escherichia. coli* contains: alanyl-tRNA synthetase (SEQ ID NO: 1), arginyl-tRNA synthetase (SEQ ID NO: 2), asparaginyl-tRNA synthetase (SEQ ID NO: 3), aspartyl-tRNA synthetase (SEQ ID NO: 4), cysteinyl-tRNA synthetase (SEQ ID NO: 5), glutamine-tRNA synthetase (SEQ ID NO: 6), glutamate-tRNA synthetase (SEQ ID NO: 7), glycine-tRNA synthetase, alpha subunit (SEQ ID NO: 8), glycine-tRNA synthetase, beta subunit (SEQ ID NO: 9), histidyl-tRNA synthetase (SEQ ID NO: 10), isoleucyl-tRNA synthetase (SEQ ID NO: 11), leucyl-tRNA synthetase (SEQ ID NO: 12), lysine-tRNA synthetase, constitutive (SEQ ID NO: 13), lysine-tRNA synthetase, inducible (SEQ ID NO: 14), methionyl-tRNA synthetase (SEQ ID NO: 15), phenylalanine-tRNA synthetase, alpha subunit (SEQ ID NO: 16), phe-nylalanine-tRNA synthetase, beta subunit (SEQ ID NO: 17), predicted lysyl-tRNA synthetase (SEQ ID NO: 18), prolyl-tRNA synthetase (SEQ ID NO: 19), seryl-tRNA synthetase, also charges selenocysteinyl-tRNA with serine (SEQ ID NO: 20), threonyl-tRNA synthetase (SEQ ID NO: 21), tryptophanyl-tRNA synthetase (SEQ ID NO: 22), tyrosyl-tRNA synthetase (SEQ ID NO: 23), and valyl-tRNA synthetase (SEQ ID NO: 24).

*Bacillus subtilis* is known to contain alanyl-tRNA synthetase (SEQ ID NO: 25), arginyl-tRNA synthetase (SEQ ID NO: 26, asparaginyl-tRNA synthetase (SEQ ID NO: 27), aspartyl-tRNA synthetase (SEQ ID NO: 28), cysteinyl-tRNA synthetase (SEQ ID NO: 29), glutamyl-tRNA synthetase (SEQ ID NO: 30), glutamyl-tRNA amidotransferase, subunit a (SEQ ID NO: 31), glycine-tRNA synthetase, alpha subunit (SEQ ID NO: 32), glycine-tRNA synthetase, beta subunit (SEQ ID NO: 33), histidyl-tRNA synthetase, hisS (SEQ ID NO: 34), isoleucyl-tRNA synthetase (SEQ ID NO: 35), leucyl-tRNA synthetase (SEQ ID NO: 36), lysine-tRNA synthetase, constitutive (SEQ ID NO: 37), histidyl-tRNA synthetase, hisZ (SEQ ID NO: 38), methionyl-tRNA synthetase (SEQ ID NO: 39), phenylalanine-tRNA synthetase, alpha subunit (SEQ ID NO: 40), phenylalanine-tRNA synthetase, beta subunit (SEQ ID NO: 41), similar to phenylaianine-tRNA synthetase, ytpR (SEQ ID NO: 42), prolyl-tRNA synthetase (SEQ ID NO: 43), seryl-tRNA synthetase, also charges selenocysteinyl-tRNA with serine (SEQ ID NO: 44), threonyl-tRNA synthetase, major (SEQ ID NO: 45), threonyl-tRNA synthetase, minor (SEQ ID NO: 46), tryptophanyl-tRNA synthetase (SEQ ID NO: 47), tyrosyl-tRNA synthetase, major (SEQ ID NO: 48), tyrosyl-tRNA synthetase, minor (SEQ ID NO: 49), valyl-tRNA synthetase (SEQ ID NO: 50), glutamyl-tRNA amidotransferase, subunit b (SEQ ID NO: 51), glutamyl-tRNA amidotransferase, subunit c (SEQ ID NO: 52), and the like.

The recombinant vector of the present invention comprises in an expressible state a gene encoding an aminoacyl-tRNA synthetase. Accordingly, when employing a bacterium such as *Escherichia coli* or *Bacillus subtilis* as a host to create an expressible state for a gene other than an aminoacyl-tRNA synthetase gene, the recombinant vector will generally comprise, for example, promoter and operator regions (including promoter, operator, and ribosome-binding regions (an SD region)), a start codon, DNA encoding the protein the production of which is targeted, a stop codon, a terminator region, and a plasmid-replicable unit. When employing an animal cell or a eukaryote such as yeast as the host cell, there will generally be a promoter, a start codon, DNA encoding a signal peptide, DNA encoding the protein the production of which is targeted, a stop codon, and the like. As needed, the recombinant vector of the present invention may also comprise cis elements such as enhancers, nontranslatable regions on the 5' or 3' end of the DNA encoding the protein the production of which is targeted, splicing joints, polyadenylation sites, replicable units, homologous regions, and selection markers. These elements are not specifically limited other than that they correspond to the host being used to express the gene encoding the protein the production of which is targeted, and may be selected based on common technical knowledge.

The selection marker is not specifically limited. When the host being used to express the gene is a bacterium, examples are genes imparting drug resistance (such as ampicillin-resistance genes, neomycin-resistance genes, cycloheximide-resistance genes, and tetramycin-resistance genes). When the host is not a bacterium, but is a yeast cell, for example, various known selection markers can be employed, including nutritional requirement genes (such as HIS4, URA3, LEU2, and ARG4).

(2) The recombinant vector of the present invention comprises a site permitting the insertion in an expressible state of a gene other than a gene encoding an aminoacyl-tRNA synthetase. This site, as set forth further below, permits the insertion in an expressible state of a foreign gene encoding the protein that is to be produced into the host cell of the present invention.

The phrase "permits the insertion of a gene in an expressible state" has the following meaning. Generally, the expression of a gene encoding a protein requires that information about the gene in the form of an RNA polymerase recognition site for transcription to mRNA and a ribosome-binding site for translating mRNA information into a peptide be present upstream from the start codon of the gene encoding the protein. In brief, the "presence of a gene in an expressible state" means that the gene, from start codon to end codon, must be positioned so that the sequence required for transcription to mRNA is present upstream from the start codon and the sequence required for translation of the mRNA information into a peptide is present upstream from the start codon. The "start codon" referred to here is not limited to the original start codon sequence of the gene on the chromosome, but may be any codon functioning as a start codon. The "ribosome binding-site" referred to here is not limited to a sequence having the consensus sequence consisting of 5'-aaaggagg-3' of a prokaryote, for example, but can be any sequence that is recognized by a ribosome. It is not limited to the sequences of the original ribosome-binding sites of the gene on the chromosome; any sequence recognizable by a ribosome may be employed.

The recombinant vector of the present invention can be constructed according to the usual methods employed in DNA recombination, such as the method described in Molecular Cloning (1989) (Cold Spring Harbor Lab). The description given in this document is specifically incorporated herein by reference.

The following vectors may be employed. Examples of plasmid vectors are pRS413, pRS415, pRS416, YCp50, pAUR112, pAUR123, and other YCp-type *Escherichia coli*-yeast shuttle vectors; pRS403, pRS404, pRS405, pRS406, pAUR101, pAUR135, and other Ylp-type *Escherichia coli*-yeast shuttle vectors; plasmids derived from *Escherichia coli* (such as pBR322, pBR325, pUC18, pUC19, pUC119, pTV118N, pTV119N, pBluescript, pHSG298, pHSG396, pTrc99A, and other ColE plasmids; pACYC177, pACYC184, and other p1A plasmids; pMW118, pMW119, pMW218, pMW219, and other pSC101 plasmids); plasmids derived from *Bacillus subtilis* (such as pUB110 and pTP5); and pHY300PLK and other *Escherichia coli-Bacillus subtilis* shuttle vectors. Examples of phage vectors are *lambda*-phages (such as Charon 4A, Charon 21A, EMBL4, *lambdag*t100, gt11, zap); *phi*X174; M13mp18; and M13mp19. An example of a retrotransposon is Ty factor. Examples of expression vectors expressed as fused proteins that can be employed are the pGEX series (made by Pharmacia) and the pMAL series (made by Biolabs).

(3) The recombinant vector of the present invention is employed in a mutant host in which a chromosomal gene encoding an aminoacyl-tRNA synthetase has been knocked out, or in a mutant host in which the expression of a chromosomal gene encoding an aminoacyl-tRNA synthetase has been diminished to a degree where the host cell cannot grow. The mutant host will be described further below.

### [The host cell (mutant host)]

The present invention relates to (4) a mutant host cell in which a chromosomal gene encoding an aminoacyl-tRNA synthetase has been knocked out, or a mutant host in which expression of a chromosomal gene encoding an aminoacyl-tRNA synthetase has been diminished to a degree where the host cell cannot grow, that is employed for transformation by (5) a recombinant vector comprising a site permitting the insertion in an expressible state of a foreign gene other than a gene encoding an aminoacyl-tRNA synthetase and (6) comprising in an expressible state a gene encoding an aminoacyl-tRNA synthetase.

(4) The host cell prior to mutation of the mutant host cell in the present invention is, for example, a bacterium such as *Escherichia coli* or *Bacillussubtilis*; a yeast such as *Saccharomyces cerevisiae, Saccharomyces pombe,* or *Pichia pastoris*; an insect cell such as sf9 or sf21; a COS cell; a Chinese hamster ovarian cell (CHO cell), or some other animal cell; or a plant cell such as tobacco. Bacteria such as *Escherichia coli* and *Bacillus subtilis*, and yeast, are preferred.

Generally, a cell has chromosomal genes encoding aminoacyl-tRNA synthetases for its own proliferation. By contrast, in the present invention, the chromosomal gene encoding an aminoacyl-tRNA synthetase originally present in the cell is knocked out or the expression of a chromosomal gene encoding an aminoacyl-tRNA synthetase is diminished to a degree where growth of the host cell is prevented. A chromosomal gene encoding an aminoacyl-tRNA synthetase can be knocked out, or its expression diminished, by the following methods.

### [Gene knockout (deactivation)]

The term "deactivation" includes methods of impeding the functional expression of one or more chromosomal genes. Deactivation is conducted by knocking out, substituting (by mutation, for example), blocking, inserting into, and/or the like the nucleic acid gene sequence. A number of forms of implementation include the deactivation of one or more genes by causing deactivation in a mutant microorganism, desirably in a stable and irreversible fashion.

### (Deactivation by insertion)

The term "insertion sequence" employed here refers to a DNA sequence that is introduced into the chromosome of a microorganism. In a number of forms of implementation, the insertion sequence is already present in the genome of the cell that is being transformed. A sequence that is not present (that is, a homologous or heterogenous sequence) can also be employed. In other forms of implementation, the insertion sequence may contain a selection marker. In still other forms of implementation, the insertion sequence may contain two homology boxes.

The term "'homology box" employed here refers to a nucleic acid sequence that is homologous to the chromosomal sequence of a microorganism. More specifically, a homology box is an upstream or downstream region having about 80 to 100 percent sequence homology, about 90 to 100 percent sequence homology, or about 95 to 100 percent sequence homology with a coding region or gene adjacent to a gene being deactivated in accordance with the present invention. The purpose of such a sequence is to replace a portion of the chromosome of a microorganism. Although not a limitation of the present invention, a homology box contains about 1 base pair (bp) to 200 kilobases (kb). A homology box desirably contains about 1 bp to 10.0 kb, 1 bp to 5.0 kb, 1 bp to 2.5 kb, 1 bp to 1.0 kb, and 0.25 kb to 2.5 kb. Further, a homology box contains about 10.0 kb, 5.0 kb, 2.5 kb, 2.0 kb, 1.5 kb, 1.0 kb, 0.5 kb, 0.25 kb, and 0.1 kb. In a number of forms of implementation, the 5' and 3' ends of a selection marker are adjacent to homology boxes; here, the homology boxes include the nucleic acid sequences that are immediately adjacent to the regions encoding the particular gene.

The term "selection marker" employed here refers to a nucleotide sequence capable of being expressed in the host cell. The expression of a selection marker in the presence of a corresponding selection factor, or in the absence of a required nutrient, allows a cell containing the gene being expressed to grow. The terms "selectable marker" and "selection marker" used here refer to nucleic acid (such as a gene) that can be expressed in a host cell to facilitate the selection of hosts containing a vector. Although not a limitation, examples of such selection markers are antibiotics. Accordingly, a "selection marker" refers to a gene imparting a sign that the targeted insertion DNA has been incorporated into the host cell, or that some other reaction has occurred. Normally, a selection marker refers to a gene imparting bacterial resistance or a metabolic advantage to a host cell that makes it possible to distinguish cells containing foreign DNA from cells that have not picked up the foreign sequence during transformation. Examples are selection markers that impart resistance to antibiotics. (Examples are *ampR, phleoR, specR, kanR, eryR, tetR, cmpR,* and *neoR*. For example, see Guerot-Fleury, Gene, 167:335-337 [1995]; Palmeros et al., Gene, 247: 255-265 [2000]; and Trieu-Cuot et al., Gene, 23: 331-341 [1983]. These descriptions are specifically incorporated herein by reference.) In a number of preferred forms of implementation, the present invention provides a gene imparting resistance to chloramphenicol (such as the gene present on pC194 and the resistance gene present in the genome of *Bacillus lichenformis*). Such a resistance gene is particularly useful in forms of implementation relating to chromosomal amplification of integrated plasmids, cassettes integrated onto chromosomes, and the present invention.

For example, when the gene being deactivated is the tryptophenyl-tRNA synthetase gene (*trpS*), the DNA structure contains a selection marker as an insertion sequence and comprises the *trpS* gene, which blocks its activity. The selection marker is inserted within the *trpS* code sequence portion. The DNA structure has essentially the same sequence as the *trpS* gene in the host chromosome. In double crossing over, the *trpS* gene is deactivated by insertion of the selection marker.

### (Deactivation by knockout)

In a number of desirable forms of implementation, deactivation is achieved by knockout. In a number of desirable forms of implementation, the gene is knocked out by homologous recombination. For example, in a number of forms of implementation, when the gene being knocked out is *trpS*, a DNA structure containing insertion sequences having selection markers adjacent to the two sides of a homology box is employed. The homology box contains nucleotide sequences that are homologous to the nucleic acid flanking regions of the chromosomal *trpS* gene. The DNA structure matches the homologous sequence of the chromosome of the microorganism host. In double crossing over, the *trpS* gene is cut out of the host chromosome.

The term "knocking out" a gene that is employed here refers to knocking out an entire code sequence, knocking out a portion of a code sequence, or knocking out a code sequence containing a flanking region. Knocking out may be partial so long as the sequence remaining in the chromosome does not exhibit the bioactivity it had prior to being knocked out. The flanking regions of the code sequence can contain about 1 bp to about 500 bp on the 5' and 3' ends. The flanking regions can be larger than 500 bp, but in accordance with the present invention, desirably do not contain other genes that can be deactivated or knocked out. Finally, the gene that has been knocked out is essentially nonfunctional. Simply stated, the term "knocked out" is defined as a change in a nucleotide or amino acid sequence whereby one or more nucleotide or amino acid residue is eliminated (that is, absent).

The term "flanking sequence" employed here refers to a sequence that is upstream or downstream from a target sequence (for example, in genes A-B-C, gene B is flanked (positioned beside) gene sequences A and C). In a desirable form of implementation, the insertion sequence is positioned on both sides of a homology box. In a number of forms of implementation, the flanking sequence is present on only one side (3' or 5'), but in a desirable form of implementation, it is positioned on both sides of the sequence. The sequences of each homology box are homologous to sequences in the chromosome of the microorganism. The purpose of these sequences is to integrate a new structure into the chromosome of the microorganism so that a portion of the chromosome of the microorganism is replaced by the insertion sequence. In a desirable form of implementation, the 5' and 3' ends of the selection marker are positioned beside the polynucleotide sequence containing a portion of the deactivated chromosomal fragment.

### (Deactivation by mutation)

In another form of implementation, deactivation is produced by mutation of the gene. Various methods of gene mutation can be employed; the method is not limited. Examples are site-specific mutation, random mutation generation, and the gapped-duplex method. (For example, see Moring et al., Biotech. 2: 646 [1984], and Kramer et al., Nucleic Acids Res., 12: 9441 [1984]. These descriptions are specifically incorporated herein by reference).

In a desirable form of implementation, a mutation is produced by inducing mutation in a codon on the chromosome. In another form of implementation, the mutant DNA sequence has 40 percent or greater, 45 percent or greater, 50 percent or greater, 55 percent or greater, 60 percent or greater, 65 percent or greater, 70 percent or greater, 75 percent or greater, 80 percent or greater, 85 percent or greater, 90 percent or greater, 95 percent of greater, or 98 percent or greater, homology with the sequence of the wild form. In another form of implementation, the mutant DNA is produced in vivo by a known mutation-inducing procedure such as UV irradiation or a chemical mutagenic agent such as nitrosoguanidine.

In terms of chromosomal mutations that knock out aminoacyl-tRNA synthetase activity, the knocking out of an aminoacyl-tRNA synthetase gene itself is desirable. However, other genes that regulate the synthesis of aminoacyl-tRNA synthetases can be employed so long as an extrachromosomal gene retaining the corresponding gene is employed. In the case of a strain in which an aminoacyl-tRNA synthetase gene has been knocked out, the gene that must be inserted into the extrachromosomal gene corresponds to the particular aminoacyl-tRNA synthetase gene.

To avoid the possibility of the homologous recombination of an extrachromosomal gene having an aminoacyl-tRNA synthetase with a mutated aminoacyl-tRNA synthetase gene of the chromosome, the targeted aminoacyl-tRNA synthetase gene is desirably essentially deleted from the chromosome.

The recombinant vector of (5) and (6) is identical to the recombinant vector of (1) and (2) above.

### [Functions]

Since the mutant host cell in which a chromosomal gene encoding an aminoacyl-tRNA synthetase has been knocked out, or the mutant host in which the expression of a chromosomal gene encoding aminoacyl-tRNA synthetase has been diminished to a degree where the host cell cannot grow, cannot express the chromosomal gene encoding the aminoacyl-tRNA synthetase, it cannot proliferate as is.

By contrast, by incorporating a recombinant vector having a gene encoding the aminoacyl-tRNA synthetase in a state permitting expression, proliferation can be maintained. That is, knocking out the aminoacyl-tRNA synthetase can be complemented by incorporating the particular aminoacyl-tRNA synthetase onto the chromosomal gene. However, if the cell loses the particular extrachromosomal gene, the cell also becomes unable to proliferate.

Accordingly, in the mutant host cell of the present invention, given the above relation, a recombinant vector into which has been inserted a foreign gene can be stably retained in the host cell without incorporating an antibiotic into the medium and without eliminating or limiting nutrients satisfying specific nutritional requirements. Thus, the mutant host cell of the present invention affords the advantage of permitting application to any medium employed in production on an industrial scale.

Further, the mutant host cell of the present invention can be employed to express a foreign gene other than a gene encoding an aminoacyl-tRNA synthetase. The host cell of the present invention contains a recombinant vector comprising in an expressible state a gene encoding the above-described aminoacyl-tRNA synthetase. Further, this recombinant vector comprises a site permitting the insertion in an expressible state of a foreign gene. By inserting a desired foreign gene at this site, the recombinant vector into which the desired foreign gene has been inserted can be stably maintained in the host cell. Expression of the foreign gene can be induced to produce a desired protein encoded by the foreign gene.

### [The method for preparing a protein or peptide]

The present invention relates to a method for preparing a protein or peptide encoded by a foreign gene by expressing a foreign gene other than a gene encoding an aminoacyl-tRNA synthetase. This method comprises the following steps:
(10) preparing a recombinant vector, comprising a site permitting the insertion in an expressible state of a foreign gene and comprising in an expressible state a gene encoding an aminoacyl-tRNA synthetase, in which a desired foreign gene has been inserted at the site permitting the insertion in an expressible state of a foreign gene;
(11) preparing a mutant host cell in which a chromosomal gene encoding an aminoacyl-tRNA synthetase has been knocked out or a mutant host cell in which the expression of a chromosomal gene encoding an aminoacyl-tRNA synthetase has been diminished to a degree where the host cell cannot grow;
(12) transforming the mutant host cell with the recombinant vector to obtain a transformant; and
(13) culturing the transformant to prepare the protein or peptide encoded by the foreign gene.

(10) The step of preparing a recombinant vector
In this step, a recombinant vector is prepared that comprises in an expressible state a gene encoding an aminoacyl-tRNA synthetase and that comprises a site permitting the insertion in an expressible state of a foreign gene, in which site a desired foreign gene has been inserted. The recombinant vector is the recombinant vector of the invention of the present application set forth above. In the preparing method of the present invention, a recombinant vector is prepared with a desired foreign gene inserted at the site of the recombinant vector permitting the insertion in an expressible state of a foreign gene. Specifically, a gene encoding an aminoacyl-tRNA synthetase and a desired foreign gene are inserted into a suitable vector. The genes can be inserted by the usual methods. Both genes are inserted in expressible states.

### [A general description of the insertion of the two genes]

The aminoacyl-tRNA synthetase gene and the foreign gene can be inserted into the recombinant vector by common methods of DNA recombination. For example, they can be inserted by the method described in Molecular Cloning (1989) (Cold Spring Harbor Lab.). The entire description of this document is specifically incorporated herein by reference. The individual genes can be inserted into the recombinant vector at essentially any position so long as they do not contribute to replication of the recombinant vector; the insertion positions will vary with the vector employed. Examples of recombinant vectors that can be employed are plasmids derived from *Escherichia coli* (such as pBR322, pBR325, pUC18, pUC19, pUC119, pTV118N, pTV119N, pBluescript, pHSG298, pHSG396, pTrc99A, and other ColE plasmids; pACYC177, pACYC184, and other p1A plasmids; and pMW118, pMW119, pMW218, pMW219, and other pSC101 plasmids); plasmids derived from *Bacillus subtilis* (such as pUB110 and pTP5); and pHY300PLK and other *Escherichia coli-Bacillus subtilis* shuttle vectors.

In the example of the pHY300PLK plasmid, which is an *Escherichia coli-Bacillus subtilis* shuttle vector, an aminoacyl-tRNA synthetase gene can be inserted at the *Eco*RI site, which is a multicloning site, and a foreign gene can be inserted at the *Bam*HI site, which is also a multicloning site. The present example will be described in some detail below.

Using PCR amplification with the chromosomal DNA of an organism having a foreign gene as template, the foreign gene is amplified from its promoter site to its stop codon. A ligation reaction is then conducted with this gene and a DNA fragment obtained by digesting pHY300PLK with *Bam*HI restriction enzyme. The ligation reaction mixture obtained is then used to transform *Escherichia coli,* transformants containing the plasmid in which a foreign gene has been inserted as targeted from the promoter site to the stop codon at the *Bam*HI site of pHY300PLK are selected, and a plasmid is prepared from a transformant. Next, the plasmid thus prepared is digested with *Eco*RI restriction enzyme and cleaved. PCR amplification with chromosomal DNA of *Bacillus subtilis* strain 168 as template is used to amplify an aminoacyl-tRNA synthetase gene from its promoter site to its stop codon, which is subjected to a ligation reaction with the plasmid-derived DNA fragment with the structure that has been cleaved with *Eco*RI. The ligation reaction mixture obtained is used to transform *Escherichia coli*, transformants containing the plasmid in which an aminoacyl-tRNA synthetase gene has been inserted as targeted at the *Eco*RI site of the constructed plasmid are selected, and the targeted plasmid can be prepared from the transformants.

(11) The step of preparing a mutant host cell
In this step, a mutant host cell in which the chromosomal gene encoding an aminoacyl-tRNA synthetase has been knocked out, or a mutant host cell in which the expression of a chromosomal gene encoding an aminoacyl-tRNA synthetase has been diminished to a degree where the host cell cannot develop, is prepared. The mutant host cell is the mutant host cell of the present invention as set forth above. The method of preparation is as set forth below.

Examples of the procedure for preparing the mutant host cell include methods of eliminating or deactivating in a planned manner the target gene present on the host chromosome and imparting random gene elimination or deactivation mutations and then using suitable methods to analyze the genes or evaluate protein productivity.

It suffices to employ homologous recombination, for example, to eliminate or deactivate a target gene. That is, the host cell is caused to incorporate a circular recombinant plasmid or linear DNA fragment obtained by cloning a DNA fragment containing part of a targeted gene into a suitable plasmid vector. Homologous recombination in a partial region of the target gene cleaves the target gene on the parent microorganism genome, thereby deactivating it. Alternatively, a target gene that has been imparted with a deactivating mutation by base substitution, base insertion, or the like, or a linear DNA fragment or the like that contains the region outside a target gene but not the target region, is construction by a method such as PCR, incorporated into a parent microorganism cell, and subjected to double crossing over homologous recombination at two spots outside the location of the mutation in the target gene in the parent microorganism genome, or in two spots outside the target gene, to substitute the eliminated or deactivated gene fragment for the target gene on the genome.

(12) The step of obtaining a transformant
The mutant host cells prepared in step (11) are transformed with the recombinant vector prepared in step (30). The transformation of the host cells with the recombinant vector can also be implemented by the usual methods.

The method of incorporating (transforming) the recombinant vector into the host cells is not specifically limited. The transformation method, transfection method, competent cell method, electroporation, or the like can be suitably selected based on the type of host cell into which the recombinant vector is being introduced and on the form of the recombinant vector.

The term "host cell" employed here refers to a cell having the ability to function as a host or an expression medium for a newly introduced DNA sequence.

The form in which the recombinant vector is present within the host cell is not specifically limited. It can be inserted into the chromosome, incorporated by substitution, or present in the form of a plasmid.

The chromosomal gene encoding the aminoacyl-tRNA synthetase contained in the recombinant vector is the same gene as the chromosomal gene in the mutant host cell encoding the aminoacyl-tRNA synthetase that has been knocked out or the chromosomal gene in the mutant host cell encoding the aminoacyl-tRNA synthetase the expression of which has been diminished to a degree where the host cell is incapable of developing. For example, when the chromosomal gene encoding the aminoacyl-tRNA synthetase that has been knocked out or the chromosomal gene encoding the aminoacyl-tRNA synthetase the expression of which has been diminished to a degree where the cost cell is incapable of developing is the alanyl-tRNA synthetase gene in the mutant host cell, the gene contained in the recombinant vector is also the alanyl-tRNA synthetase gene.

(13) The step of preparing a protein or peptide
The transformant obtained in step (12) is cultured to prepare a protein or peptide encoding the foreign gene. The transformant is cultured using a suitable medium and under suitable conditions based on the type of host cell. The host cell need only be capable of producing the protein or peptide encoded by the foreign gene, and may be of the wild type or a mutated form. Specific examples are bacteria of the genus *Bacillus,* such as *Bacillus subtilis*; bacteria of the genus *Escherichia*; bacteria of the genus *Clostridium*; and yeast. Of these, bacteria of the genus *Bacillus* are desirable. *Bacillus subtilis* is preferred because its entire genome is known, genetic engineering and genomal engineering techniques have been established, and it can be made to produce protein and secrete it externally.

In the present specification, the term "genus *Bacillus*" covers all the generally known strains contained in the genus *Bacillus* without limitation. For example, it means: *Bacillus subtilis, Bacillus licheniformis, Bacillus lentus, Bacillus stearothermophilus, Bacillus alkalophilus, Bacillus amyloliquefaciens, Bacillus cereus, Bacillus pumilus, Bacillus clausii, Bacillus halodurans, Bacillus megaterium, Bacillus coagulans, Bacillus circulans,* and *Bacillus thuringiensis.* Classification of the genus Bacillus is continuing, and strains that are classified in this genus are also included. For example, the genera *Geobacillus, Alkalibacillus, Amphibacillus, Amylobacillus, Anoxybacillus, Goribacillus, Cerasibacillus, Gracilibacillus, Halolactibacillus, Halalkalibacillus, Filobacillus, Jeotgalibacillus, Salibacillus, Oceanobacillus, Marinibacillus, Lysinibacillus, Lentibacillus, Ureibacillus, Salinibacillus, Pontibacillus, Piscibacillus, Paraliobacillus, Virgibacillus, Salsuginibacillus, Tenuibacillus, Thalassobacillus, Thermalkalibacillus,* and *Tumebacillus* exist. These are also included in the genus *Bacillus* in the present specification.

For example, when the host cell is *Bacillus subtilis*, it suffices for the culture medium employed to contain a suitable nitrogen source, carbon source, and minerals for growth of the *Bacillus subtilis* host of the present invention and production of the targeted protein. For example, when recombinant *Bacillus subtilis* strain 168 is used to produce a protein or peptide encoded by a foreign gene, a medium compounded with a carbon source in the form of a monosaccharide such as glucose or fructose, a disaccharide such as sucrose or maltose, or a polysaccharide such as soluble starch, and a medium compounded with a nitrogen source in the form of peptones, soybean extract, yeast extract, fish extract, corn steep liquor (CSL), or metal salts can be employed.

The pH of the medium need only be a pH falling within a range permitting growth of the recombinant organism employed. For example, in the case of *Bacillus subtilis*, the pH is suitably adjusted to 6.0 to 8.0, and the growth conditions are stirred culturing with ventilation or shaking for 2 to 7 days at 15 to 42°C, desirably 28 to 37°C.

The targeted protein or peptide is recovered after the above culturing. The targeted protein or polypeptide can be recovered by the usual methods. The targeted protein or polypeptide that is recovered can be purified by the usual methods as needed.

Examples of the targeted protein or peptide produced by the manufacturing method of the present invention are enzymes that are useful in foods, pharmaceuticals, cosmetics, detergents, textile processing, medical test reagents, and the like, as well as polypeptides and proteins such as physiologically active factors.

The targeted protein or peptide gene is not specifically limited, and includes enzymes employed in various industries, such as detergents, foods, textiles, feeds, chemicals, medical treatment, and diagnosis; as well as physiologically active peptides. Based on function, industrial enzymes include oxidoreductases, transferases, hydrolases, phosphorylases, lyases, isomerases, ligases/synthetases, and modifying enzymes. More specific examples are the genes of sugar-degrading enzymes such as cellulase, agarase, and *gamma-*cyclodextrin synthetase.

### [Method of causing a recombinant vector to be stably retained within a host cell]

The present invention relates to a method of causing a targeted recombinant vector to be stably retained in a host cell. This method comprises (20) to (22) below:
(20) knocking out, or diminishing to a degree where the host cell cannot grow, a chromosomal gene encoding an aminoacyl-tRNA synthetase contained in the host cell, to convert the host cell to a mutant host cell;
(21) incorporating in an expressible state a gene encoding an aminoacyl-tRNA synthetase into the targeted recombinant vector; and
(22) transforming the mutant host cell with the targeted recombinant vector containing the gene encoding an aminoacyl-tRNA synthetase in an expressible state.

(20) The method of converting a host cell to a mutant host cell is as stated in the description of mutant host cells above.

(21) The incorporation in an expressible state of a gene encoding an aminoacyl-tRNA synthetase into the targeted recombinant vector is also as stated in the description of the recombinant vector of the present invention. The fact that the recombinant vector has a site permitting the insertion in an expressible state of a foreign gene other than a gene encoding an aminoacyl-tRNA synthetase is also as set forth above.

(22) The transformation of the mutant host cell is also as stated in the description of the above-described method for preparing a protein or peptide.

The present invention includes a method of causing a targeted recombinant vector to be stably retained in a host cell. In this method, the mutant host cell of the present invention is employed as the host cell. The method of the present invention is one of stabilizing a extrachromosomal gene contained in a cell such as that set forth above. For example, the chromosomal mutation rendering an aminoacyl-tRNA synthetase deficient is an at least partial knockout of a particular aminoacyl-tRNA synthetase gene and the extrachromosomal gene is the particular aminoacyl-tRNA synthetase gene.

Based on the present invention, for example, a transformed cell strain in which an extrachromosomal gene is stable in non-limited medium can be obtained without requiring selection by an antibiotic or a medium with a limited content of a specific amino acid. Further, based on the method described in the present invention, cells that have lost the extrachromosomal gene cannot proliferate. Accordingly, the present invention relates to cells that have been modified by an aminoacyl-tRNA synthetase gene and an extrachromosomal gene that contains a factor that renders reliable the expression of an industrially useful protein. Further, the present invention relates to a method of manufacturing an industrially useful protein employing a transformed cell, **characterized in that** the cell that has been transformed by an extrachromosomal gene of the present invention is cultured in non-limited medium. The extrachromosomal gene retains the gene for the particular protein.

The term "protein gene" employed here means a chromosomal fragment of DNA relating to the production of a peptide chain that may or may not comprise a region preceding or succeeding the region encoding the protein.

The embodiments set forth below compare the stability of a plasmid comprising the tryptophanyl-tRNA synthetase gene in *Bacillus subtilis* having a deficiency in tryptophanyl-tRNA synthetase, which is one of aminoacyl-tRNA synthetases, to the stability of the same plasmid in *Bacillus sublilis* that does not have a deficiency in tryptophanyl-tRNA synthetase. They also compare the stability of a plasmid comprising the tryptophanyl-tRNA synthetase gene in *Bacillus subtilis* having a deficiency in tryptophanyl-tRNA synthetase, which is one of aminoacyl-tRNA synthetases, to the stability of the same plasmid not comprising the tryptophanyl-tRNA synthetase gene in *Bacillus subtilis* not having a deficiency in tryptophanyl-tRNA synthetase.

Examples of the use of recombinant vectors comprising the tryptophanyl-tRNA synthetase gene to express genes encoding cellulase and agarase transported in the same vector are also given.

### [Embodiments]

The present invention is described in greater detail below through embodiments.

### [Embodiment 1] The introduction of an extrachromosomal gene including the tryptophanyl-tRNA synthetase gene into Bacillus subtilis

The plasmid pDATS14, which is an extrachromosomal gene containing the tryptophanyl-tRNA synthetase gene, was constructed by the following method. First, primers A and B were used with a commonly employed *Bacillus subtilis-Escherichia coli* shuttle plasmid in the form of pHY300PLK (made by Yakult) as template to conduct PCR amplification. The amplified fragment obtained was digested with *Xho*I restriction enzyme and the two ends were joined to form a circular plasmid. *Escherichia coli* strain HB101 was transformed with this plasmid. Plasmid was prepared from the transformant and named pDA2. Additionally, primers C and D were used with the chromosome of *Bacillus subtilis* strain 1SW1214 as template to obtain a DNA fragment comprising the tryptophanyl-tRNA synthetase gene and nearby regions. This DNA fragment was digested with *Xho*I restriction enzyme and spliced to the pDA2 that had been digested with *Xho*I restriction enzyme. This was then used to transform *Escherichia coli* strain HB101. Plasmid was prepared from the transformant obtained and named pDATS14. Using pDATS14, a mutant strain derived from *Bacillus subtilis* strain ISW1214 having deficiencies in two proteases (alkali protease E and neutral protease E) (see Reference Appl. Microbiol. Biotechnol 65: 583-592 (2004) Hatada, Y. et al., this description being incorporated herein in its entirety by reference) was transformed by the protoplast transformation method (see Reference Mol. Gen. Genet. 168: 111-115 (1979) Chang, S. and Cohen, S.N., this description being incorporated herein in its entirety by reference) to obtain a *Bacillus subtilis* transformant having an extrachromosomal tryptophanyl-tRNA synthetase gene.

### [Embodiment 2] The construction of a DNA fragment to impair the tryptophanyl-tRNA synthetase gene on the chromosome of Bacillus subtilis

An about 2.5 kb DNA fragment containing the tryptophanyl-tRNA synthetase gene and nearby regions was obtained using primers E and F with the chromosome of *Bacillus subtilis* strain ISW1214 as template. This was digested with the restriction enzymes *Eco*RI and *Sal*I. It was then spliced with general purpose plasmid pUC18 that had been predigested with *Eco*RI and *Sal*I to obtain the plasmid pTSAF. Using primers G and H with a general-purpose plasmid in the form of pC194 as template, an about 1.6 kb DNA fragment containing a Staphylococcus-derived chloramphenicol resistance gene and nearby areas was obtained. Using primers I and J with pTSAF as template, a PCR reaction was conducted to obtain a DNA fragment. This DNA fragment and the 1.6 kb DNA fragment containing the chloramphenicol resistance gene and nearby regions were digested with restriction enzymes *Bam*HI and *Xba*I, spliced, and used to transform *Escherichia coli* strain HB101. Plasmid was prepared from the transformant and named tryptophanyl-tRNA synthetase gene-impairing plasmid pINTTS. Using primers K and L with pINTTS as template, a PCR reaction was conducted to obtain a linear tryptophanyl-tRNA synthetase gene-impairing DNA fragment.

### [Embodiment 3] Impairing the tryptophanyl-tRNA synthetase gene on the chromosome of Bacillus subtilis

Using the above-described tryptophanyl-tRNA synthetase gene-impairing DNA fragment, the competent cell method was employed to further transform the above-described transformant containing the extrachromosomal tryptophanyl-tRNA synthetase gene, yielding a transformant with chloramphenicol resistance. Chromosomal DNA was prepared from the transformant. The chromosomal DNA was subjected to a PCR reaction using primers M and N or O and P. The fact that the region encoding the tryptophanyl-tRNA synthetase gene on the chromosome had been replaced with the chloramphenicol resistance gene derived from the DNA fragment for impairing the tryptophanyl-tRNA synthetase gene was confirmed. This bacterial strain, a tryptophanyl-tRNA synthetase gene knockout strain, was named strain DTS1451(pDATS14).

### [Embodiment 4] Introducing plasmid having a kanamycin resistance gene into strain DTS1451

Using primers C and D with the chromosome of *Bacillus subtilis* strain ISW1214 as template, a DNA fragment containing the tryptophanyl-tRNA synthetase gene and nearby regions was obtained. This DNA fragment was digested with *Xho*I restriction enzyme and spliced with pDA2 that had been predigested with the restriction enzyme *Xho*I. This was then used to transform *Escherichia coli* strain HB101. Plasmid was prepared from the transformant obtained and named pDATS13. Using primers Q and R with general purpose plasmid pUB110 as template, a DNA fragment containing the kanamycin resistance gene and nearby regions was obtained. This DNA fragment was digested with *Eco*RI, amplified by PCR using primers S and T with pDATS14 as template, and then spliced with the DNA fragment obtained by digestion with *Eco*RI. It was then used to transform *Escherichia coli* strain HB101. Plasmid was prepared from the transformant having kanamycin resistance obtained. The plasmid was named pDATSK. Using pDATSK, strain DTS1451 containing pDATS14 was transformed by the protoplast transformation method and selection was conducted in regenerated medium containing 30 microgram/mL of kanamycin (composition: 8 percent sodium succinate, 1 percent agar, 0.5 percent casamino acids, 0.5 percent yeast extract, 0.15 percent potassium dihydrogenphosphate, 0.35 percent dipotassium hydrogenphosphate, 0.5 percent glucose, 0.4 percent magnesium sulfate, 0.01 percent bovine serum albumin, 0.001 percent methionine, and 0.001 percent leucine). The transformants obtained were inoculated into kanamycin-containing LB agar medium and regenerated medium containing 7.5 micrograms/mL of tetracycline. A strain exhibiting resistance to kanamycin and sensitivity to tetracycline was named DTS1451(pDATSK).

### [Embodiment 5] Producing cellulase in DTS1451

A PCR reaction was conducted using primers U and V with the chromosome of *Bacillus akibai* strain 1139 (JCM 9157T), a cellulase-producing bacterium (see J. Gen. Microbiol. 1986, 132, 2329-2335, Fukumori et al.; Int J Syst Evol Microbiol. (2005) 55: 2309-15. Nogi, Y. et al.; these descriptions are hereby incorporated in their entirety by reference) as template to obtain a DNA fragment containing cellulase and nearby regions. This DNA fragment and pDATS13 were digested with the restriction enzyme *Bam*HI and then spliced to obtain plasmid pDATSC1. This plasmid was employed to transform DTS1451(pDATSK) and selection was conducted with a regenerated medium containing tetracycline. The transformants obtained were inoculated onto kanamycin-containing LB agar medium and regenerated medium containing 7.5 microgram/mL of tetracycline. A strain that exhibited cellulase activity, was sensitive to kanamycin, and was resistant to tetracycline was named DTS1451(pDATSC1). DTS1451(pDATSC1) was cultured with stirring at 130 rpm for 72 hours at 30°C in PPS medium (3 percent polypeptone S, 0.5 percent fish extract, 0.05 percent yeast extract, 0.1 percent potassium dihydrogenphosphate, 4 percent maltose, 0.02 percent magnesium sulfate, and 0.05 percent calcium chloride) (to which no antibiotic was added). The cellulase activity of the supernatant of the culture obtained was measured. As a result, substantial production of cellulase of about one gram per liter of culture solution was confirmed.

### [Embodiment 6] The retention of plasmid pDATSC1 in strain DTS1451

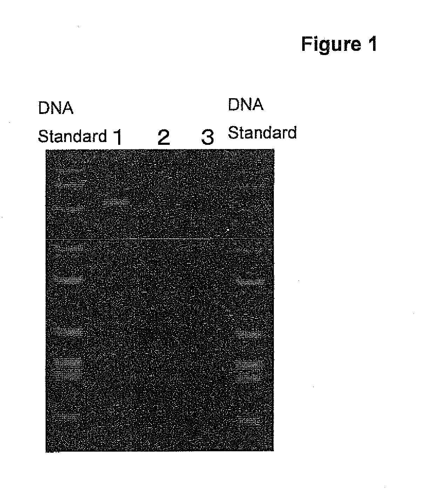

Strain DTS1451 containing pDATSC1 was inoculated into LB media to which 7.5 micrograms/mL of tetracycline had been added or into which no tetracycline had been added (1 percent polypeptone, 0.5 percent yeast extract, and 1 percent sodium chloride) and cultured with stirring at 130 rpm for 24 hours at 30°C. A 10 microliter quantity of the culture solution was collected and transplanted to another 100 mL of LB medium. Culturing was conducted for another 24 hours. A 10 microliter quantity of the culture solution was collected, transplanted to another 100 mL of LB medium, and similarly cultured for 24 hours. Subsequently, operations were conducted to recover the plasmid from the cultured cells. As a result, in the DTS1451 strain containing pDATSC1, regardless of whether tetracycline was added or not, no change was observed in the content of plasmid per the culture solution. In the mutant strain of *Bacillus subtilis* strain ISW1214 having pDATSC1 and not deficient in aminoacyl-tRNA synthetase genes, when tetracycline was added, the content of plasmid per the culture solution was maintained, but when tetracycline was not added, the content of plasmid was observed to drop sharply. Further, plasmid pDAC1 was prepared in which a cellulase gene having pDATSC1 was inserted into the *Bam*HI recognition site of pDA2 and the same tests were conducted. This revealed that in the mutant strain of *Bacillus subtilis* strain ISW1214 having pDAC1, when tetracycline was added, the plasmid content per the culture solution was maintained, but when it was not added, the plasmid content was observed to drop sharply. To facilitate comprehension of these results, Fig. 1 shows the results of electrophoresis of solutions of the plasmids recovered from the various cells cultured in a medium to which no antibiotic was added. Following the electrophoresis operation, the agarose gel was immersed in ethidium bromide to dye the DNA. In Fig. 1, lane 1 is the transformation combination of a host cell deficient in the particular aminoacyl-tRNA synthetase gene on the chromosome but containing the aminoacyl-tRNA synthetase gene in a plasmid; lane 2 is the transformation combination of a host cell non deficient in the particular aminoacyl-tRNA synthetase gene on the chromosome and having the aminoacyl-tRNA synthetase gene in a plasmid; and lane 3 is the transformation combination of a host cell not deficient in the particular aminoacyl-tRNA synthetase gene on the chromosome but not containing the aminoacyl-tRNA synthetase gene in a plasmid.

### [Embodiment 7] Production of agarase in strain DTS1451

A PCR reaction was conducted using primers W and X with the chromosome of *Microbulbifer* sp. A94, which is an agarase-producing bacterium (see Biosci Biotechnol Biochem. (2004) 68: 1073-81. Ohta, Y. et al., the entire description of which is hereby incorporated in its entirety) as template. A DNA fragment encoding agarase was obtained. Additionally, a PCR reaction was conducted using primers Y and Z with above-described pDATSC1 as template. The 5' end of the DNA fragment obtained was phosphorylated, after which the fragment was spliced to DNA encoding agarase. This was then used to transform *Escherichia coli* HB101. A plasmid prepared from a transformant obtained that had agarase activity was named pDATSA1. This plasmid was used to transform DTS1451(pDATSK). Selection was conducted with regenerated medium containing 7.5 microgram/mL of tetracycline. The transformant obtained was inoculated into LB agar medium containing kanamycin and regenerated medium containing 7.5 micrograms/mL of tetracycline. A strain exhibiting agarase activity, sensitivity to kanamycin, and resistance to tetracycline was named DTS1145 (pDATSA1). DTS1451(pDATSA1) was cultured with stirring at 130 rpm for 72 hours at 30°C in PPS medium (to which no antibiotic was added) and the agarase activity of the supernatant of the culture obtained was measured. As a result, the production of about 0.1 g of agarase per liter of culture solution was confirmed. Next, a PCR reaction was conducted using primer A1 (SEQ ID NO: 79) and primer B1 (SEQ ID NO: 80) with pDATSA1 as template. The DNA fragment obtained was digested with *Xho*I and closed into a circle by a ligation reaction. This operation yielded a plasmid from which the tryptophanyl-tRNA synthetase gene had been eliminated. This plasmid was cleaved with the restriction enzyme *Eco*RI. Additionally, DNA fragments containing the tryptophanyl-tRNA synthetase gene and nearby regions were amplified by PCR in combinations of primers C1 (SEQ ID NO: 81) and D1 (SEQ ID NO: 82), primers E1 (SEQ ID NO: 83) and F1 (SEQ ID NO: 84), or primers G1 (SEQ ID NO: 85) and H1 (SEQ ID NO: 86), with pDATSC1 as template. The various PCR-amplified DNA fragments were cleaved with the reduction enzyme *Eco*RI and spliced with the above plasmid that had been digested in advance with the restriction enzyme *Eco*RI. These were then used to transform *Escherichia coli* HB101. Plasmids prepared from those transformants exhibiting agarase activity were named pDATSA2, pDATSA3, and pDATSA4. The base sequences of pDATSA2, pDATSA3, and pDATSA4 were determined, revealing that the tryptophanyl-tRNA synthetase gene and the surrounding portions had the sequences indicated in SEQ ID NOS: 87, 88, and 89. Next, these plasmids were used to transform DTS1451(pDATSK) and selection was conducted with regenerated medium containing 7.5 micrograms/mL of tetracycline. The transformants were inoculated into LB agar medium containing kanamycin and regenerated medium containing 7.5 microgram/mL of tetracycline. Those strains exhibiting agarase activity, sensitivity to kanamycin, and resistance to tetracycline were selected. The DTS1451(pDATSA2), DTS1451(pDATSA3), and DTS1451(pDATSA4) obtained were cultured with stirring at 130 rpm for 72 hours at 30°C in PPS medium (to which no antibiotic was added) and the agarase activity of the supernatant of the cultures obtained was measured. The results confirmed an agarase production of about 0.1 g per liter of culture solution for each of the three.

**[Table 1]**

| Primer | Nulceic acid sequence | SEQ ID NO |
|---|---|---|
| A | GTGCCTCGAGGATTAAGCATTGGTAACTGTC | 53 |
| B | TATTCTCGAGACATTAACCTAGAAAGCACTAAGG | 54 |
| C | CTGCCTGTCTCGAGGCTGATAGCAGTTATC | 55 |
| D | CAACTCGAGGCTGGTCGGACAAACACCTAG | 56 |
| E | TGCGAATTCCGGATGTAAGGAGAACGGCTC | 57 |
| F | CTTGTCGACGTACATCAGAAATCGTACATTCG | 58 |
| G | GGTGGATCCCCTCGGCGGCAATAGTTACCC | 59 |
| H | AATCTAGACTTTCGTTATACAAATTTTAACC | 60 |
| I | TGTAGGATCCAATATGGGCAGAAAACACATG | 61 |
| J | ATATCTAGATTCCACTCTGTGTACCAGTAGC | 62 |
| K | GTTAGCTCCTTCGGTCCTCC | 63 |
| L | GTAACTGGCTTCAGCAGAGC | 64 |
| M | CTGCCACATCGTCTAGGCTGC | 65 |
| N | CAGGAGTCCAAATACCAGAG | 66 |
| O | CCCTTGCCTATACTGTGGAC | 67 |
| P | GTTACAATAGCGACGGAGAG | 68 |
| Q | GTCTGAATTCGAGOAAGGTTTACACCG | 69 |
| R | CTGCGAATTCATCTTCATGGTGAACC | 70 |
| S | CATTGTCATTAGTTGGCTGG | 71 |
| T | GCAAGAATTCAAAATCCATCTTCATCGG | 72 |
| U | GTGACTGAGGATCCGCTAGTTCCAGATCG | 73 |
| V | CTCTTGGATCCCTTCATCATTCTATCACAC | 74 |
| W | GCAGATTGGGATGGAGTTCCCGTAC | 75 |
| X | TTACAGCTTCACAAAGCGGATTTC | 76 |
| Y | TGCTGCAAGAGCTGTCGGAAATAAAG | 77 |
| Z | AAGAAGAAAAGAAAGAAGCTAAAG | 78 |

### [Industrial Applicability]

The present invention is useful in the field of producing various proteins and peptides by culturing transformants obtained by transforming host cells such as bacteria.

### [Brief Description of the Drawings]

[Fig. 1] Fig. 1 shows the results of electrophoresis of a plasmid recovery solution.

## Claims

1. A recombinant vector, comprising a site permitting insertion in an expressible state of a foreign gene other than a gene encoding an aminoacyl-tRNA synthetase and comprising a gene encoding aminoacyl-tRNA synthetase in an expressible state, for use in a mutant host in which a chromosomal gene encoding an aminoacyl-tRNA synthetase has been knocked out, or in a mutant host in which expression of a chromosomal gene encoding an aminoacyl-tRNA synthetase has been diminished to a degree to which the host cell cannot grow.

2. The recombinant vector according to claim 1, wherein said aminoacyl-tRNA synthetase is tryptophanyl-tRNA synthetase, alanyl-tRNA synthetase, arginyl-tRNA synthetase, asparginyl-tRNA synthetase, aspartyl-tRNA synthetase, cysteinyl-tRNA synthetase, glutamine-tRNA synthetase, glutamate-tRNA synthetase, glycine-tRNA synthetase, histidyl-tRNA synthetase, isoleucyl-tRNA synthetase, leucyl-tRNA synthetase, lysine-tRNA synthetase, methionyl-tRNA synthetase, phenylalanine-tRNA synthetase, prolyl-tRNA synthetase, seryl-tRNA synthetase, threonyl-tRNA synthetase, tyrosyl-tRNA synthetase, or valyl-tRNA synthetase.

3. The recombinant vector according to claim 1 or 2, wherein said recombinant vector is in the form of a plasmid, bacteriophage, or retrotransposon.

4. A mutant host cell in which a chromosomal gene encoding an aminoacyl-tRNA synthetase has been knocked out, or a mutant host in which the expression of a chromosomal gene encoding an aminoacyl-tRNA synthetase has been diminished to a degree where the host cell cannot grow, that is used for transformation by a recombinant vector comprising a site permitting insertion in an expressible state of a foreign gene other than a gene encoding an aminoacyl-tRNA synthetase and comprising in an expressible state a gene encoding an aminoacyl-tRNA synthetase.

5. The mutant host cell according to claim 4, wherein said aminoacyl-tRNA synthetase is tryptophanyl-tRNA synthetase, alanyl-tRNA synthetase, arginyl-tRNA synthetase, asparginyl-tRNA synthetase, aspartyl-tRNA synthetase, cysteinyl-tRNA synthetase, glutamine-tRNA synthetase, glutamate-tRNA synthetase, glycine-tRNA synthetase, histidyl-tRNA synthetase, isoleucyl-tRNA synthetase, leucyl-tRNA synthetase, lysine-tRNA synthetase, rnethionyC-tRNA synthetase, phenylalanine-tRNA synthetase, prolyl-tRNA synthetase, seryl-tRNA synthetase, threonyl-tRNA synthetase, tyrosyl-tRNA synthetase, or valyl-tRNA synthetase.

6. The mutant host cell according to claim 4 or 5, wherein the host cell into which a mutation is introduced is a bacterium, yeast, animal cell, or plant cell.

7. The mutant host cell according to claim 4 or 5, wherein the host cell into which a mutation is introduced is a bacterium or yeast.

8. The mutant host cell according to claim 6 or 7, wherein the bacterium is a bacterium of the genus Bacillus.

9. A method of preparing a protein or peptide encoded by a foreign gene by expressing a foreign gene other than a gene encoding an aminoacyl-tRNA synthetase, comprising:
a step of preparing a recombinant vector, comprising a site permitting insertion in an expressible state of a foreign gene and comprising in an expressible state a gene encoding an aminoacyl-tRNA synthetase, in which a desired foreign gene has been inserted at the site permitting insertion in an expressible state of a foreign gene;
a step of preparing a mutant host cell in which a chromosomal gene encoding an aminoacyl-tRNA synthetase has been knocked out or a mutant host cell in which the expression of a chromosomal gene encoding an aminoacyl-tRNA synthetase has been diminished to a degree where the host cell cannot grow;
a step of transforming said mutant host cell with said recombinant vector to obtain a transformant; and
a step of culturing said transformant to prepare the protein or peptide encoded by the foreign gene.

10. A method of causing a targeted recombinant vector to be stably retained within a host cell, comprising:
knocking out, or diminishing to a degree where the host cell cannot grow, a chromosomal gene encoding an aminoacyl-tRNA synthetase contained in the host cell, to convert the host cell to a mutant host cell;
incorporating in an expressible state a gene encoding an aminoacyl-tRNA synthetase into the targeted recombinant vector; and
transforming the mutant host cell with said targeted recombinant vector containing the gene encoding an aminoacyl-tRNA synthetase in an expressible state.

11. The method according to claim 10, wherein the recombinant vector comprises a site permitting insertion in an expressible state of a foreign gene other than a gene encoding an aminoacyl-tRNA synthetase.

12. The method according to any one of claims 9 to 11, wherein said aminoacyl-tRNA synthetase is tryptophanyl-tRNA synthetase, alanyl-tRNA synthetase, arginyl-tRNA synthetase, asparginyl-tRNA synthetase, aspartyl-tRNA synthetase, cysteinyl-tRNA synthetase, glutamine-tRNA synthetase, glutamate-tRNA synthetase, glycine-tRNA synthetase, histidyl-tRNA synthetase, isoleucyl-tRNA synthetase, leucyl-tRNA synthetase, lysine-tRNA synthetase, methionyl-tRNA synthetase, phenylalanine-tRNA synthetase, prolyl-tRNA synthetase, seryl-tRNA synthetase, threonyl-tRNA synthetase, tyrosyl-tRNA synthetase, or valyl-tRNA synthetase.

13. The method according to any one of claims 9 to 12, wherein said host cell into which a mutation is incorporated is a bacterium, yeast, animal cell, or plant cell.

14. The method according to any one of claims 9 to 12, wherein said host cell into which a mutation is incorporated is a bacterium or yeast.

15. The method according to claim 13 or 14, wherein said bacterium is a bacterium of the genus Bacillus.

16. The method according to any one of claims 9 to 15, wherein the protein encoded by the foreign gene is an enzyme selected from the group consisting of oxidoreductases, transferases, hydrolases, phosphorylases, lyases, isomerases, ligases/synthetases, and modifying enzymes.
